(19) Europäisches Patentamt
European Patent Office
Office européen des brevets

(11) Veröffentlichungsnummer: **0 049 683**
**A2**

(12) # EUROPÄISCHE PATENTANMELDUNG

(21) Anmeldenummer: 81810387.1

(22) Anmeldetag: 21.09.81

(51) Int. Cl.$^3$: **C 07 D 498/18**
C 07 D 498/08, A 61 K 31/335
//(C07D498/18, 307/00, 267/00, 235/00), (C07D498/08, 307/00, 267/00)

(30) Priorität: 25.09.80 CH 7184/80
25.09.80 CH 7185/80

(43) Veröffentlichungstag der Anmeldung:
14.04.82 Patentblatt 82/15

(84) Benannte Vertragsstaaten:
AT BE CH DE FR IT LI LU NL SE

(71) Anmelder: CIBA-GEIGY AG
Patentabteilung Postfach
CH-4002 Basel(CH)

(72) Erfinder: Kump, Wilhelm, Dr.
Friedrich-Oser-Strasse 10
CH-4105 Biel-Benken(CH)

(72) Erfinder: Traxler, Peter, Dr.
Bündtenring 5
CH-4124 Schönenbuch(CH)

(72) Erfinder: Scartazzini, Riccardo, Dr.
Conrad Ferdinand Meyer-Strasse 38
CH-4059 Basel(CH)

(54) **Imidazo-Rifamycine, Verfahren zu ihrer Herstellung, diese enthaltende pharmazeutische Präparate sowie ihre Verwendung.**

(57) N,N,Disubstituierte Derivate von 4-Aminoimidazolo-[4,5-c]rifamycin SV oder S der Formel

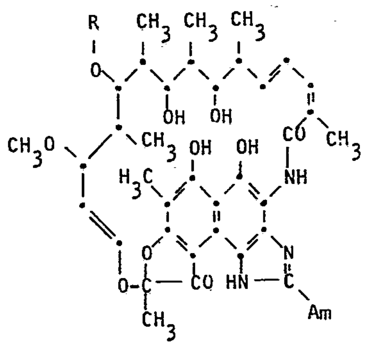

(1A) bzw.

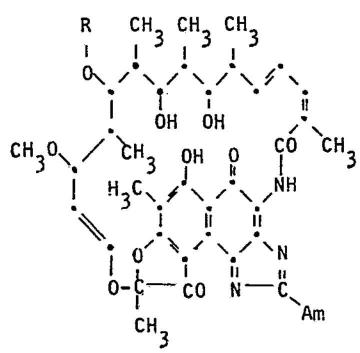

(1B),

worin R Wasserstoff oder Acetyl und Am eine von einem sekundären Amin abgeleitete Aminogruppe bedeutet, sowie Salze von entsprechenden Verbindungen mit salzbildenden Eingenschaften können in der Therapie von Infektionskrankheiten Anwendung als Antibiotika finden. Sie können mittels an sich bekannter Methoden, z.B. aus 3-Aminorifamycin-Verbindungen durch die Behandlung mit Ammoniak und mit einem reaktionsfahigen Derivat eines Formamids der Formel Am−CH=O in beliebiger Reihenfolge hergestellt werden.

Zum Erfindungsgegenstand gehören auch die als Zwischenprodukte erhältlichen Formamidine der Formel

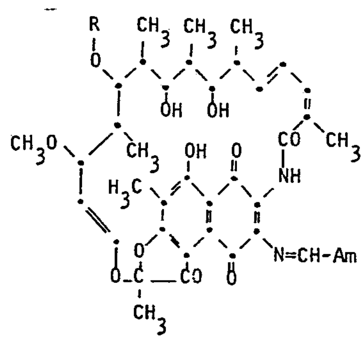

worin Am und R die obige Bedeutung haben, sowie entsprechende Verbindungen der SV-Reihe. Sie können durch die Behandlung einer entsprechenden 3-Aminorifamycin-Verbindung mit einem reaktionsfähigen Derivat eines entsprechenden sekundären N-Formylamins in an sich bekannter Weise hergestellt werden und besitzen analoge antibiotische Eigenschaften wie die Imidazol-Derivate der Formel I.

EP 0 049 683 A2

4-13075/13076/+

BEZEICHNUNG GEÄNDERT
siehe Titelseite

Neue 2-Aminoimidazole, Verfahren zu ihrer Herstellung, pharmazeutische Präparate enthaltend dieselben, sowie ihre Verwendung.

Die Erfindung betrifft neue 2-Aminoimidazole, welche durch ihre Atome C-4 und C-5 mit Ring A einer Rifamycin-Verbindung in 3,4-Stellung anelliert sind, insbesondere N,N-disubstituierte Derivate von 4-Aminoimidazolo[4,5-c]rifamycin SV oder S der Formel I, d.h.

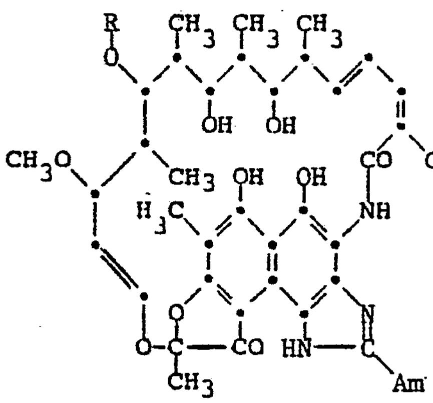

(IA) bzw.

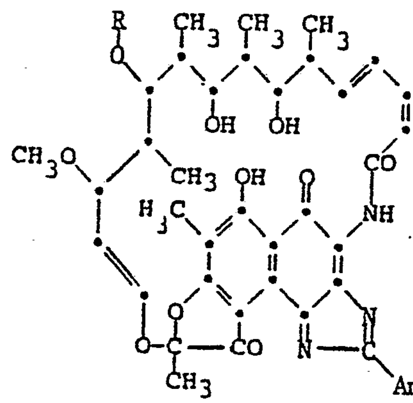

(IB)

worin R Wasserstoff oder Acetyl und Am eine von einem sekundären Amin abgeleitete Aminogruppe bedeutet, sowie Salze von entsprechenden Verbindungen mit salzbildenden Eigenschaften. - Die Erfindung betrifft ferner auch Verfahren zur Herstellung der Verbindungen der Formeln IA und IB, diese enthaltende pharmazeutische Präparate, sowie Verwendung dieser Verbindungen und Präparate.

Infolge der sehr engen Beziehung zwischen der 1,4-Chinon- und -Hydrochinon-Form (entsprechend Rifamycin-S und SV) und der Leichtigkeit, mit welcher die beiden Formen ineinander übergehen, sind überall, wo nicht spezifisch anders angegeben, beide Formen inbegriffen, wobei jedoch die SV-Form (IA) als die bevorzugte anzusehen ist.

Die von einem sekundären Amin abgeleitete Aminogruppe Am ist eine solche, derer Stickstoffatom zwei gleiche oder verschiedene einwertige, gegebenenfalls substituierte Hydrocarbylreste trägt oder zusammen mit einem zweiwertigen, gegebenenfalls substituierten Hydrocarbylrest einen stickstoffhaltigen nichtaromatischen heterocyclischen Rest bildet.

Der Hydrocarbylrest (Kohlenwasserstoffrest) kann ein acyclischer, carbocyclischer oder carbocyclisch-acyclischer Kohlenwasserstoffrest sein, der vorzugsweise höchstens 20 Kohlenstoffatome hat und gesättigt oder ungesättigt, unsubstituiert oder substituiert sein kann. Im Hydrocarbyl können ein, zwei oder mehrere C-Atome, die mit dem Stickstoffatom der Aminogruppe nicht unmittelbar verbunden sind, durch Heteroatome, wie insbesondere Sauerstoff, Schwefel oder Stickstoff, aber auch Phosphor oder Silicium, ersetzt sein; ein cyclischer Hydrocarbylrest dieser Art wird dann spezifisch als ein heterocyclischer Rest (Heterocyclyl) bezeichnet.

Als ungesättigte Reste sind solche bezeichnet, die eine, zwei oder mehrere Mehrfachbindungen, wie Doppel- und Dreifachbindungen, enthalten. Cyclische Reste, worin mindestens ein 6-gliedriger carbocyclischer oder 5- bis 8-gliedriger heterocyclischer Ring die maximale Anzahl nichtcumulierter Doppelbindungen enthält, werden als aromatisch bezeichnet. Carbocyclische Reste, worin mindestens ein Ring als ein 6-gliedriger aromatischer Ring (d.h. Benzolring) vorliegt, werden als Arylreste bezeichnet. - Als bi- und polycyclische Reste sind solche mehrkernige Reste bezeichnet, in welchen zwei oder mehrere Ringe mindestens ein gemeinsames Atom haben und somit ein anelliertes, verbrücktes oder spiro-gebundenes Ringsystem bilden.

Wenn nicht anders angegeben, enthalten in der vorliegenden Offenbarung mit "nieder" bezeichnete organische Reste höchstens 7, vorzugsweise höchstens 4 Kohlenstoffatome.

Ein acyclischer Kohlenwasserstoffrest ist insbesondere ein gerader oder verzweigter Alkyl-, Alkenyl-, Alkadienyl- oder Alkynylrest, vor allem ein Niederalkyl-, Niederalkenyl-, Niederalkadienyl- oder Niederalkynylrest. Niederalkyl ist z.B. Methyl, Ethyl, n-Propyl, Isopropyl, n-Butyl, Isobutyl, sek-Butyl oder tert-Butyl, sowie n-Pentyl, 3-Pentyl, Isopentyl, 2-Methylbutyl, n-Hexyl, Isohexyl, 2-Methylpentyl, 2-Ethylbutyl, oder n-Heptyl; Niederalkenyl ist z.B. Vinyl, Allyl, Propenyl, Isopropenyl, 2- oder 3-Methallyl, 2- oder 3-Butenyl, 2-Ethyl-2-butenyl und 2,3-Dimethyl-2-butenyl; Niederalkynyl ist z.B. Propargyl oder 2-Butynyl.

Ein carbocyclischer Kohlenwasserstoffrest ist insbesondere ein mono-, bi- oder polycyclischer Cycloalkyl-, Cycloalkenyl- oder Cycloalkadienylrest, oder ein entsprechender, aromatische Ringe enthaltender Arylrest. Bevorzugt sind Reste mit höchstens 12 Ringkohlenstoffatomen und 3- bis 8-, insbesondere 5- und/oder 6-gliedrigen Ringen, wobei sie auch einen oder mehrere acyclische Reste, z.B. die oben genannten, und insbesondere die Niederalkylreste, oder weitere carbocyclische Reste tragen können. Carbocyclisch-acyclische Reste sind solche, in welchen ein acyclischer Rest, insbesondere einer mit höchstens 7, vorzugsweise höchstens 4 Kohlenstoffatomen, einen oder mehrere carbocyclische, gegebenenfalls aromatische Reste der obigen Definition trägt. Insbesondere sind Cycloalkyl-niederalkyl- und Arylniederalkylreste, sowie ihre im Ring und/oder Seitenkette ungesättigten Analogen, zu erwähnen, und darunter speziell diejenigen, die höchstens 2 Ringe tragen.

Cycloalkyl ist z.B. Cyclopropyl, Cyclobutyl, Cyclopentyl, Cyclohexyl, Cycloheptyl und Cyclooctyl, sowie 1- oder 2-Bicyclo[2,2,2]octyl, 2-Bicyclo[2,2,1]heptyl 1- oder 2-Decahydronaphthyl und 1- oder 2-Adamantyl, ferner auch 1-, 2- oder 3-Methylcyclopentyl, 4-tert-Butylcyclohexyl, 4,4-Dimethylcyclohexyl, 2,4,6-Trimethylcyclohexyl und 2,4,4,6-Tetramethylcyclohexyl; Cycloalkenyl ist z.B. einer der bereits genannten Cycloalkylreste, der eine Doppelbindung in 1-, 2- oder 3-Stellung trägt, wie 1-, 2- oder 3-Cyclopentenyl und 1-, 2- oder

3-Cyclohexenyl. Cycloalkyl-niederalkyl oder -niederalkenyl ist z.B. Cyclopropyl-, Cyclopentyl-, Cyclohexyl- oder Cycloheptyl-methyl, -1- oder -2-ethyl bzw. -vinyl, -1-, -2- oder -3-propyl bzw. -allyl, ferner auch Dicyclohexylmethyl und Tricyclohexylmethyl; Cycloalkenyl-niederalkyl oder -niederalkenyl ist z.B. 1-, 2- oder 3-Cyclopentenyl- oder 1-, 2- oder 3-Cyclohexenyl-methyl, -1- oder -2-ethyl bzw. -vinyl, -1-, -2- oder -3-propyl bzw. -allyl.

Ein Arylrest ist in erster Linie ein Phenyl, ferner ein Naphthyl, wie 1- oder 2-Naphthyl, ein teilweise hydriertes Naphthyl, wie insbesondere 1-, 2-, 5- oder 6-(1,2,3,4-Tetrahydronaphthyl) ein Biphenylyl, wie insbesondere 4-Biphenylyl, weiter auch ein Anthryl, Fluorenyl und Azulenyl. Bevorzugte Aryl-niederalkyl- und -niederalkenyl-Reste sind z.B. Phenylniederalkyl oder Phenyl-niederalkenyl, wie z.B. Benzyl, 1- oder 2- Phenylethyl, 1-, 2- oder 3-Phenylpropyl, Diphenylmethyl (d.h. Benzhydryl), Trityl und 1- oder 2-Naphthylmethyl, bzw. Styryl oder Cinnamyl.

Heterocyclische Reste, einschliesslich der heterocyclisch-acyclischen Reste sind insbesondere monocyclische, aber auch bi- oder polycyclische, aza-, thia-, oxa-, thiaza-, thiadiaza-, oxaza-, diaza-, triaza- oder tetrazacyclische Reste die aromatisch, partiell gesättigt oder vollgesättigt sein können. Sie können auch in analoger Weise wie die obgenannten carbocyclischen oder Arylreste, weitere acyclische, carbocyclische oder heterocyclische Reste tragen und mono-, di- oder polysubstituiert sein. Der acyclische Teil in heterocyclisch-acyclischen Resten hat z.B. die für die entsprechenden carbocyclisch-acyclischen Reste gegebene Bedeutung. In erster Linie sind es unsubstituierte oder substituierte monocyclische monoaza-, monothia- oder monooxacyclische Reste, wie Aziridinyl, Oxiranyl und Thiiranyl, ferner auch Azepinyl und Azocinyl, und insbesondere heterocyclische Reste mit 5 oder 6 Ringgliedern, wie Pyrryl, z.B. 2-Pyrryl oder 3-Pyrryl, Pyridyl, z.B. 2-, 3- oder 4-Pyridyl, ferner Thienyl, z.B. 2- oder 3-Thienyl, oder Furyl, z.B. 2-Furyl; bicyclische monoaza-, monooxa- oder monothiacyclische Reste, wie Indolyl, z.B. 2- oder 3-Indolyl, Chinolinyl, z.B. 2- oder 4-Chinolinyl, Isochinolinyl, z.B. 1-Isochinolinyl, Benzofuranyl, z.B. 2- oder 3-Benzofuranyl, oder Benzothienyl, z.B. 2- oder 3-Benzothienyl; monocyclische diaza-, triaza-, tetraza-, oxaza-, thiaza- oder thiadiazacyclische Reste, wie Imidazolyl, z.B. 2-Imidazolyl, Pyrimidinyl, z.B. 2- oder 4-Pyrimidinyl, Triazolyl, z.B. 1,2,4-Triazol-3-yl, Tetrazolyl, z.B. 1- oder 5-Tetrazolyl, Oxazolyl, z.B. 2-Oxazolyl, Isoxazolyl, z.B. 3- oder 4-Isoxazolyl, Thiazolyl, z.B. 2-Thiazolyl, Isothiazolyl, z.B. 3- oder 4-Isothiazolyl oder 1,2,4- oder 1,3,4-Thiadiazolyl, z.B. 1,2,4-Thiadiazol-3-yl oder 1,3,4-Thiadiazol-2-yl; oder bicyclische diaza-, oxaza- oder thiazacyclische Reste, wie Benzimidazolyl, z.B. 2-Benzimidazolyl, Benzoxazolyl, z.B. 2-Benzoxazolyl, oder Benzthiazolyl, z.B. 2-Benzthiazolyl. Entsprechende partiell oder ganz gesättigte Reste sind z.B. Tetrahydrothienyl, wie 2-Tetrahydrothienyl, Dihydrofuryl, wie 2,5-Dihydro-2-furyl, Tetrahydrofuryl, wie 2-Tetrahydrofuryl, 2-(1,3-Dioxolanyl)-, Tetrahydropyranyl, wie 2- und 4-Tetrahydropyranyl, Pyrrolidyl, wie 2-Pyrrolidyl, Tetrahydropyridyl, wie $\Delta^1$-, $\Delta^2$- oder $\Delta^3$-Piperideinyl, oder Piperidyl, wie 2-, 3-, oder 4-Piperidyl, welche am N-Atom substituiert, wie niederalkyliert sein können, wie z.B. N-Methyl-4-piperidyl, ferner auch Morpholinyl, Thiomorpholinyl, Piperazinyl und N,N'-Bis-niederalkylpiperazinyl, wie insbesondere N,N'-Dimethylpiperazinyl, sowie Perhydroazepinyl und Perhydroazocinyl. Diese Reste können auch eine oder mehrere acyclische, carbocyclische oder heterocyclische Reste, insbesondere die oben genannten, tragen. Heterocyclisch-acyclische Reste sind insbesondere von acylischen Resten mit höchstens 7, vorzugsweise höchstens 4 Kohlenstoffatomen, z.B. von den oben genannten, abgeleitet und können einen, zwei oder mehrere heterocyclische Reste, z.B. die oben genannten, tragen.

Wie bereits erwähnt wurde, kann ein Hydrocarbylrest (einschliesslich eines Heterocyclylrests) durch einen, zwei oder mehrere gleichartige oder verschiedenartige Substituenten substituiert sein; die folgenden Substituenten kommen insbesondere in Betracht: freie, veretherte und veresterte Hydroxylgruppen; Mercapto- sowie Niederalkylthio- und

gegebenenfalls substituierte Phenylthiogruppen; Halogenatome (wie Chlor und Fluor, aber auch Brom und Jod);Formyl- (d.h. Aldehydo-) und Keto-gruppen, auch als Acetale bzw. Ketale; Azido- und Nitrogruppen; primäre, sekundäre und vorzugsweise tertiäre Aminogruppen, die auch in Form von Additionssalzen mit geeigneten anorganischen und organischen Säuren vorliegen können, durch konventionelle Schutzgruppen geschützte primäre oder sekundäre Aminogruppen (wie geeignete Acylaminogruppen und Diacylaminogruppen);ferner freie und in Salz-Form (wie Alkalimetallsalz-Form) vorliegende Sulfaminogruppen; freie und funktionell abgewandelte Carboxylgruppen (wie in Salzform vorliegende oder veresterte Carboxylgruppen, gegebenenfalls einen oder zwei Kohlenwasserstoffreste tragende Carbamoyl-, Ureidocarbonyl- oder Guanidinocarbonylgruppen, und Cyangruppen);sowie gegebenenfalls funktionell abgewandelte Sulfogruppen (wie Sulfamoyl- oder in Salzform vorliegende Sulfogruppen) und Trifluormethyl.

Eine als Substituent im Hydrocarbyl vorliegende veretherte Hydroxylgruppe ist z.B. eine Niederalkoxygruppe, wie die Methoxy-, Ethoxy-, Propoxy-, Isopropoxy-, Butoxy- und tert-Butoxygruppe, welche auch substituiert sein kann. So kann eine solche Niederalkoxygruppe durch Halogenatome, insbesondere in 2-Stellung (wie im 2,2,2-Trichlorethoxy-, 2-Chlorethoxy- oder 2-Jodethoxyrest) oder durch Niederalkoxyreste, insbesondere in 1-Stellung (wie im 1-Butoxyethoxyrest) oder in 2-Stellung (wie im 2-Methoxyethoxyrest), oder aber durch eine Hydroxylgruppe, insbesondere in 2-Stellung (wie im 2-Hydroxyethoxyrest) substituiert sein. Ferner sind solche veretherte Hydroxylgruppen auch gegebenenfalls substituierte Phenoxyreste und Phenylniederalkoxyreste, wie vor allem Benzyloxy-, Benzhydryloxy- und Triphenylmethoxy-(Trityloxy-)-Reste, sowie Heterocyclyloxyreste, wie insbesondere 2-Tetrahydrofuranyloxy- und 2-Tetrahydropyranyloxyreste. Unter veretherten Hydroxylgruppen sind in diesem Zusammenhang auch silylierte Hydroxylgruppen zu verstehen, wie sie z.B. in Triniederalkylsilyloxy-, wie Trimethylsilyloxy- oder Dimethyl-tert-butylsilyloxy-, oder Phenyldiniederalkylsilyloxy- bzw. Niederalkyldiphenylsilyloxygruppen vorliegen.

Eine als Substituent im Hydrocarbyl vorliegende veresterte Hydroxylgruppe ist insbesondere eine solche, worin das Wasserstoffatom der Hydroxylgruppe durch einen weiter unten definierten Acylrest Ac ersetzt ist. Ferner kann sie auch eine lactonisierte Hydroxylgruppe sein.

Eine als Substituent im Hydrocarbyl vorliegende veresterte Carboxylgruppe ist eine solche, in welcher das Wasserstoffatom durch einen der oben charakterisierten Kohlenwasserstoffreste, vorzugsweise einen Niederalkyl- oder Phenylniederalkylrest., ersetzt ist; als Beispiel einer veresterten Carboxylgruppe sind insbesondere die Methoxy-, Niederalkoxycarbonylgruppen, wie Ethoxy- und tert-Butoxy-carbonylgruppe oder Phenylniederalkoxycarbonylgruppen, wie die Benzyloxycarbonylgruppe, sowie auch eine lactonisierte Carboxylgruppe zu nennen. Ferner ist eine veresterte Carboxylgruppe eine solche, die als Silylester vorliegt, d.h. ein Trihydrocarbylsilyl, wie Triniederalkylsilyl und insbesondere Trimethylsilyl, trägt.

Eine primäre Aminogruppe $-NH_2$ als Substituent des Hydrocarbyls kann auch in geschützter Form vorliegen, vorzugsweise als eine entsprechende Acylaminogruppe der Formel -NH-Ac, worin Ac die nachstehend charakterisierte Bedeutung hat. Eine sekundäre Aminogruppe trägt anstelle eines der beiden Wasserstoffatome einen unsubstituierten Kohlenwasserstoffrest, wie oben definiert wurde; sie kann auch in einer geschützten Form vorliegen, z.B. als eine davon abgeleitete Acylaminogruppe, die einen nachstehend charakterisierten monovalenten Acylrest Ac trägt. Eine als Substituent des Hydrocarbyls vorkommende tertiäre Aminogruppe ist eine solche, die anstelle beider Wasserstoffatome gleiche oder verschiedene Reste trägt, die den oben charakterisierten unsubstituierten Hydrocarbylresten entsprechen. Die beiden Hydrocarbylreste können durch eine Kohlenstoff-Kohlenstoff-Bindung oder ein Sauerstoff-, Schwefel- oder gegebenenfalls substituiertes Stickstoffatom untereinander gebunden sein und zusammen mit dem Stickstoffatom der Aminogruppe einen stickstoffhaltigen heterocyclischen Rest bilden. Als Beispiel von besonders bevorzugten, als Substituent des Hydrocarbyls dienenden Aminogruppen sind die folgenden zu nennen: Diniederalkylamino (wie Dimethylamino und Diethylamino), Pyrrolidino, Piperidino, Morpholino, Thiomorpholino und Piperazino oder 4-Methylpiperazino, gegebenenfalls durch Niederalkyl, Niederalkoxy, Halogen und/oder Nitro substituierte Diphenylamino und Dibenzylamino; unter den geschützten auch insbesondere Halogenniederalkoxycarbonylamino, wie 2,2,2-Trichlorethoxycarbonylamino, Phenylniederalkoxycarbonylamino, wie 4-Methoxybenzyloxycarbonylamino, sowie 2-(Trihydrocarbylsilyl)-ethoxycarbonylamino, wie 2-Triphenylsilylethoxycarbonylamino, 2-(Dibutyl-methylsilyl)-ethoxycarbonylamino und 2-Trimethylsilylethoxycarbonylamino.

Der Acylrest Ac einer Carbonsäure ist der Rest eines Kohlensäurehalbesters, wie ein Niederalkoxycarbonyl oder Arylniederalkoxycarbonyl, oder das Formyl, oder der Rest einer gegebenenfalls substituierten acyclischen, carbocyclischen, carbocyclisch-acyclischen, heterocyclischen oder heterocyclisch-acyclischen Carbonsäure, welcher einer der oben definierten unsubstituierten oder substituierten Hydrocarbylreste zugrundeliegt. Besonders bevorzugt sind Acylreste der folgenden Monocarbonsäuren mit höchstens 18 Kohlenstoffatomen: acyclische Carbonsäuren, insbesondere Niederalkancarbonsäuren, wie die Propion-, Butter-, Isobutter-, Valerian-, Isovalerian, Capron-, Trimethylessig-, Oenanth- und Diethylessigsäure und vor allem die Essig- und Ameisensäure, aber auch entsprechende halogenierte, insbesondere chlorierte und bromierte, Niederalkancarbonsäuren, wie die Chloressigsäure, Dichloressigsäure, Bromessig- oder α-Bromisovaleriansäure; carbocyclische oder carbocyclisch-acyclische Monocarbonsäuren, z.B. die Cyclopropan-, Cyclobutan-, Cyclopentan- und Cyclohexan-carbonsäure bzw. die Cyclopropan-, Cyclobutan-, Cyclopentan- oder Cyclohexan-essigsäure oder -propionsäure; aromatische carbocyclische Carbonsäuren, z.B. Benzoesäure, die einfach oder mehrfach durch Halogene (wie Fluor, Chlor oder Brom) und/oder Hydroxy, Niederalkoxy, Niederalkyl, Trifluormethyl und/oder Nitro substituiert sein kann; Aryl- oder Aryloxy-niederalkancarbonsäuren

und deren in der Kette ungesättigte Analoga, z.B. gegebenenfalls, wie oben für die Benzoesäure angegeben, substituierte Phenylessig- bzw. Phenoxyessigsäuren, Phenylpropionsäuren und Zimtsäuren; und heterocyclische Säuren, z.B. Furan-2-carbonsäure, 5-tert-Butylfuran-2-carbonsäure, 5-Bromfuran-2-carbonsäure, Thiophen-2-carbonsäure, Nicotin- oder Isonicotinsäure, 4-Pyridinpropionsäure, und gegebenenfalls durch Niederalkylreste substituierte Pyrrol-2- oder -3-carbonsäuren; ferner auch entsprechende α-Aminosäuren, insbesondere die in der Natur vorkommenden Aminosäuren der L-Reihe, z.B. Glycin, Phenylglycin, Prolin, Leucin, Valin, Tyrosin, Histidin und Asparagin, vorzugsweise in einer N-geschützten Form, d.h. in einer solchen, in welcher die Aminogruppe durch eine konventionelle, z.B. eine der obgenannten, Aminoschutzgruppe substituiert ist. Unter den Carbonsäuren kommen auch solche insbesondere in Betracht, worin das Hydrocarbyl selbst noch durch ein weiteres, gegebenenfalls funktionell abgewandeltes Carboxyl substituiert ist, d.h. Dicarbonsäuren, vorzugsweise solche mit höchstens 12 Kohlenstoffatomen, welchen einer der oben charakterisierten gegebenenfalls substituierten acyclischen, carbocyclischen, carbocyclisch-acyclischen, heterocyclischen und heterocyclisch-acyclischen Hydrocarbylresten zugrundeliegt. Beispielsweise sind die folgenden Dicarbonsäuren zu nennen: Oxalsäure, Malonsäure, Mono- oder Di-niederalkylmalonsäuren, Bernsteinsäure, Glutarsäure, Adipinsäure, Maleinsäure, Itaconsäure, Citraconsäure, Angelicasäure, 1,1-Cyclopentan- oder 1,1-Cyclohexandicarbonsäure, eine durch Halogen, insbesondere Chlor oder Brom, und/oder Niederalkyl, Niederalkoxy und Nitro gegebenenfalls substituierte Phthal-, Chinolin- oder Phenylbernsteinsäure, sowie auch Tartronsäure, Mesoxalsäure, Oxalessigsäure, Apfelsäure, Weinsäure, eine an den Hydroxylgruppen veresterte oder veretherte Weinsäure, Glutaminsäure und Asparaginsäure, wobei die zwei letztgenannten Säuren vorzugsweise mit geschützten Aminogruppen vorliegen. Wie bereits gesagt wurde, kann die zweite Carboxylgruppe nicht nur frei, sondern auch funktionell abgewandelt, z.B. als ein Ester mit einem Alkohol, oder als ein Salz, vorzugsweise als ein physiologisch verträgliches

Salz, mit einer salzbildenden basischen Komponente vorhanden sein. In Betracht kommen in erster Linie Metall- oder Ammoniumsalze, wie Alkalimetall- und Erdalkalimetall-, z.B. Natrium-, Kalium-, Magnesium- oder Calciumsalze, bzw. Ammoniumsalze mit Ammoniak oder geeigneten organischen Aminen. Unter diesen kommen besonders tertiäre Monoamine und heterocyclische Basen in Frage, z.B. Triethylamin, Tri-(2-hydroxyethyl)-amin, 1-Ethyl-piperidin, sowie Pyridin, Collidin oder Chinolin, in Betracht.

Die Erfindung betrifft insbesondere diejenigen Rifamycin-Verbindungen der Formel I, worin die Aminogruppe Am durch die Partialformel

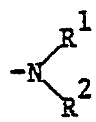

( $Am_A$)

charakterisiert ist, worin $R^1$ und $R^2$ unabhängig voneinander je ein Niederalkyl oder Niederalkenyl, das einfach oder mehrfach durch Hydroxyl, Mercapto, Niederalkoxyl, Niederalkoxycarbonyl, ein gegebenenfalls N-mono- oder -di-substituiertes Carbamoyl, Formyl, Oxo, acetalisiertes, bzw. ketalisiertes Oxo und/oder eine disubstituierte Aminogruppe substituiert sein kann, ein Niederalkynyl, ein Cycloalkyl oder Cycloalkyl-niederalkyl, das gegebenenfalls eine oder zwei Doppelbindungen aufweisen kann, ein unsubstituiertes oder durch Halogen, Hydroxyl, Trifluormethyl, ein gegebenenfalls N-mono- oder -di-substituiertes Carbamoyl oder Niederalkoxycarbonyl einfach oder mehrfach substituiertes Phenyl oder Phenylniederalkyl, oder ein höchstens bicyclisches Heterocyclyl oder Heterocyclylalkyl mit höchstens zwei Heteroatomen ausgewählt von Sauerstoff, Stickstoff und/oder Schwefel bedeuten, wobei $R^1$ und $R^2$ durch eine einfache Kohlenstoff-Kohlenstoff-Bindung oder über ein Sauerstoff-, Schwefel(II)- oder gegebenenfalls substituiertes Stickstoffatom miteinander verbunden sein können und zusammen mit dem Aminostickstoffatom einen 4- bis 8-gliedrigen nichtaromatischen heterocyclischen Ring bilden können.

Wenn nicht spezifisch anders definiert, haben alle Begriffe der voranstehenden Definition die eingangs angegebenen allgemeinen und bevorzugten Bedeutungen.

Als Substituent eines gesättigten Restes ist eine Hydroxyl-, Mercapto- oder disubstituierte Aminogruppe vorzugsweise von der zentralen Aminogruppe durch mindestens zwei Kohlenstoffatome getrennt; als Substituent eines ungesättigten Restes befinden sich diese Gruppen vorzugsweise auf einem gesättigten Kohlenstoffatom.

In ungesättigten nichtaromatischen Resten befindet sich die Mehrfachbindung vorzugsweise auf Kohlenstoffatomen, die mit der zentralen Aminogruppe nicht unmittelbar verbunden sind.

Als Phenylniederalkylreste in Bedeutung von $R^1$ and $R^2$ kommen insbesondere 1-Phenylethyl und Phenethyl, vor allem aber Benzyl in Betracht; sie können auch substituiert, wie oben angegeben, sein, wobei sich der Substituent vorzugsweise in p-Stellung befindet.

Als Heterocyclylreste in Bedeutung von $R^1$ oder $R^2$ kommen insbesondere monocyclische Reste in Betracht, wie die eingangs genannten, darunter vor allem 2- und 4-Pyridyl, 2-Imidazolyl, 2-Benzimidazolyl, 3-Indolyl und 4-Chinolyl, als Heterocyclyl-niederalkylreste sind Furfuryl und Thenyl, und unter entsprechenden gesättigten Resten z.B. N-Methyl-4-piperidyl, 4-Tetrahydropyranyl, Tetrahydrofuryl-2-methyl, 1,3-Dioxolanyl-2-methyl, 2-(1,3-Dioxolanyl-2)-ethyl und 2,2-Dimethyl-1,3-dioxolanyl-4-methyl hervorzuheben.

Nichtaromatische heterocyclische Reste in Bedeutung von $Am_A$, welche durch die Verknüpfung der Reste $R^1$ und $R^2$ mittels einer einfachen C-C-Bindung gebildet sind, sind beispielsweise die folgenden: für den Fall, dass beide Reste $R^1$ und $R^2$ Niederalkylreste (oder die entsprechenden Cycloalkyl, Phenyl- bzw. Heterocyclyl-tragende Niederalkylreste) sind , kommen die folgenden Reste $Am_A$ insbesondere in Betracht: 1-Azetidinyl, 1-Perhydroazepinyl, 1-Perhydroazocinyl, und vor allem 1-Pyrrolidinyl und Piperidino, welche auch durch die oben genannten Substituenten substituiert sein und/oder entsprechend substituierte oder vorzugsweise unsubstituierte Niederalkyl-, Cycloalkyl-, Cycloalkyl-niederalkyl-, Phenyl-, Phenyl-niederalkyl-, Heterocyclyl und Heterocyclyl-niederalkyl-Reste tragen können, wobei vorzugsweise nur ein solcher Substituent und/oder Rest vorhanden ist und die Gesamtzahl der Kohlenstoffatome höchstens 15 ist; im analogen Fall, worin einer der Reste $R^1$ und $R^2$ ein cyclischer Rest ist, kommen die folgenden Reste $Am_A$ insbesondere in Betracht: 1-Indolinyl, 2-Isoindolinyl, 2-Perhydroisoindolyl und 4-aza-tricyclo[5,2,2,$0^{2,6}$]non-8-en-4-yl, welche auch in analoger Weise, und vorzugsweise mit analoger Beschränkung, substituiert sein und/oder die erwähnten kohlenstoffhaltigen Reste tragen können.Unter allen derartigen Resten sind die folgenden hervorzuheben: ein durch Hydroxyl oder Carboxyl substituiertes 1-Pyrrolidinyl, ein durch Formyl, Carboxyl, Niederalkoxycarbonyl, Carbamoyl (auch N,N-Diniederalkylcarbamoyl, wie N,N-Diethylcarbamoyl) oder Oxo (auch als ketalisiertes Oxo, wie Ethylendioxy) substituiertes Piperidino, wobei der Substituent sich vorzugsweise in 3- oder 4-Stellung befindet, ein Niederalkyl-, 1-Hydroxyniederalkyl- oder 2-Hydroxyniederalkyl-Piperidino, wobei der Rest sich vorzugsweise in der 4-Stellung befindet, ein 4-Diniederalkylaminopiperidino- (wie 4-Dimethylaminopiperidino), 4-Piperidinopiperidino, sowie 8-Aza-1,4-dioxa-spiro[4,5]dec-8-yl (d.h. 4-Piperidono-ethylen-ketal).

Nichtaromatische heterocyclische Reste in Bedeutung von $Am_A$, welche durch die Verknüpfung der Reste $R^1$ und $R^2$ mittels eines Sauerstoff- oder Schwefel(II)-atoms gebildet sind, sind beispielsweise Morpholino, Thiomorpholino und 3-Thiazolidinyl, weiter auch 3,5-Dimethylmorpholino und 2,6-Dimethylmorpholino.

Nichtaromatische heterocyclische Reste in Bedeutung von $Am_A$, welche durch die Verknüpfung der Reste $R^1$ und $R^2$ mittels eines gegebenenfalls substituierten Stickstoffatoms gebildet sind, sind diejenigen, welche

sich von 2 Niederalkylresten $R^1$ und $R^2$ ableiten und worin das verbindende gegebenenfalls substituierte Stickstoffatom einen als $R^N$ unten definierten Substituenten trägt. Ein derartiger nichtaromatischer heterocyclischer Rest ist insbesondere ein solcher der Partialformel

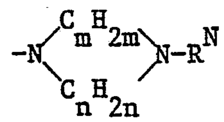

$(Am_B)$

worin m und n unabhängig je eine ganze Zahl von 1 bis 5, vorzugsweise 2 oder 3, darstellen, wobei ein mindestens 5-gliedriger Ring gebildet wird, und $R^N$ ein Wasserstoffatom, eine Formylgruppe, eine funktionell abgewandelte Carboxylgruppe oder einen unsubstituierten oder substituierten, höchsten 10 C-Atome aufweisenden Hydrocarbyl- oder Heterocyclyl-Rest bedeutet. - Die Reste $C_mH_{2m}$ und $C_nH_{2n}$ sind bivalente Radikale, die sich von Niederalkanen mit 1-6 C-Atomen ableiten, wie in erster Linie Ethylen, Trimethylen und Propylen, aber auch Tetramethylen, 1,1-Dimethylethylen, 1,2-Dimethylethylen, Ethylethylen, Propylethylen, Butylethylen, 1,2-Diethylethylen und 2,2-Dimethyltrimethylen, sowie auch Methylen, Ethyliden, Propyliden, Isopropyliden etc., die aber jeweils nur als einer der beiden Reste stehen können. Eine funktionell abgewandelte Carboxylgruppe in der Bedeutung von $R^N$ ist eine der oben definierten, insbesondere eine veresterte (wie insbesondere die in Form eines Niederalkylesters) oder in der Amid-Form vorliegende Carboxylgruppe, wie insbesondere eine unsubstituierte oder durch 1 oder 2 Niederalkylreste substituierte Carbamoylgruppe). Ein Hydrocarbyl bzw. Heterocyclylrest in der Bedeutung von $R^N$ ist einer der eingangs genannten, und kann die eingangs genannten Substituenten einzeln oder in Kombination tragen, beim Heterocyclylrest geht die freie Valenz jeweils von einem C-Atom aus. Unter cyclischen Resten sind monocyclische Reste bevorzugt, unter heterocyclischen solche mit höchstens 2 nicht benachbarten Heteroatomen, unter substituierten Resten solche, die nur einen Substituenten (= funktionelle Gruppe) tragen.

Unter den Rifamycin-Verbindungen der Formeln IA bzw. IB, die den Rest $Am_A$ tragen, sind ganz besonders diejenigen bevorzugt, worin $R^1$ und $R^2$ je ein unsubstituiertes Alkyl mit 1-4 C-Atomen darstellen, oder worin beide Symbole $R^1$ und $R^2$ zusammen mit dem Stickstoffatom einen gesättigten, monocyclischen, 5-8 Ringglieder enthaltenden Heterocyclylrest, welcher auch ein Sauerstoff- oder Schwefel(II)-Atom als Ringglied enthalten kann, bilden, oder worin der Rest $Am_A$ einer der oben definierten bevorzugten Bedeutungen von $Am_B$ entspricht.

Vor allem steht als das Symbol $Am_A$ die Dimethylamino-, Diethylamino-, Methylethylamino-, Methylisopropylamino- und Methylbutylamino-Gruppe, ferner auch der 1-Pyrrolidyl-, Piperidino-, 1-Perhydroazepinyl-, 1-Perhydroazocinyl-, Morpholino- und Thiomorpholino-rest, sowie auch ein gegebenenfalls 4-substituierter 1-Piperazinyl-rest der Formel

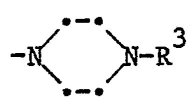

($Am_C$)

worin $R^3$ für Wasserstoff steht oder eine der individuellen Bedeutungen von $R^1$ oder $R^2$ hat. Unter den Bedeutungen von $R^3$ sind in erster Linie unsubstituierte geradkettige und einfach verzweigte Niederalkyl- und Niederalkenylreste (wie vor allem Methyl, Propyl, Isobutyl oder Allyl) besonders bevorzugt, ferner sind auch unsubstituierte Niederalkynyl- (wie Propargyl), Cycloalkyl- und Cycloalkyl-alkyl- (insbesondere Cycloalkyl-methyl)- mit 3-6 Ringgliedern, sowie Phenyl- und Benzylreste hervorzuheben.

Besonders hervorzuheben sind die in den Beispielen gezeigten und ihnen nahe analogen Verbindungen, insbesondere Salze davon.

Diejenigen der oben allgemein oder als bevorzugt charakterisierten Rifamycin-Analogen der Formel I, welche eine genügende Basicität haben, können als Säureadditionssalze, insbesondere als physiologisch verträgliche Salze, mit üblichen pharmazeutisch anwendbaren Säuren vorliegen; von der anorganischen Säuren sind die Halogenwasserstoffsäuren, wie die Chlorwasserstoffsäure, aber auch Schwefelsäure und Phosphor- bzw. Pyrophosphorsäure zu nennen, von den organischen Säuren sind es in erster Linie die Sulfonsäuren, wie die Benzol- oder p-Toluol-sulfonsäure, oder Niederalkansulfonsäuren, wie Methansulfonsäure, ferner auch Carbonsäuren, wie Essigsäure, Milchsäure, Palmitin- und Stearinsäure, Aepfelsäure, Weinsäure, Ascorbinsäure und Zitronensäure.

Diejenigen der erfindungsgemässen Verbindungen, die infolge einer sauer reagierenden funktionellen Gruppe (insbesondere dank dem phenolischen Charakter der 1-Hydroxylgruppe in Verbindungen der SV-Reihe) eine genügende Acidität aufweisen, können als Salze mit Basen, insbesondere als physiologisch verträgliche Salze, vorliegen. Als Salze kommen in Frage: Metall- und Ammoniumsalze, wie einerseits Erdalkalimetall und Alkalimetallsalze (z.B. Calcium-, Magnesium- und vorzugsweise Natrium- und Kaliumsalze), andererseits auch Ammoniumsalze abgeleitet vom Ammoniak selber oder einer geeigneten, vorzugsweise physiologisch verträglichen, organischen stickstoffhaltigen Base. Als Base kommen sowohl Amine, wie Niederalkylamine (z.B. Triethylamin), Hydroxyniederalkylamine [z.B. 2-Hydroxyethylamin, Di-(2-hydroxyethyl)-amin oder Tri-(2-hydroxyethyl)-amin], Cycloalkylamine (z.B. Dicyclohexylamin) oder Benzylamine (z.B. Benzylamin und N,N'-Dibenzylethylendiamin), als auch stickstoffhaltige heterocyclische Verbindungen, z.B. solche aromatischen Charakters (wie Pyridin oder Chinolin) oder solche mit einem mindestens teilweise gesättigten heterocyclischen Ring, (wie N-Ethylpiperidin, Morpholin, Piperazin oder N,N'-Dimethyl-piperazin) in Betracht. - Verbindungen, die sowohl eine saure wie auch eine basische funktionelle Gruppe enthalten, können als innere Salze vorliegen.

Die vorliegende Erfindung umfasst auch Verfahren zur Herstellung der oben charakterisierten Verbindungen der Formel I.

Die Verbindungen der Formel I können in an sich bekannter Weise hergestellt werden, indem man eine 3-Aminorifamycin-S-Verbindung der Formel

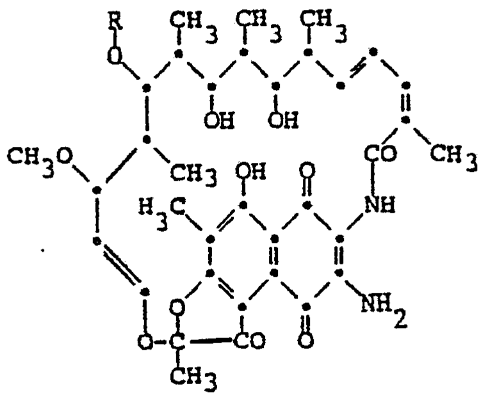

(II)

worin R die oben genannte Bedeutung hat, oder ein Salz davon, in beliebiger Reihenfolge mit Ammoniak und mit einem reaktionsfähigen Derivat eines N,N-disubstituierten Formamids oder Thioformamids der Formel

Am-CH=Y (III)

worin Y Sauerstoff oder Schwefel ist und Am die eingangs definierte Bedeutung hat, umsetzt und gewünschtenfalls eine erhaltene Verbindung des SV-Typs zu einer Verbindung des S-Typs oxidiert oder eine erhaltene Verbindung des S-Typs zu einer Verbindung des SV-Typs reduziert und/oder eine erhaltene freie Verbindung mit salzbildenden Eigenschaften in ein entsprechendes Salz oder ein erhaltenes Salz in die entsprechende freie Verbindung umwandelt.

Die Ausgangsstoffe der Formel II, d.h. 3-Aminorifamycin S, sowie das entsprechende 25-Desacetylderivat, sind bekannte Verbindungen. Sie können auch als entsprechende Säureadditionssalze eingesetzt werden. Auch typische Vertreter der Ausgangsstoffe der Formel III sind bekannt, oder, sofern sie unbekannt sind, können aus bekannten sekundären Aminen bzw. deren Strukturanalogen in analoger Weise wie die bekannten Verbindungen durch herkömmliche Verfahren hergestellt werden. - Die

zur Umsetzung als Reagens verwendeten reaktionsfähigen Derivate der Formamide bzw. Thioformamide der Formel III sind auch durch typische Vertreter bekannt oder durch an sich bekannte Arbeitsweisen zugänglich. In vielen Fällen ist es sogar nicht notwendig, diese reaktionsfähigen Formen als definierte chemische Individuen zu isolieren. - Unter den reaktionsfähigen Derivaten des Formamids bzw. Thioformamids kommen in erster Linie Säureamidacetale und Säureorthoamide, ferner auch Säureamidhalogenide, Säureamid-dialkylsulfatkomplexe, sowie Säurethioamidalkylhalogenid-komplexe, in Betracht; die letzteren insbesondere dann, wenn sie zur Umsetzung des Ausgangsstoffes der Formel II direkt eingesetzt werden.

Entsprechende Ameisensäureamidacetale sind insbesondere solche abgeleitet von $C_1$-$C_4$-Alkanolen, die durch die Formel

$$Am\text{-}CH(OR^4)_2 \qquad (III_a)$$

charakterisiert sind, worin Am die eingangs angegebene Bedeutung hat und $R^4$ ein höchstens 4 C-Atome aufweisendes Alkyl, vor allem Methyl oder Ethyl, bedeutet. Sie sind insbesondere deshalb vorteilhaft, weil sie sich leicht in stabiler individueller Form herstellen lassen. Zur Herstellung erhitzt man z.B. ein sekundäres Amin Am-H mit N,N-Dimethylformamid-diniederalkylacetal (z.B.-dimethylacetal), wodurch der Austausch der Dimethylaminogruppe gegen die gewünschte Aminogruppe Am erfolgt. Für flüchtige sekundäre Amine Am-H ist eine alternative Methode besser geeignet, u.zw. die Behandlung eines N,N-disubstituierten Formamiddialkylsulfatkomplexes (siehe weiter unten) mit einem Alkalimetallalkoholat der Formel $R^4OM$, worin $R^4$ die oben genannte Bedeutung hat und M ein Alkalimetall, insbesondere Natrium, darstellt.

Die als reaktionsfähige Formamidderivate verwendeten Ameisensäureorthoamide sind charakterisiert durch die Formel

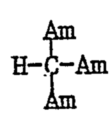

$(III_{aa})$

worin Am die obgenannte Bedeutung hat. Sie können z.B. durch den Austausch von Niederalkoxygruppen $OR^4$ gegen die disubstituierte Aminogruppe Am beim Erhitzen eines N,N-Dimethylformamid-diniederalkylacetals oder eines Formamidacetals der Formel $III_a$ mit dem entsprechenden sekundären Amin Am-H erhalten werden. Typische Vertreter dieser Verbindungen sowie Verfahren zu ihrer Herstellung sind bekannt.

Die verwendeten Ameisensäureamidhalogenide ("Iminoylhalogenide") sind vorzugsweise durch die Formel

$$[Am^+{=}CHX]X^- \qquad (III_b)$$

charakterisiert, worin Am die eingangs genannte Bedeutung hat und X Brom oder vorzugsweise Chlor darstellt. (Die Doppelbindung geht dabei vom Aminostickstoff der Aminogruppe Am aus und erteilt ihr somit die positive Ladung.) Diese Reagentien (d.h. "Iminoylchloride" und "Iminoylbromide") herstellt man z.B. durch die Behandlung der entsprechenden Formamide der Formel III mit herkömmlichen Halogenierungsmitteln. Vorzugsweise werden Halogenierungsmittel verwendet, die während der Reaktion gasförmige Nebenprodukte bilden, wie Phosgen, Oxalylhalogenide oder Thionylhalogenide, jedoch können auch andere verwendet werden. Die Umsetzung kann in inerten trockenen organischen Lösungsmitteln, beispielsweise Ether oder Toluol durchgeführt werden, worin das Amidhalogenid in den meisten Fällen unlöslich ist und aus dem es durch Abfiltrieren nach Beendigung der Reaktion isoliert werden kann. Die Säureamidhalogenide sind hygroskopisch und ziemlich instabil und werden deshalb vorzugsweise ohne Reinigung in der erfindungsgemässen Umsetzung verwendet.

Die verwendeten Formamid-dialkylsulfatkomplexe sind insbesondere diejenigen der Formel

$$[Am^+{=}CH{-}OR^4] \quad R^4O{-}SO_2{-}O^- \qquad (III_c)$$

worin Am und $R^4$ die oben angegebenen Bedeutungen haben.

Die Säureamiddialkylsulfatkomplexe können durch Behandlung der Amide der Formel III mit Dialkylsulfat, vorzugsweise Dimethylsulfat, unter bekannten Bedingungen, z.B. analog wie die Amidhalogenide $III_b$, hergestellt werden.

Wenn ein Ameisensäurethioamid als Ausgangsmaterial verwendet wird, so kann ein reaktionsfähiges Derivat in Form eines Säurethioamidalkylhalogenid-Komplexes durch Behandlung mit einem Alkylhalogenid, beispielsweise $C_1$-$C_4$-Alkyljodid, gebildet werden. Diese Reaktion ist aus der Fachliteratur bekannt.

Bei praktischer Durchführung wird jede der beiden erfindungsgemässen Verfahrensstufen in an sich bekannter Weise durchgeführt, wobei ihre Reihenfolge auf die konkreten Reaktionsbedingungen keinen Einfluss hat.

Die Umsetzung mit Ammoniak erfolgt vornehmlich durch Einleiten von trockenem Ammoniakgas in eine Lösung der Rifamycin-Verbindung in einem herkömmlichen aprotischen organischen Lösungsmittel, z.B. einem Ether (wie Diethylether, 1,2-Dimethoxyethan oder Dioxan und insbesondere Tetrahydrofuran) oder einem halogenierten Niederalkan (wie insbesondere Chloroform oder Methylenchlorid) bei Temperaturen im Bereich von etwa -10° bis etwa +40°C, vorzugsweise zwischen +5° bis +25°C, vornehmlich leicht unterhalb der Zimmertemperatur.

Die Reaktionsbedingungen für die erfindungsgemässe Umsetzung mit dem reaktionsfähigen Amidderivat hängen vorwiegend von dieser Reaktionskomponente ab. Wenn für die Umsetzung einer Rifamycin-Verbindung Säureamidacetale vom Typ der Formel $III_a$ verwendet werden, so liegt die Reaktionstemperatur bei etwa 0-40°, üblicherweise bei Raumtemperatur. Die Umsetzung wird in inerten organischen Lösungsmitteln, beispielsweise Ether, Dichlormethan oder Chloroform, die vorzugsweise alkoholfrei sind, und vorzugsweise in Anwesenheit einer organischen Base, wie Triethylamin, N,N-Diisopropyl-ethylamin, N-Ethylpiperidin, N-Methylpiperidin, N-Methylmorpholin oder N,N'-Dimethylpiperazin durchgeführt.

In analoger Weise setzt man auch mit den Orthoamiden der Formel $III_{aa}$ um.

Wenn Säureamidhalogenide, Dialkylsulfatkomplexe oder Thioamidalkylhalogenid-Komplexe verwendet werden, so wird die Umsetzung ebenfalls in inerten organischen Lösungsmitteln, die trocken und frei von Spuren von Alkoholen sind, vorzugsweise in Dichlormethan oder Chloroform , durchgeführt, worin die Reaktionskomponenten löslich sind, jedoch können auch Lösungsmittel, worin die Ausgangsmaterialien unlöslich sind, verwendet werden, wie beispielsweise Ether. Die Umsetzung wird bei einer Temperatur zwischen etwa -70° bis etwa 20°, üblicherweise bei oder unter 0° und in Gegenwart von mindestens 1 Aequivalent eines tertiären Amins, beispielsweise eines der unmittelbar oben genannten, insbesondere Triethylamin durchgeführt. Wenn 1 Äquivalent des tertiären Amins für die Umsetzung mit einem Säureamidhalogenid vom Typ $III_b$ verwendet wird, wird das Reaktionsprodukt der Formel I als ein Salz erhalten, wenn dagegen mit 1 Aequivalent des tertiären Amins die Dialkylsulfatkomplexe und Thioamidalkylhalogenidkomplexe verwendet werden, resultiert das Reaktionsprodukt der Formel I in freier Form. Wenn zwei oder mehrere Äquivalente des tertiären Amins verwendet werden, resultiert immer die freie Form der Verbindung der Formel I, die gewünschtenfalls in ein Salz umgewandelt werden kann.

Die Reaktionszeit hängt von den Reaktionsteilnehmern, der Temperatur und den Lösungsmitteln ab, die bei dem Verfahren verwendet werden. Wenn im Ausgangsstoff der Formel III ein freies Carboxyl vorkommt, so wird, es vorzugsweise als ein Trimethylsilylester oder ein Dimethylsilyldiester vorübergehend geschützt und nach der Umsetzung leicht wieder abgespalten. Diese Umsetzung wird vorzugsweise mit einem Säureamidacetalreagens durchgeführt. Die Herstellung der Silylester ist aus der Literatur bekannt. Die Silylester der Endstoffe der Formel I werden vorzugsweise durch eine Hydrolyse oder eine Alkoholyse unter milden Bedingungen gespalten.

Im primären Reaktionsgemisch liegt der Endstoff praktisch nur in der stabileren 1,4-Hydrochinon-Form der SV-Reihe vor, in welcher er auch zweckmässig isoliert wird. Falls jedoch die Chinon-Form erwünscht ist, behandelt man das rohe Reaktionsgemisch, beziehungsweise eine isolierte Verbindung der SV-Reihe, mit einem Oxidationsmittel, insbesondere einem solchen, das für die Oxidation bekannter Hydrochinone üblich ist, z.B. Ammoniumpersulfat, Wasserstoffperoxid, Luftsauerstoff oder vorzugsweise Kaliumferricyanid; die Oxidation erfolgt vornehmlich unter basischen Bedingungen. Wünscht man eine Verbindung der S-Reihe in die Hydrochinon-Form (als ein Rifamycin-SV-Derivat) umzuwandeln, behandelt man die erstere mit einem üblichen Chinon-Reduktionsmittel, wie Hydrosulfit, Dithionit, Ferrocyanid oder insbesondere Ascorbinsäure oder Zink-Eisessig.

Säureadditionssalze werden aus entsprechenden basischen Verbindungen in konventioneller Weise erhalten, z.B. durch eine vorsichtige Behandlung dieser mit einer äquivalenten oder überschüssigen Menge der gewünschten Säure, vorzugsweise in einem inerten Lösungsmittel. Auch Salze mit Basen werden in konventioneller Weise durch die Behandlung von entsprechenden sauren Verbindungen mit den gewünschten Basen (insbesondere im Falle von Ammoniak und organischen Basen) oder mit den entsprechenden Metallhydroxiden oder vorzugsweise Carbonaten oder Hydrocarbonaten erhalten. Innere Salze werden vorzugsweise durch übliches acidobasisches Titrieren zum Neutralpunkt bzw. zum isoelektrischen Punkt gebildet.

Die Erfindung betrifft auch diejenigen Ausführungsformen des obigen Verfahrens, bei denen ein Ausgangsstoff unter den Reaktionsbedingungen gebildet oder in Form eines Salzes eingesetzt wird, oder bei denen man von einer auf irgendeiner Stufe als Zwischenprodukt erhältlichen Verbindung ausgeht und die fehlenden Schritte durchführt.

So kann man z.B. die beiden Stufen des erfindungsgemässen Verfahrens in einer einzigen Operation durchführen, indem man z.B. ein Gemisch einer 3-Aminorifamycin-Verbindung der Formel II und eines reaktionsfähigen Derivats eines N,N-disubstituierten Formamids der oben charakterisierten Formel III in einem aprotischen organischen Lösungsmittel, z.B. einem der oben genannten, mit Ammoniakgas sättigt, oder insbesondere indem man mit dem letztgenannten Reagens eine Verbindung der oben charakterisierten Formel IV behandelt, welche unmittelbar in derselben Reaktionslösung durch die Einwirkung von Ammoniak auf den 3-Aminorifamycin-Derivat der Formel II entstanden ist.

Man kann aber auch das verwendete reaktionsfähige Derivat des Formamids der Formel III aus seinen Bildungskomponenten direkt in situ, d.h. im Reaktionsgemisch, bilden. Ein derartiges reaktionsfähiges Derivat wird beispielsweise in situ erhalten, indem ein Formamid der Formel III mit einem 1,1-Dihalogendiniederalkylether, vorzugsweise 1,1-Dichlordimethylether, in Gegenwart einer tertiären organischen Base in Reaktion mit einer 3-Aminorifamycin-Verbindung der Formel II eingesetzt wird. Die Umsetzung kann ohne Isolieren des bei dieser Arbeitsweise gebildeten Zwischenprodukts durchgeführt werden. (Bei dem handelt es sich höchst wahrscheinlich um ein Methoxymethylenderivat der Verbindung der Formel III, welche dem oben beschriebenen Typ der Formel $III_c$, jedoch mit Halogen als Anion, entspricht). Die Umsetzungen werden unterhalb oder bei Raumtemperatur und in Gegenwart eines inerten Lösungsmittels, beispielsweise Chloroform oder Methylenchlorid, durchgeführt. Ein reaktionsfähiges Formamid-Derivat kann in situ erhalten werden, indem ein sekundäres Amin der Formel Am-H, worin Am die oben genannte Bedeutung hat, mit einem Niederalkyl-orthoformiat oder -orthothioformiat der Formel $CH(YR^4)_3$, worin Y und $R^4$ die oben genannten Bedeutungen haben, z.B. Methylorthoformiat oder Ethylorthoformiat, und mit einer 3-Aminorifamycin-Verbindung der Formel II in Gegenwart von Bortrifluoridetherat (oder einem analogen stark sauren Katalysator vom Typ Lewis-Säure) eingesetzt wird. Beim intermediär gebildeten

Reagens handelt es sich sehr wahrscheinlich um ein Formamid-diniederalkylacetal der Formel $III_a$ oder ein Iminoethersalz vom Typ der Formel $III_c$. Die Umsetzungen werden unterhalb oder bei Raumtemperatur und in Gegenwart eines inerten Lösungsmittels, beispielsweise Chloroform oder Methylenchlorid durchgeführt.

Die Erfindung betrifft auch ein Verfahren, gemäss dem man die Verbindungen der Formel IA oder IB dadurch herstellt, dass man ein 3-Aminorifamycin-S-4-imin der Formel

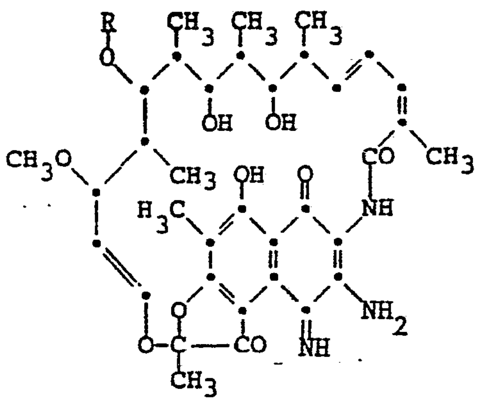

(IV)

worin R die oben genannte Bedeutung hat, oder ein Salz davon, mit einem reaktionsfähigen Derivat eines N,N-disubstituierten Formamids oder Thioformaids der Formel

Am-CH=Y (III)

worin Y Sauerstoff oder Schwefel ist und Am die eingangs definierte Bedeutung hat, umsetzt und gewünschtenfalls eine erhaltene Verbindung des SV-Typs zu einer Verbindung des S-Typs oxidiert oder eine erhaltene Verbindung des S-Typs zu einer Verbindung des SV-Typs reduziert und/oder eine erhaltene freie Verbindung mit salzbildenden Eigenschaften in ein entsprechendes Salz oder ein erhaltenes Salz in die entsprechende freie Verbindung umwandelt. - Das erfindungsgemässe Verfahren wird in der oben angegebenen Weise durchgeführt. Die beiden Ausgangsstoffe der Formel IV, d.h. das 3-Aminorifamycin S-4-imin und sein 25-Desacetylderivat sind bekannt, die reaktionsfähigen Derivate eines Formamids der Formel III sind die oben beschriebenen, insbesondere diejenigen der Formeln IIIa - IIIc.

Ebenso betrifft die Erfindung auch das Verfahren, gemäss dem man die Verbindungen der Formel IA oder IB dadurch herstellt, dass man ein N',N'-disubstituiertes 3-Aminomethylenaminorifamycin-Derivat (3-Aminorifamycin-formamidin) der Formel

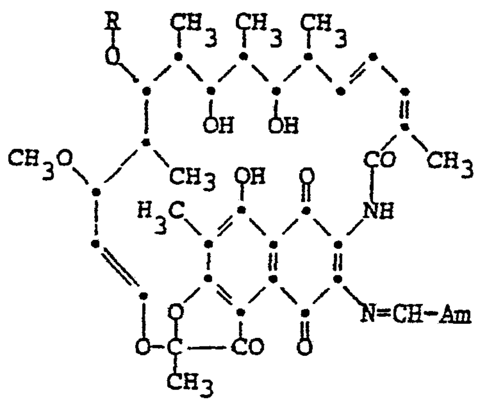

(V)

worin R und Am die oben genannten allgemeinen und bevorzugten Bedeutungen haben oder ein Salz davon, mit Ammoniak behandelt und gewünschtenfalls eine erhaltene Verbindung des SV-Typs zu einer Verbindung des S-Typs oxidiert oder eine erhaltene Verbindung des S-Typs zu einer Verbindung des SV-Typs reduziert und/oder eine erhaltene freie Verbindung mit salzbildenden Eigenschaften in ein entsprechendes Salz oder ein erhaltenes Salz in die entsprechende freie Verbindung umwandelt. Wenn erwünscht ist, von einem Formamidin der SV-Reihe auszugehen, so wird dieses vorher zur entsprechenden Verbindung der S-Reihe oxidiert.

Die Zwischenprodukte der oben definierten Formel V und ihre Analogen der SV-Reihe der Formel

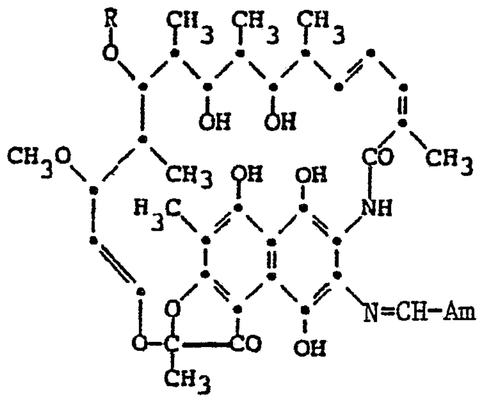

(VI)

worin R und Am die obgenannten allgemeinen und bevorzugten Bedeutungen haben, sowie ihre Salze, sind neu und gehören auch zum Gegenstand der vorliegenden Erfindung. Sie werden durch die oben eingehend beschriebene Behandlung der entsprechenden 3-Aminorifamycin S-Verbindung der Formel II (auch in Form eines Säureadditionssalzes davon), bzw. eines entsprechenden Analogen davon der SV-Reihe, mit einem oben definierten reaktionsfähigen Derivat eines N,N-disubstituierten Formamids oder Thioformamids der oben angegebenen Formel III, insbesondere einem Derivat der oben definierten Formeln IIIa - IIIc, hergestellt, und geünschtenfalls eine erhaltene Verbindung des SV-Typs zu einer Verbindung des S-Typs oxidiert oder eine erhaltene Verbindung des S-Typs zu einer Verbindung des SV-Typs reduziert und/oder eine erhaltene freie Verbindung mit salzbildenden Eigenschaften in ein entsprechendes Salz oder ein erhaltenes Salz in die entsprechende freie Verbindung umwandelt. Auch dieses Herstellungsverfahren gehört zum Gegenstand der vorliegenden Erfindung.

Die neuen erfindungsgemässen Verbindungen der Formel I zeichnen sich vor allem durch ihre wertvollen pharmakologischen Eigenschaften aus: so besitzen sie gemäss den Resultaten in vitro antibiotische, insbesondere antibakterielle Eigenschaften, wie z.B. einerseits gegen grammpositive Kokken, wie Staphylococcus aureus, (im getesteten Konzentrationsbereich 0,005 bis 128 γ/ml), andererseits gegen grammnegative Stäbchenbakterien, wie Enterobacteriaceae, z.B. Escherichia coli, Pseudomonas aeruginosa und Proteus morganii (im getesteten Konzentrationsbereich von 1 bis 64 γ/ml), sowie gegen Mycobacterium tuberculosis (im getesteten Konzentrationsbereich von 0,48 bis 1,9 γ/ml). Besonders hervorzugheben als Wirkstoffe sind 4-Dimethylamino-, 4-(4-Methylpiperazinyl)-, 4-(4-Isobutylpiperazinyl)-, 4-Morpholino- und 4-Pyrrolidinyl-imidazolo[4,5-c]rifamycin SV, die alle eine deutliche Hemmwirkung auf Staphylococcus aureus bereits bei oder unterhalb der genannten untersten Konzentrationsgrenze aufweisen und auch die Enterobacterien im angegebenen Konzentrationsbereich wirksam hemmen. (Für die Testresultate siehe auch Tabelle 1.)

Auch die neuen erfindungsgemässen Verbindungen der Formeln V und VI zeichnen sich durch analoge wertvolle pharmakologische Eigenschaften aus, d.h. sie besitzen gemäss den Resultaten in vitro antibiotische, insbesondere antibakterielle Eigenschaften, die sich bezüglich des Anwendungsbereichs sowie der getesteten effektiven Konzentrationen den Verbindungen der Formel I praktisch gleichen. Besonders hervorzuheben als Wirkstoffe sind 3-(4-Methyl- und 4-Phenyl-piperazinyl)-, 3-Thiomorpholino- und insbesondere 3-Indolinylmethylenaminorifamycin S, die alle eine deutliche Hemmwirkung auf Staphylococcus aureus bereits bei der genannten untersten Konzentrationsgrenze aufweisen und auch die Enterobacterien im angegebenen Konzentrationsbereich wirksam hemmen. (Für die Testresultate siehe auch Tabelle 2.)

Die erfindungsgemässen Verbindungen der Formel I können auch als wertvolle Zwischenprodukte zur Herstellung anderer Rifamycin-Derivate, insbesondere solcher mit therapeutischer Anwendung, Verwendung finden. In Anlehnung an die oben erwähnten günstigen pharmakologischen Eigenschaften umfasst die Erfindung auch die Verwendung der erfindungsgemässen Wirkstoffe, allein oder in Kombination mit einem anderen Antibiotikum oder Chemotherapeutikum, als ein Mittel zur Bekämpfung von Infektionen, insbesondere solchen, die durch Bakterien oder Kokken, z.B. die genannten, hervorgerufen werden, und zwar sowohl als Heilmittel, wie auch als Desinfektionsmittel. Bei der Verwendung als Heilmittel wird der erfindungsgemässe Wirkstoff vorzugsweise in Form eines pharmazeutischen Präparats zusammen mit mindestens einem konventionellen pharmazeutischen Träger oder Hilfsstoff einem Warmblüter, vor allem Menschen, verabreicht. (Unter der Bezeichnung "Wirkstoff" oder "der erfindungsgemässe Wirkstoff" ist vorangehend und nachfolgend ein Rifamycin-Derivat der oben definierten Formel IA bzw. IB, und der Formeln V bzw. VI, sowie biologisch verträgliche Salze davon, zu verstehen.

Gegenstand der Erfindung sind auch pharmazeutische Zusammensetzungen enthaltend einen der oben definierten Wirkstoffe, insbesondere zusammen mit mindestens einem pharmazeutischen Trägermaterial, sowie Verfahren zu deren Herstellung auf nicht-chemischem Wege. Zwecks Herstellung pharmazeutischer Zusammensetzungen kann jede einzelne der erfindungsgemässen Verbindungen, vor allem eine der besonders hervorgehobenen, mit einem für die topische, enterale oder parenterale Applikation geeigneten anorganischen oder organischen Trägermaterial vermischt werden. Für dasselbe kommen solche Stoffe in Betracht, die mit der neuen Verbindung nicht reagieren, wie z.B. Gelatine, Milchzucker, Stärke, Magnesiumstearat, pflanzliche Oele, Benzylalkohol, oder andere Arzneimittelträger. Die pharmazeutischen Zusammensetzungen können zu pharmazeutischen Darreichungsformen, wie Tabletten, Dragées, Pulver, Suppositorien, oder in flüssiger Form zu Lösungen, Suspensionen, Emulsionen, Cremen oder Salben, verarbeitet werden. Gegebenenfalls sind diese sterilisiert und/oder enthalten Hilfsstoffe, wie Konservierungsmittel, Stabilisierungs-, Netz- oder Emulgiermittel. Sie können auch noch andere therapeutisch wertvolle Stoffe enthalten. Auch die Desinfektionsmittel können mit geeigneten Trägerstoffen, wie bekannt, vermischt werden.

Zur oralen Verabreichung verwendet man Tabletten, Steckkapseln oder Gelatinekapseln, die den Wirkstoff zusammen mit Verdünnungsmitteln, z.B. Laktose, Glucose, Saccharose, Mannit, Sorbit, Cellulose und/oder Glycin, und Schmiermitteln, z.B. Kieselerde, Talk, Stearinsäure oder Salze davon, wie Magnesium- oder Calciumstearat, und/oder Polyethylenglykol, aufweisen. Tabletten enthalten ebenfalls Bindemittel,

z.B. Magnesiumaluminiumsilikat, Stärken, wie Mais-, Weizen-, Reis- oder Pfeilwurzstärke, Gelatine, Tragant, Methylcellulose, Natriumcarboxymethylcellulose und/oder Polyvinylpyrrolidon, und, wenn erwünscht, Sprengmittel, z.B. Stärken, Agar, Alginsäure oder Salze davon, wie Natriumalginat, und/oder Brausemischungen, oder Absorptionsmittel, Farbstoffe, Geschmackstoffe und Süssmittel. Suppositorien sind in erster Linie Fettemulsionen oder -suspensionen.

Die pharmazeutischen Präparate können sterilisiert sein und/oder Hilfstoffe, z.B. Konservier-, Stabilisier-, Netz- und/oder Emulgiermittel, Löslichkeitsvermittler, und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Mischungs-, Lösungs-, oder Lyophilisierungsverfahren, hergestellt und enthalten von etwa 0,1 % - 100 %, insbesondere von etwa 1 % - etwa 50 %, Lyophilisate bis zu 100 % des Wirkstoffes.

Besonders bevorzugt vorliegen die oben charakterisierten pharmazeutischen Präparate in Form von Dosierungseinheiten. Mit dem Ausdruck "Dosierungseinheit" ist eine einheitliche, d.h. einzige Dosis gemeint, die eine antimikrobiell wirksame Menge des Wirkstoffes enthält, welche an einen Patienten verabreicht wird. Sie kann leicht gehandhabt und verpackt werden, wobei sie als eine physikalisch stabile Einheit entweder das aktive Material als solches oder eine Mischung davon mit einem festen pharmazeutischen Träger enthält. Die Dosierung der Wirkstoffe, z.B. der oben besonders hervorgehobenen, erfolgt im Prizip analog derjenigen von anerkannten Antibiotika vom Rifamycin-Typ; sie hängt jedoch auch einerseits von Spezies, Körpergewicht, Alter und individuellem Zustand des Warmblüters, andererseits von der Applikationsweise und insbesondere von der jeweiligen Empfindlichkeit des Krankheitserregers ab, die man im Routine-Test in bekannter Weise feststellen kann. Demnach ist Gegenstand der vorliegenden Erfindung auch die Auswahl einer Dosis der

erfindungsgemässen Verbindungen, die verabreicht werden kann, so dass die gewünschte Aktivität ohne gleichzeitige Nebeneffekte erreicht wird.

Die Erfindung betrifft auch eine Methode zur Tötung oder Wachstumsbehinderung (d.h. Inhibition) eines gegen mindestens einen der erfindungsgemässen Wirkstoffe empfindlichen Mikroorganismus, welche durch die Behandlung dieses Mikroorganismus oder eines durch diesen Mikroorganismus infizierten Mediums mit einer antimikrobiell wirksamen Dosis eines der erfindungemässen Wirkstoffe charakterisiert ist. - Unter der Bezeichnung "eine antimikrobiell wirksame Dosis" ist eine solche Menge des Wirkstoffes zu verstehen, die zu einer wirkungsvollen Inhibition des betreffenden zu behandelnden Mikroorganismus ausreicht.

Die Verbindungen werden zweckmässigerweise in Dosierungseinheiten verabreicht, die nicht weniger Substanz enthalten, als 0,025 bis 1 g des Wirkstoffes (als freie Base) und vorzugsweise 0,05 bis 0,5 g davon entspricht. Die Wirkstoffe können in Form einer Dosierungseinheit einmal oder mehrmals täglich in geeigneten Intervallen verabreicht werden, was jedoch immer von dem Zustand des Patienten abhängt. Eine tägliche Dosis beträgt somit vorzugsweise 0,2 bis 5,0 g der erfindungsgemässen Wirkstoffe, berechnet als freie Base.

Die nachfolgenden Beispiele illustrieren die obenbeschriebene Erfindung; sie sollen jedoch diese in ihrem Umfang in keiner Weise einschränken. Temperaturen werden in Celsiusgraden angegeben. Die Zusammensetzung von Lösungsmittelgemischen ist im Volumenverhältnis angegeben. Die Struktur der isolierten Verbindungen wird durch die massenspektrometrische Bestimmung des Molekularions, sowie durch die Elementaranalyse routinemässig kontrolliert.

Beispiel 1: 4-Dimethylamino-imidazolo[4,5-c]rifamycin SV.

Man versetzt eine Lösung von 1,5 g 3-Amino-4-iminorifamycin-S in 15 ml Tetrahydrofuran mit 2,0 g Dimethylformamid-dimethylacetal und lässt bei Raumtemperatur solange stehen, bis das eingesetzte Rifamycinderivat vollständig reagiert hat (Dünnschichtchromatographische Kontrolle an Kieselgel, Laufmittel Methylenchlorid-Aceton 9:1). Anschliessend wird das Reaktionsgemisch mit Essigsäureethylester aufgenommen, mit wässriger Zitronensäurelösung und Kochsalzlösung gewaschen. Nach dem Abdampfen des Lösungsmittels chromatographiert man den braungefärbten Rückstand an Kieselgel mit dem Laufmittel Methylenchlorid/Aceton 9:1, wobei raschwandernde, dunkelgefärbte Anteile entfernt werden. Man vereinigt die gelbgefärbten Fraktionen, dampft ein, und kristallisiert den Rückstand aus Diethylether/Aceton. Das Dimethylaminoimidazolorifamycin-SV wird in hellgelben Kristallen vom Smp. 180-185° (Zersetzung) erhalten. Im Massenspektrum erscheint das Molekülion bei m/z = 764. Berechnet für $C_{40}H_{52}N_4O_{11}$ = 764.

Beispiel 2: 4-Morpholino-imidazolo[4,5-c]rifamycin SV.

Eine Lösung von 1,42 g 3-Amino-4-iminorifamycin-S in 15 ml Tetrahydrofuran wird unter Stickstoff mit 0,5 ml N-Formylmorpholindimethylacetal und 2 ml Triethylamin versetzt und 24 Stunden bei Raumtemperatur stehen gelassen. Danach nimmt man das Reaktionsgemisch in Essigsäureethylester auf, wäscht nacheinander mit wässriger Zitronensäurelösung und Kochsalzlösung, und entfernt das Ethylacetat im Vakuum. Der Rückstand kristallisiert aus Ether/Aceton und liefert Morpholinoimidazolorifamycin-SV als gelbe Kristalle, Smp. 192-193° (Zersetzung). Im Massenspektrum wird das Molekülion der Verbindung bei m/z = 806 gefunden (Berechnet für $C_{42}H_{54}N_4O_{12}$ = 806).

Beispiel 3: 4-(4-Methylpiperazinyl)-imidazolo[4,5-c]rifamycin SV.

Man setzt einer Lösung von 2,0 g 3-Amino-4-iminorifamycin-S in 25 ml Tetrahydrofuran 2,5 g 1-Formyl-4-methylpiperazin-dimethylacetal zu und lässt bis zum völligen Umsatz des eingesetzten Rifamycinderivates bei Ramtemperatur stehen.

Dann nimmt man das Reaktionsgemisch in Ethylacetat auf, wäscht mit wässriger Zitronensäurelösung sowie mit Kochsalzlösung und dampft ein. Der Rückstand wird an Kieselgel mit Methylenchlorid/Methanol 95:5 chromatographiert. Nach dem Entfernen raschlaufender dunkelgefärbter Stoffe werden die danach folgenden gelbgefärbten Eluate gesammelt und eingedampft. Durch Kristallisation aus Ether/Aceton erhält man das N-Methylpiperazinylimidazolo-rifamycin-SV in gelben Kristallen vom Smp. 185-187° (Zersetzung).
Das 100 MHz-Protonenresonanzspektrum der Verbindung in $CDCl_3$ zeigt neben den üblichen Rifamycinsignalen zusätzliche Signale bei 3,5-4,0 ppm ($CH_2$ neben N;M) sowie bei 2,40 ppm ($NCH_3$; S).

Beispiel 4: 4-(4-Isobutylpiperazinyl)-imidazolo[4,5-c]rifamycin SV.
Man lässt ein Reaktionsgemisch aus 2,5 g 3-Aminorifamycin-S, 20 ml Tetrahydrofuran, 1,5 g 1-Formyl-4-isobutylpiperazindimethylacetal und 1,5 g Triethylamin 24 Stunden bei Raumtemperatur stehen. Danach dampft man im Vakuum zur Trockne ein. Der Rückstand, welcher rohes 3-(4-Isobutylpiperazinyl)methyleniminorifamycin S mit nur geringen Verunreinigungen enthält, wird in 20 ml Tetrahydrofuran gelöst. In die Lösung wird bei Raumtemperatur während 30 Minuten Ammoniakgas zur Sättigung geleitet. Anschliessend dampft man die Lösung ein und chromatographiert den gelbbraunen Rückstand an Polyamid (Woehn) mit Ether/Methylenchlorid 5:1 als Laufmittel. Es bilden sich 3 Zonen: Das Eluat der langsamsten, gelbgefärbten Zone enthält dann das gewünschte 4-(4-Isobutylpiperazinyl)-imidazolorifamycin-SV, das im Massenspektrum ein Molekülion bei m/z = 861 zeigt.
(Berechnet für $C_{46}H_{63}N_5O_{11}$ = 861).

Beispiel 5: 4-(4-Cyclohexylmethyl-piperazinyl)-imidazolo[4,5-c]rifamycin SV
Man versetzt eine Lösung von 1,5 g 3-Amino-4-imino-rifamycin-S in 15 ml Tetrahydrofuran mit 3,0 g 1-Formyl-4-cyclohexylmethylpiperazindimethylacetal und lässt bei Raumtemperatur 8 Stunden stehen. Danach

wird das Reaktionsgemisch in Ethylacetat aufgenommen, die organische Lösung nacheinander mit wässriger Zitronensäurelösung sowie mit Kochsalzlösung gewaschen, getrocknet und eingedampft. Der Rückstand liefert bei der Chromatographie an Kieselgel mit Methylenchlorid/Methanol 97:3 als Laufmittel das Cyclohexylmethylpiperazinyl-imidazolorifamycin-SV als Hauptprodukt. Die Verbindung zeigt im Massenspektrum ein Molekülion bei m/z = 901 (Berechnet für $C_{49}H_{67}N_5O_{11}$ = 901). UV -Spektrum : (0.01-N alkoholische Salzsäure) $\lambda_{max}$ (nm)/$\varepsilon$ : 224/38400, 228/39200, 231/38600,245 (Schulter), 304/26400, 420/11200.

Beispiel 6: 4-Diethylamino-imidazolo[4,5-c]rifamycin SV(6).
In die Lösung von 380 mg Diethylaminomethylenamino-rifamycin S in 15 ml Tetrahydrofuran wird während 15 Minuten bei Raumtemperatur Ammoniakgas eingeleitet. Die Lösung wird über Nacht stehengelassen und anschliessend zur Trockne eingedampft. Der gelbbraune Rückstand wird an Silicagel chromatographiert; durch Eluieren mit Gemischen von Chloroform mit ansteigendem (2-10 %) Anteil Methanol resultiert die Titelverbindung ($C_{42}H_{56}N_4O_{11}$) als gelbes, amorphes Pulver.

Die Herstellung des Ausgangsstoffes wird im Beispiel 79 beschrieben.

Beispiel 7: 4-Dipropylamino-imidazolo[4,5-c]rifamycin SV (7).
In analoger Weise, wie in Beispiel 1 beschrieben, wird aus 4,0 g 3-Amino-4-iminorifamycin S in 50 ml Tetrahydrofuran und 4,0 g N,N-Dipropylformamid-dimethylacetal das obgenannte Imidazolorifamycin-SV (7) ($C_{44}H_{60}N_4O_{11}$, MG = 820) hergestellt. Orangefarbene Kristalle aus Ether/Methylenchlorid, die bei 175-185° (Zersetzung) schmelzen; MG gefunden: 820.

Das als Reagens benötigte N,N-Dipropylformamid-dimethylacetal wird in analoger Weise wie im Beispiel 81 aus 10 g Dipropylamin und 20 g N,N-Dimethylformamid-dimethylacetal erhalten.

Beispiel 8: 4-(N-Isopropyl-N-methylamino)-imidazolo[4,5-c]rifamycin SV (8)

In analoger Weise, wie in Beispiel 1 beschrieben, wird aus 4,0 g 3-Amino-4-iminorifamycin-S in 50 ml Tetrahydrofuran und 4,0 g N-Isopropyl-N-methylformamid-dimethylacetal das 4-(N-Isopropyl-N-methylamino)-imidazolo[4,5-c]rifamycin-SV (8) ($C_{42}H_{56}N_4O_{11}$, MG = 792) hergestellt.

Der als Reagens benötigte N-Isopropyl-N-methylformamid-dimethylacetal wird in analoger Weise wie im Beispiel 80 bzw. 81 aus 10 g N-Isopropyl-N-methylamin und 20 g N,N-Dimethylformaid-dimethylacetal erhalten.

Beispiel 9: 4-(N-Butyl-N-methylamino)-imidazolo[4,5-c]rifamycin SV (9)

In analoger Weise, wie in Beispiel 1 beschrieben, wird aus 4,0 g 3-Amino-4-iminorifamycin S in 50 ml THF und 4,0 g N-Butyl-N-methylformamid-dimethylacetal 4-(N-Butyl-N-methylamino)-imidazolo[4,5-c]-rifamycin SV (9) ($C_{43}H_{48}N_4O_{11}$, MG = 806) hergestellt. MG gefunden: 806.

Das als Reagens benötigte N-Butyl-N-methylformamid-dimethylacetal wird in analoger Weise wie im Beispiel 80 bzw. 81 aus 10 g Butylmethylamin und 20 g N,N-Dimethylformamid-dimethylacetal erhalten.

Beispiel 10: 4-(N-Cyclopentyl-N-methylamino)-imidazolo[4,5-c]rifamycin SV (10)

In analoger Weise, wie in Beispiel 1 beschrieben, wird aus 4,0 g 3-Amino-4-iminorifamycin S in 50 ml THF und 4,0 g N-Cyclopenyl-N-methylformamid-dimethylacetal 4-(N-Cyclopentyl-N-methylamino)-imidazolo [4,5-c]rifamycin SV (10) ($C_{44}H_{58}N_4O_{11}$, MG = 818) hergestellt. MG gefunden: 818.

Das als Reagens benötigte N-Cyclopentylformamid-dimethylacetal wird in analoger Weise wie im Beispiel 80 bzw. 81 aus 10 g Cyclopentylmethylamin und 20 g N,N-Dimethylformamid-dimethylacetal erhalten.

Beispiel 11: 4-(N-Cyclopropylmethyl-N-propylamino)-imidazolo[4,5-c]-rifamycin SV (11)

In analoger Weise, wie in Beispiel 1 beschrieben, wird aus 4,0 g 3-Amino-4-iminorifamycin S in 50 ml THF und 4,0 g N-Cyclopropylmethyl-N-propylformamid-dimethylacetal 4-(N-Cyclopropylmethyl-N-propylamino)-imidazolo[4,5-c]rifamycin SV (11) ($C_{45}H_{60}N_{4}O_{11}$, MG = 832) hergestellt. Es bildet aus Ether gelbe Kristalle vom Smp. 223-224°. MG gefunden: 832.

Das als Reagens benötigte N-Cyclopropylmethyl-N-propylformamid-dimethylacetal wird in analoger Weise, wie in Beispielen 80-82 beschrieben, aus 10 g N-Cyclopropylmethyl-N-propylamin und 20 g N,N-Dimethylformamid-dimethylacetal erhalten.

Beispiel 12: 4-(N-Benzyl-N-methylamino)-imidazolo[4,5-c]rifamycin SV (12)

In analoger Weise,wie in Beispiel 1 beschrieben, werden aus 0,7 g 3-Amino-4-iminorifamycin S in 10 ml THF und 0,425 g N-Benzyl-N-methylformamid-dimethylacetal 0,155 g 4-(N-Benzyl-N-methylamino)-imidazolo-[4,5-c]rifamycin SV (12) ($C_{47}H_{56}N_{4}O_{11}$, MG = 852) hergestellt.

Die Herstellung des Reagens ist im Beispiel 80 beschrieben.

Beispiel 13: 4-[N-methyl-N-(1-methyl-4-piperidyl)-amino]-imidazolo-[4,5-c]rifamycin SV (13)

In analoger Weise, wie in Beispiel Nr. 1 beschrieben, wird aus 4,0 g 3-Amino-4-iminorifamycin S in 50 ml THF und 4,0 g N-Methyl-N-(1-methyl-4-piperidyl)-formamid-dimethylacetal 4-[N-Methyl-N-(1-methyl-4-piperidyl)-amino]-imidazolo[4,5-c]rifamycin SV (13) ($C_{45}H_{61}N_{5}O_{11}$, MG = 847) hergestellt, MG gefunden: 847.

Das als Reagens benötigte N-Methyl-N-(1-methyl-4-piperidyl)-formamid-dimethylacetal wird in analoger Weise, wie im Beispiel 82 beschrieben aus 10 g N-Methyl-N-(1-methyl-4-piperidyl)-amin und 20 g N,N-Dimethyl-formamid-dimethylacetal erhalten.

Beispiel 14: 4-[N-Ethyl-N-(2-hydroxyethyl)-amino]-imidazolo[4,5-c]rifamycin SV (14)

In analoger Weise, wie in Beispiel 1 beschrieben, wird aus 4,0 g 3-Amino-4-iminorifamycin S in 50 ml THF und 4,0 g N-Ethyl-N-(2-hydroxyethyl)-formamid-dimethylacetal das gewünschte Imidazolorifamycin-SV (14) ($C_{42}H_{56}N_4O_{12}$, MG = 808) hergestellt. Gelbe Prismen aus Methylenchlorid/Ether, die bei 192-193° schmelzen (Zersetzung).
MG gefunden: 808.

Das als Reagens benötigte N-Ethyl-N-(2-hydroxyethyl)-formamid-dimethylacetal wird in analoger Weise, wie in Beispielen 80-82 beschrieben, aus 10 g N-Ethylethanolamin und 20 g N,N-Dimethylformamid-dimethylacetal erhalten.

Beispiel 15: 4-[N-(2,2-Dimethoxyethyl)-N-methylamino]-imidazolo[4,5-c]-rifamycin SV (15)

In analoger Weise, wie in Beispiel 1 beschrieben, wird aus 4,0 g 3-Amino-4-iminorifamycin S in 50 ml THF und 4,0 g N-Methyl-N-(2,2-dimethoxyethyl)-formamid-dimethylacetal das gewünschte Imidazolo-rifamycin SV (15) ($C_{43}H_{58}N_4O_{13}$, MG = 838) vom Smp. 162°C (Zersetzung) hergestellt. MG gefunden ($^m/2$ von $TMS_4$-derivat): 838.

Das als Reagens benötigte N-Methyl-N-(2,2-dimethoxyethyl)formamid-dimethylacetal wird in analoger Weise, wie in Beispielen 80-82 beschrieben, aus 10 g Methylaminoacetaldehyddimethylacetal und 20 g N,N-Dimethylformamid-dimethylacetal erhalten.

Beispiel 16: 4-[N-Di-(2-methoxyethyl)-amino]-imidazolo[4,5-c]rifamycin SV (16)

In analoger Weise, wie in Beispiel 1 beschrieben, wird aus 4,0 g 3-Amino-4-iminorifamycin S in 50 ml THF und 4,0 g N,N-Di-(2-methoxyethyl)-formamid-dimethylacetal 4-[Di-(2-methoxyethyl)-amino]-imidazolo-[4,5-c]rifamycin SV (16) ($C_{44}H_{60}N_4O_{13}$, MG = 852) hergestellt.
MG gefunden: 852.

Das als Reagens benötigte N,N-Di-(2-methoxyethyl)-formamid-dimethylacetal wird in analoger Weise wie im Beispiel 80 aus 10 g Di-(2-methoxyethyl)-amin und 20 g N,N-Dimethylformamid-dimethylacetal erhalten.

Beispiel 17: 4-[N-Methyl-N-(2-dimethylaminoethyl)-amino]-imidazolo-[4,5-c]rifamycin SV (17)

In analoger Weise, wie in Beispiel 1 beschrieben, wird aus 4,0 g 3-Amino-4-iminorifamycin S in 50 ml THF und 4,0 g N-Methyl-N-(2-dimethylaminoethyl)-formamid-dimethylacetal 4-[Methyl-(2-dimethylaminoethyl)-amino]-imidazolo[4,5-c]rifamycin SV (17) ($C_{43}H_{59}N_5O_{11}$, MG = 821) hergestellt. Aus Methylenchlorid dreieckige Kristallplättchen, die bei 187-188° schmelzen (Zersetzung). MG gefunden: 821.

Das als Reagens benötigte N-Methyl-N-(2-dimethylaminoethyl)-formamid-dimethylacetal wird in analoger Weise wie in Beispielen 80-82 aus 10 g N,N,N'-Trimethylethylendiamin und 20 g N,N-Dimethylformamid-dimethylacetal erhalten.

Beispiel 18: 4-[N-Ethyl-N-(2-diethylaminoethyl)-amino]-imidazolo-[4,5-c]rifamycin SV (18)

In analoger Weise, wie in Beispiel 1 beschrieben, wird aus 4,0 g 3-Amino-4-iminorifamycin S in 50 ml THF und 4,0 g N-Ethyl-N-diethylaminoethyl-formamid-dimethylacetal das gewünschte Imidazolorifamycin SV (18) ($C_{46}H_{65}N_5O_{11}$, MG = 863) vom Smp. 173-174°C (Zersetzung) hergestellt.

Das als Reagens benötigte N-Ethyl-N-diethylaminoethylformamid-dimethylacetal wird in analoger Weise wie in Beispielen 80-82 aus 10 g N,N',N'-Triethylethylendiamin und 20 g N,N-Dimethylformamid-dimethylacetal erhalten.

Beipiel 19: 4-Pyrrolidinyl-imidazolo[4,5-c]rifamycin SV (19)

Man erhitzt 20 g Dimethylformamiddimethylacetal mit 10 g Pyrrolidin während vier Stunden auf 100°. Danach setzt man 100 ml Toluol zu und

dampft im Wasserstrahlvakuum bei 50° ein. Der Rückstand wird ohne weitere Reinigung als rohes Reagens Dimethoxypyrrolidinomethan verwendet. Ein Gemisch von 4 g Dimethoxypyrrolidinomethan, 50 ml Tetrahydrofuran und 4 g 3-Amino-4-iminorifamycin S werden sechs Stunden bei 25° stehen gelassen, wobei die anfangs tiefrote Farbe des Reaktionsgemisches allmählich nach gelbbraun umschlägt. Anschliessend säuert man mit wässriger Zitronensäurelösung an und nimmt das Reaktionsprodukt mit Methylenchlorid auf. Das Reaktionsprodukt reinigt man durch Chromatographie an Kieselgel. Mit einem Gemisch von Ethylacetat mit 10 % Methanol als Elutionsmittel lässt sich gereinigtes Pyrrolidinyl-imidazolo-rifamycin-SV (19) erhalten, welches aus Aceton/Ether gelbe Kristalle vom Smp. 200° bildet. MW gefunden: 790 (MS als $TMS_4$-Derivat $^m/2$ 1078) berechnet für $C_{12}H_{54}N_4O_{11}$ MW = 790).

Beispiel 20: 4-Indolinyl-imidazolo[4,5-c]rifamycin SV (20)

In analoger Weise, wie in Beispiel 1 beschrieben, werden aus 2,0 g 3-Amino-4-iminorifamycin S in 100 ml THF und 3,26 g N-Formylindolin-dimethylacetal 1,1 g 4-Indolinyl-imidazolo[4,5-c]rifamycin-SV (20) ($C_{46}H_{53}N_4O_{11}$, MG = 837) hergestellt.

Das als Reagens benötigte N-Formylindolin-dimethylacetal wird gemäss Beispiel 82 hergestellt.

Beispiel 21: 4-Piperidino-imidazolo[4,5-c]rifamycin SV (21)

In analoger Weise, wie in Beispiel 1 beschrieben, wird aus 4,0 g 3-Amino-4-Iminorifamycin S in 50 ml THF und 4,0 g N-Formylpiperidin-dimethylacetal 4-Piperidinyl-imidazolo [4,5-c] rifamycin-SV (21) ($C_{43}H_{56}N_4O_{11}$, MG = 802). Gelbe Kristalle vom Smp. 190°, (Zersetzung) MG gefunden: 802.

Das als Reagens benötigte N-Formylpiperidin-dimethylacetal wird gemäss Beispiel 83 hergestellt.

Beispiel 22: 4-(4-Ethoxycarbonyl-1-piperidyl)-imidazolo[4,5-c]rifamycin SV (22)

In analoger Weise, wie in Beispiel 1 beschrieben, wird aus 4,0 g 3-Amino-4-iminorifamycin S in 50 ml THF und 4,0 g N-Formyl-4-ethoxycarbonylpiperidin-dimethylacetal 4-(4-Ethoxycarbonylpiperidyl)-imidazolo-[4,5-c] rifamycin SV (22) ($C_{46}H_{60}N_4O_{13}$, MG = 876) hergestellt.

Das als Reagens benötigte N-Formyl-4-ethoxycarbonylpiperidin-dimethylacetal wird in analoger Weise wie in Beispielen 80-82 aus 10 g 4-Ethoxycarbonylpiperidin und 20 g N,N-Dimethylformamid-dimethylacetal erhalten.

Beispiel 23: 4-(4-Dimethylamino-1-piperidyl)-imidazolo[4,5-c]rifamycin SV (23)

In analoger Weise wie in Beispiel 1 beschrieben, wird aus 4,0 g 3-Amino-4-iminorifamycin S in 50 ml THF und 4,0 g N-Formyl-4-dimethylaminopiperidin-dimethylacetal 4-(4-Dimethylaminopiperidyl)-imidazolo-[4,5-c]rifamycin SV (23) ($C_{45}H_{61}N_5O_{11}$, MG = 847) hergestellt. Aus Methanol-Ether gelbe Kristalle, die sich ab ~140° zersetzen. MG gefunden ($^m/2$ des $TMS_5$-derivats): 847.

Das als Reagens benötigte N-Formyl-4-dimethylaminopiperidin-dimethylacetal wird in analoger Weise wie in Beispielen 80-82 aus 10 g 4-Dimethylaminopiperidin und 20 g N,N-Dimethylformamid-dimethylacetal erhalten.

Beispiel 24: 4-(4-Piperidino-1-piperidyl-)-imidazolo[4,5-c]rifamycin SV (24)

In analoger Weise, wie in Beispiel 1 beschrieben, wird aus 4,0 g 3-Amino-4-iminorifamycin S in 50 ml THF und 4,0 g N-Formyl-4-piperidinopiperidin-dimethylacetal 4-(4-Piperidinopiperidyl-imidazolo[4,5-c]-rifamycin SV (24) ($C_{48}H_{65}N_5O_{11}$, MG = 887) hergestellt. Aus Methanol-Methylenchlorid gelbe Kristalle, die sich ab 190° zersetzen.

Das als Reagens benötigte N-Formyl-4-piperidinopiperidin-dimethylacetal wird in analoger Weise wie in Beispielen 80-82 aus 10 g 4-Piperidinopiperidin und 20 g N,N-Dimethylformamid-dimethylacetal erhalten.

Beispiel 25: 4-[8-Aza-1,4-dioxaspiro-(4,5)-8-decyl]-imidazolo[4,5-c]-rifamycin SV (25)

In analoger Weise, wie in Beispiel 1 beschrieben, wird aus 4,0 g 3-Amino-4-iminorifamycin S in 50 ml THF und 4,0 g N-Formyl-(4-piperidonethylenketal)-dimethylacetal die Titelverbindung (25) ($C_{45}H_{58}N_4O_{13}$, MG = 862.) hergestellt. MG gefunden (MS des $TMS_5$-derivats): 862.

Das als Reagens benötigte N-Formyl-(4-piperidonethylenketal)-dimethylacetal wird gemäss Beispiel 85 hergestellt.

Beispiel 26: 4-(3-Diethylaminocarbonyl-1-piperidyl)-imidazolo[4,5-c]-rifamycin SV (26)

In analoger Weise, wie in Beispiel 1 beschrieben, wird aus 4,0 g 3-Amino-4-iminorifamycin S in 50 ml THF und 4,0 g N-Formyl-3-diethylaminocarbonylpiperidin-dimethylacetal 4-(3-Diethylaminocarbonylpiperidyl)-imidazolo [4,5-c] rifamycin SV (26) ($C_{48}H_{65}N_5O_{12}$, MG = 903), hergestellt.

Das als Reagens benötigte N-Formyl-3-diethylaminocarbonylpiperidin-dimethylacetal wird in analoger Weise wie in Beispielen 80-82 aus 10 g Piperidin-3-carbonsäurediethylamid und 20 g N,N-Dimethylformamid-dimethylacetal erhalten.

Beispiel 27: 4-(1-Perhydroazepinyl)-imidazolo[4,5-c]rifamycin SV (27)

In analoger Weise, wie in Beispiel 1 beschrieben, wird aus 4,0 g 3-Amino-4-iminorifamycin S in 50 ml THF und 4,0 g N-Formyl-perhydroazepin-dimethylacetal das gewünschte Imidazolorifamycin-SV (27) $C_{44}H_{58}N_4O_{11}$, MG = 818) hergestellt.

Das als Reagens benötigte 1-Formyl-perhydroazepin-dimethylacetal wird in analoger Weise wie in Beispielen 80-82 aus 10 g Perhydroazepin und 20 g N,N-Dimethylformamid-dimethylacetal erhalten.

Beispiel 28: 4-(1-Perhydroazocinyl)-imidazolo[4,5-c]rifamycin SV (28)

In analoger Weise, wie in Beispiel 1 beschrieben, wird aus 4,0 g 3-Amino-4-iminorifamycin S in 50 ml THF und 4,0 g N-Formyl-perhydroazocin-dimethylacetal die Titelverbindung (28) ($C_{45}H_{60}N_4O_{11}$, MG = 832) vom Smp. 180°C (Zersetzung) hergestellt. MG gefunden: 832.

Das als Reagens benötigte N-Formyl-perhydroazocin-dimethylacetal wird in analoger Weise wie in Beispielen 80-82 aus 10 g Perhydroazocin und 20 g N,N-Dimethylformamid-dimethylacetal erhalten.

Beispiel 29: 4-(2,6-Dimethyl-morpholino)-imidazolo[4,5-c]rifamycin SV (29)

In analoger Weise, wie in Beispiel 1 beschrieben, wird aus 4,0 g 3-Amino-4-iminorifamycin S in 50 ml THF und 4,0 g 4-Formyl-2,6-dimethylmorpholin-dimethylacetal 4-(2,6-Dimethyl-4-morpholinyl)-imidazolo-[4,5-c]rifamycin SV (29) ($C_{44}H_{58}N_4O_{12}$, MG = 834) hergestellt. Gelbe Kristalle, die sich ab 240° allmählich zersetzen, ohne zu schmelzen. MG gefunden: 834.

Das als Reagens benötigte 4-Formyl-2,6-dimethylmorpholin-dimethylacetal wird in analoger Weise wie in Beispielen 80-82 aus 10 g 2,6-Dimethylmorpholin und 20 g N,N-Dimethylformamid-dimethylacetal erhalten.

Beispiel 30: 4-Thiomorpholino-imidazolo[4,5-c]rifamycin SV (30)

In analoger Weise, wie in Beispiel 1 beschrieben, werden aus 1,4 g 3-Amino-4-iminorifamycin S in 100 ml THF und 1,06 g N-Formylthiomorpholin-dimethylacetal 0,45 g 4-Thiomorpholino-imidazolo[4,5-c]rifamycin SV (30) ($C_{42}H_{54}N_4SO_{11}$, MG = 822) hergestellt.

Die Herstellung von N-Formylthiomorpholin-dimethylacetal wird im

Beispiel 87 beschrieben.

Beispiel 31: 4-(4-Ethylpiperazinyl)-imidazolo[4,5-c]rifamycin SV (31)

In analoger Weise, wie in Beispiel 1 beschrieben, werden aus 2,0 g 3-Amino-4-iminorifamycin S in 100 ml THF und 3,18 g 1-Formyl-4-ethylpiperazin-dimethylacetal 0,95 g 4-(4-Ethylpiperazinyl)-imidazolo-[4,5-c]rifamycin SV (31) $C_{44}H_{59}N_5O_{11}$, MG = 833) hergestellt.

Das als Reagens benötigte 1-Formyl-4-ethylpiperazin-dimethylacetal (Sdp.: 98-100 °C/20 Torr) wird in analoger Weise wie im Beispiel 80 aus 11,4 g N-Ethylpiperazin und 25 g N,N-Dimethylformamid-dimethylacetal erhalten.

Beispiel 32: 4-(4-Propylpiperazinyl)-imidazolo[4,5-c]rifamycin SV (32)

In analoger Weise, wie in Beispiel 1 beschrieben, werden aus 2,0 g 3-Amino-4-iminorifamycin S in 100 ml THF und 3,42 g 1-Formyl-4-propylpiperazin-dimethylacetal 0,9 g 4-(4-Propylpiperazinyl)-imidazolo[4,5-c]-rifamycin SV (32) ($C_{45}H_{61}N_5O_{11}$, MG = 847) hergestellt.

Das als Reagens benötigte 1-Formyl-4-propylpiperazin-dimethylacetal wird in analoger Weise wie im Beispiel 82 aus 12,8 g N-Propylpiperazin und 29,75 g N,N-Dimethylformamid-dimethylacetal erhalten.

Beispiel 33: 4-(4-Butylpiperazinyl)-imidazolo[4,5-c]rifamycin SV (33)

In analoger Weise, wie in Beispiel 1 beschrieben, wird aus 4,0 g 3-Amino-4-iminorifamycin-S in 50 ml THF und 4,0 g 1-Formyl-4-butylpiperazin-dimethylacetal die gewünschte Titelverbindung (33) ($C_{46}H_{63}N_5O_{11}$, MG = 861) erhalten. MG gefunden: 861.

Das als Reagens benötigte 1-Formyl-4-butyl-piperazin-dimethylacetal wird in analoger Weise wie im Beispiel 82 aus 10 g N-Butylpiperazin und 20 g N,N-Dimethylformamid-dimethylacetal erhalten.

Beispiel 34: 4-(4-Pentylpiperazinyl)-imidazolo[4,5-c]rifamycin SV (34)

In analoger Weise, wie in Beispiel 1 beschrieben, werden aus 2 g 3-Amino-4-iminorifamycin S in 100 ml THF und 3,5 g 1-Formyl-4-pentylpiperazin-dimethylacetal 1,4 g 4-(4-Pentylpiperazinyl)-imidazolo[4,5-c]-rifamycin SV (34) ($C_{47}H_{65}N_5O_{11}$, MG = 875) hergestellt.

Das als Reagens benötigte 1-Formyl-4-pentylpiparazin-dimethylacetal wird in analoger Weise wie im Beispiel 82 aus 6,46 g N-Pentylpiperazin und 12,3 g N,N-Dimethylformamid-dimethylacetal erhalten.

Beispiel 35: 4-(4-Hexylpiperazinyl)-imidazolo[4,5-c]rifamycin SV (35)

In analoger Weise, wie in Beispiel 1 beschrieben, werden aus 2,0 g 3-Amino-4-iminorifamycin S in 100 ml THF und 4,13 g 1-Formyl-4-hexylpiperazin-dimethylacetal 1,25 g 4-(4-Hexylpiperazinyl)-imidazolo-[4,5-c]rifamycin SV (35) ($C_{48}H_{67}N_5O_{11}$, MG = 889) hergestellt.

Das als Reagens benötigte 1-Formyl-4-hexylpiperazin-dimethylacetal (Sdp.: 125-130 °C/0,3 Torr) wird in analoger Weise wie im Beispiel 80 aus 17 g N-Hexylpiperazin und 29,75 g N,N-Dimethylformamid-dimethylacetal erhalten.

Beispiel 36: 4-(4-Heptylpiperazinyl)-imidazolo[4,5-c]rifamycin SV (36)

In analoger Weise, wie in Beispiel 1 beschrieben, werden aus 2,0 g 3-Amino-4-iminorifamycin S in 100 ml THF und 4,33 g 1-Formyl-4-heptylpiperazin-dimethylacetal 1,2 g 4-(4-Heptylpiperazinyl)-imidazolo-[4,5-c]rifamycin SV (36) ($C_{49}H_{69}N_5O_{11}$, MG = 903) hergestellt.

Das als Reagens benötigte 1-Formyl-4-heptylpiperazin-dimethylacetal (Sdp.: 128°-129°C/0.15 Torr) wird in analoger Weise wie im Beispiel 80 aus 18,4 g N-Heptylpiperazin und 30 g N,N-Dimethylformamid-dimethylacetal erhalten.

Beispiel 37: 4-[4-(1-Methylpropyl)-piperazinyl]-imidazolo[4,5-c]-rifamycin SV (37)

In analoger Weise, wie in Beispiel 1 beschrieben, wird aus 4,0 g 3-Amino-4-iminorifamycin S in 50 ml THF und 4,0 g 1-Formyl-4-(1-methylpropyl)piperazin-dimethylacetal die Titelverbindung (37) ($C_{46}H_{63}N_5O_{11}$, MG = 861) hergestellt.

Das als Reagens benötigte 1-Formyl-4-(1-methylpropyl)-piperazin-dimethylacetal wird in analoger Weise, wie im Beispiel 80 beschrieben, aus 10 g N-(1-Methylpropylpiperazin und 20 g N,N-Dimethylformamid-dimethylacetal erhalten.

Beispiel 38: 4-[4-(2-Methyl-butyl)piperazinyl]-imidazolo[4,5-c]rifamycin SV (38)

In analoger Weise, wie in Beispiel 1 beschrieben, wird aus 4,0 g 3-Amino-4-iminorifamycin S in 50 ml THF und 4,0 g 1-Formyl-4-(2-methylbutyl)-piperazin-dimethylacetal 4-[4-(2-Methylbutyl)-1-piperazinyl]-imidazolo[4,5-c]rifamycin SV (38) ($C_{47}H_{65}N_5O_{11}$, MG = 875) vom Smp. 185 °C (Zersetzung) hergestellt. MG gefunden: 875.

Das als Reagens benötigte 1-Formyl-4-(2-methylbutyl)-piperazin-dimethylacetal wird in analoger Weise wie in Beispielen 80-82 aus 10 g N-(2-Methylbutyl)-piperazin und 20 g N,N-Dimethylformamid-dimethylacetal erhalten.

Beispiel 39: 4-[4-(2-Methylpentyl)-piperazinyl]-imidazolo[4,5-c]-rifamycin SV (39)

In analoger Weise, wie in Beispiel 1 beschrieben,wird aus 4,0 g 3-Amino-4-iminorifamycin S in 50 ml THF und 4,0 g 1-Formyl-4-(2-methylpentyl)-piperazin-dimethylacetal 4-[4-(2-Methylpentyl)-1-piperazinyl]-imidazolo[4,5-c]rifamycin SV (39) ($C_{48}H_{67}N_5O_{11}$, MG = 889) vom Smp. 171-172°C (Zersetzung) hergestellt. MG gefunden: 889.

Das als Reagens benötigte 1-Formyl-4-(2-methylpentyl)-piperazin-dimethylacetal wird in analoger Weise, wie in Beispielen 80-82 beschrieben, aus 10 g N-(2-Methylpentyl)-piperazin und 20 g N,N-Dimethylformamid-dimethylacetal erhalten.

Beispiel 40: 4-[4-(3-Methylbutyl)piperazinyl]-imidazolo[4,5-c]rifamycin SV (40)

In analoger Weise, wie in Beispiel 1 beschrieben, wird aus 4,0 g 3-Amino-4-iminorifamycin S in 50 ml THF und 4,0 g 1-Formyl-4-(3-methylbutyl)-piperazin-dimethylacetal 4-[4-(3-Methylbutyl)-1-piperazinyl]-imidazolo[4,5-c]rifamycin SV (40) ($C_{47}H_{65}N_5O_{11}$, MG = 875) hergestellt.

Das als Reagens benötigte 1-Formyl-4-(3-methylbutyl)-piperazin)-dimethylacetal wird in analoger Weise, wie in Beispielen 80-82 beschrieben, aus 10 g N-(3-Methylbutyl)-piperazin und 20 g N,N-Dimethylformamid-dimethylacetal erhalten.

Beispiel 41: 4-[4-(1-Ethylpropyl)-piperazinyl]-imidazolo[4,5-c]rifamycin SV (41)

In analoger Weise, wie in Beispiel 1 beschrieben, wird aus 4,0 g 3-Amino-4-iminorifamycin S in 50 ml THF und 4,0 g 1-Formyl-4-(2-ethylpropyl)-piperazin-dimethylacetal 4-[4-(2-Ethylpropyl)-1-piperazinyl]-imidazolo[4,5-c]rifamycin SV (41) ($C_{47}H_{65}N_5O_{11}$, MG = 875) hergestellt. MG gefunden: 875.

Das als Reagens benötigte 1-Formyl-4-(2-ethylpropyl)-piperazin-dimethylacetal wird in analoger Weise wie in Beispielen 80-82 aus 10 g N-(2-Ethylpropylpiperazin und 20 g N,N-Dimethylformamid-dimethylacetal erhalten.

Beispiel 42: 4-[4-(3-Methylpentyl)-piperazinyl]-imidazolo[4,5-c]-rifamycin SV (42)

In analoger Weise, wie in Beispiel 1 beschrieben, wird aus 4,0 g 3-Amino-4-iminorifamycin S in 50 ml THF und 4,0 g 1-Formyl-4-(3-methylpentyl)-piperazin-dimethylacetal 4-[4-(3-Methylpentyl)-1-piperazinyl]-imidazolo[4,5-c]rifamycin SV (42) ($C_{48}H_{67}N_5O_{11}$, MG = 889)

hergestellt. MG gefunden: 889.

Das als Reagens benötigte 1-Formyl-4-(3-methylpentyl)-piperazin-dimethylacetal wird in analoger Weise wie in Beispielen 80-82 aus 10 g N-(3-Methylpentyl)-piperazin und 20 g N,N-Dimethylformamid-dimethylacetal erhalten.

Beispiel 43: 4-[4-(1,3-Dimethylpropyl)-piperazinyl]-imidazolo[4,5-c]-rifamycin SV (43)

In analoger Weise, wie in Beispiel 1 beschrieben, werden aus 2,0 g 3-Amino-4-iminorifamycin S in 100 ml THF und 3,2 g 1-Formyl-4-(1,3-Dimethylpropyl)-piperazin-dimethylacetal 1,7 g der Titelverbindung (43) ($C_{47}H_{65}N_{5}O_{11}$, MG = 875) hergestellt.

Das als Reagens benötigte 1-Formyl-4-(1,3-Dimethylpropyl)-piperazin-dimethylacetal wird in analoger Weise wie im Beispiel 82 aus 2,55 g N-(1,3-Dimethylpropyl)-piperazin und 4,87 g N,N-Dimethylformamid-dimethylacetal erhalten.

Beispiel 44: 4-(4-Allylpiperazinyl)-imidazolo[4,5-c]rifamycin SV (44)

In analoger Weise, wie in Beispiel 1 beschrieben, werden aus 2,0 g 3-Amino-4-iminorifamycin S in 100 ml THF und 3,4 g 1-Formyl-4-allylpiperazin-dimethylacetal 1,2 g 4-(4-Allylpiperazinyl)-imidazolo-[4,5-c]-rifamycin SV (44) ($C_{45}H_{59}N_{5}O_{11}$, MG = 845) hergestellt.

Das als Reagens benötigte 1-Formyl-4-allylpiperazin-dimethylacetal wird in analoger Weise wie im Beispiel 82 aus 5 g N-Allylpiperazin und 11,9 g N,N-Dimethylformamid-dimethylacetal erhalten.

Beispiel 45: 4-[4-(3-Butenyl)-piperazinyl]-imidazolo[4,5-c]rifamycin SV (45)

In analoger Weise wie in Beispiel 1 beschrieben, wird aus 4,0 g 3-Amino-4-iminorifamycin S in 50 ml THF und 4,0 g 1-Formyl-4-(3-butenyl)-piperazin-dimethylacetal 4-[4-(3-Butenyl)-1-piperazinyl]-imidazolo-[4,5-c]rifamycin SV (45) ($C_{46}H_{61}N_5O_{11}$, MG = 859) hergestellt. MG gefunden (MS): 859.

Das als Reagens benötigte 1-Formyl-4-(3-butenyl)-piperazin-dimethylacetal wird in analoger Weise wie im Beispiel 82 aus 10 g N-(3-Butenyl)-piperazin und 20 g N,N-Dimethylformamid-dimethylacetal erhalten.

Beispiel 46: 4-[4-(2-Methyl-2-propenyl)-piperazinyl]-imidazolo[4,5-c]-rifamycin SV (46)

In analoger Weise, wie in Beispiel 1 beschrieben, werden aus 2,0 g 3-Amino-4-iminorifamycin S in 100 ml THF und 3,62 g 1-Formyl-4-(2-methyl-2-propenyl)-piperazin-dimethylacetal 1,15 g der Titelverbindung (46) ($C_{46}H_{61}N_5O_{11}$, MG = 859) hergestellt.

Das als Reagens benötigte 1-Formyl-4-(2-methyl-2-propenyl)-piperazin-dimethylacetal wird in analoger Weise wie im Beispiel 82 aus 21,13 g N-(2-Methyl-2-propenyl)-piperazin und 45 g N,N-Dimethylformamid-dimethylacetal erhalten.

Beispiel 47: 4-[4-(2-Ethyl-2-butenyl)-piperazinyl]-imidazolo[4,5-c]-rifamycin SV (47)

In analoger Weise, wie in Beispiel 1 beschrieben, wird aus 4,0 g 3-Amino-4-iminorifamycin S in 50 ml THF und 4,0 g 1-Formyl-4-(2-ethyl-2-butenyl)-piperazin-dimethylacetal die Titelverbindung (47) ($C_{48}H_{65}N_5O_{11}$, MG = 887) hergestellt, MG gefunden: 887.

Die Herstellung des Formamid-Reagens wird im Beispiel 89 beschrieben.

Beispiel 48: 4-[4-(2,3-Dimethyl-2-butenyl)-piperazinyl]-imidazolo-[4,5-c]rifamycin SV (48)

In analoger Weise, wie in Beispiel 1 beschrieben, werden aus 2,0 g 3-Amino-4-iminorifamycin S in 100 ml THF und 4,0 g 1-Formyl-4-(2,3-dimethyl-2-butenyl)-piperazin-dimethylacetal 0,42 g 4-[4-(2,3-Dimethyl-2-butenyl)-piperazinyl]-imidazolo[4,5-c]rifamycin SV (48) ($C_{48}H_{65}N_5O_{11}$, MG = 887) hergestellt.

Das als Reagens benötigte 1-Formyl-4-(2,3-dimethyl-2-butenyl)-piperazin-dimethylacetal wird in analoger Weise wie im Beispiel 82 aus 30 g N-(2,3-Dimethyl-2-butenyl)-piperazin und 53 g N,N-Dimethylformamid-dimethylacetal erhalten.

Beispiel 49: 4-(4-Propargylpiperazinyl)-imidazolo[4,5-c]rifamycin SV (49)

In analoger Weise, wie in Beispiel 1 beschrieben, werden aus 2,0 g 3-Amino-4-iminorifamycin S in 100 ml THF und 3,7 g 1-Formyl-4-propargylpiperazin-dimethylacetal 1,05 g 4-(4-Propargylpiperazinyl)-imidazolo-[4,5-c]rifamycin SV (49) ($C_{45}H_{57}N_5O_{11}$, MG = 843) hergestellt.

Das als Reagens benötigte 1-Formyl-4-propargylpiperazin-dimethylacetal wird in analoger Weise wie im Beispiel 82 aus 7,65 g Propargylpiperazin und 15,6 g N,N-Dimethylformamid-dimethylacetal erhalten.

Beispiel 50: 4-(4-Cyclopentylpiperazinyl)-imidazolo[4,5-c]rifamycin SV (50)

In analoger Weise, wie in Beispiel 1 beschrieben, wird aus 4,0 g 3-Amino-4-iminorifamycin S in 50 ml THF und 4,0 g 1-Formyl-4-cyclopentylpiperazin-dimethylacetal 4-(4-Cyclopentyl-1-piperazinyl)-imidazolo[4,5-c]rifamycin SV (50) ($C_{47}H_{63}N_5O_{11}$, MG = 873) hergestellt.
MG gefunden: 873.

Das als Reagens benötigte 1-Formyl-4-cyclopentylpiperazin-dimethylacetal wird in analoger Weise, wie in Beispielen 80-82 beschrieben, aus 10 g N-Cyclopentylpiperazin und 20 g N,N-Dimethylformamid-dimethylacetal erhalten.

Beispiel 51: 4-(4-Cyclohexylpiperazinyl)-imidazolo[4,5-c]rifamycin SV (51)

In analoger Weise, wie in Beispiel 1 beschrieben, wird aus 4,0 g 3-Amino-4-iminorifamycin S in 50 ml THF und 4,0 g 1-Formyl-4-cyclohexylpiperazin-dimethylacetal 4-(4-Cyclohexyl-1-piperazinyl)-imidazolo-[4,5-c]rifamycin SV (51) ($C_{48}H_{65}N_5O_{11}$, MG = 887) hergestellt.

Das als Reagens benötigte 1-Formyl-4-cyclohexylpiperazin-dimethylactal wird in analoger Weise, wie in Beispielen 80-82 beschrieben, aus 10 g N-Cyclohexylpiperazin und 20 g N,N-Dimethylformamid-dimethylacetal erhalten.

Beispiel 52: 4-(4-Phenylpiperazinyl)—imidazolo[4,5-c]rifamycin SV (52)

In analoger Weise, wie in Beispiel 1 beschrieben, werden aus 2,0 g 3-Amino-4-iminorifamycin S in 100 ml THF und 3,32 g 1-Formyl-4-phenylpiperazin-dimethylacetal 0,79 g 4-(4-Phenylpiperazinyl)-imidazolo-[4,5-c]rifamycin SV (52) ($C_{48}H_{59}N_5O_{11}$, MG = 881) hergestellt.

Die Herstellung des Acetal-Reagens wird in Beispiel 91 beschrieben.

Beispiel 53: 4-[4-(3-Trifluormethylphenyl)-piperazinyl]-imidazolo-[4,5-c]rifamycin SV (53)

In analoger Weise, wie in Beispiel 1 beschrieben, werden aus 2,0 g 3-Amino-4-iminorifamycin S in 100 ml THF und 5,14 g 1-Formyl-4-(3-trifluormethylphenyl)-piperazin-dimethylacetal 0,73 g 4-[4-(3-Trifluormethylphenyl)-piperazinyl]-imidazolo[4,5-c]rifamycin SV (53) ($C_{49}H_{58}N_5O_{11}F_3$, MG = 949) hergestellt.

Das als Reagens benötigte 1-Formyl-4-(3-trifluormethylphenyl)-piperazin-dimethylacetal (Sdp.: 158-160°C/0,2 Torr) wird in analoger Weise, wie in Beispiel 80 beschrieben, aus 25 g N-(3-Trifluormethylphenyl)-piperazin und 32,4 g N,N-Dimethylformamid-dimethylacetal erhalten.

Beispiel 54: 4-(4-Cyclopropylmethylpiperazinyl)-imidazolo[4,5-c]rifamycin SV (54)

In analoger Weise, wie in Beispiel 1 beschrieben, wird aus 4,0 g 3-Amino-4-iminorifamycin S in 50 ml THF und 4,0 g 1-Formyl-4-cyclopropylmethylpiperazin-dimethylacetal 4-(4-Cyclopropylmethyl-piperazinyl)-imidazolo[4,5-c]rifamycin SV (54) ($C_{46}H_{61}N_5O_{11}$, MG = 859) hergestellt, MG gefunden: 859.

Das als Reagens benötigte 1-Formyl-4-cyclopropylmethylpiperazin-dimethylacetal wird in analoger Weise, wie in Beispielen 80-82 beschrieben, aus 10 g N-Cyclopropylmethylpiperazin und 20 g N,N-Dimethylformamid-dimethylacetal erhalten.

Beispiel 55: 4-(4-Cyclopentylmethylpiperazinyl)-imidazolo[4,5-c]-rifamycin SV (55)

In analoger Weise, wie in Beispiel 1 beschrieben, werden aus 1,0 g 3-Amino-4-iminorifamycin S in 50 ml THF und 1,26 g 1-Formyl-4-cyclopentylmethylpiperazin-dimethylacetal 0,275 g 4-(4-Cyclopentylmethylpiperazinyl)-imidazolo[4,5-c]rifamycin SV (55) ($C_{48}H_{65}N_5O_{11}$, MG = 887) hergestellt.

Das als Reagens benötigte 1-Formyl-4-cyclopentylmethylpiperazin-dimethylacetal wird in analoger Weise, wie im Beispiel 82 beschrieben, aus 4,71 g Cyclopentylmethylpiperazin und 9,5 g N,N-Dimethylformamid-dimethylacetal erhalten.

Beispiel 56: 4-(4-Cycloheptylmethylpiperazinyl)-imidazolo[4,5-c]-rifamycin SV (56)

In analoger Weise, wie in Beispiel 1 beschrieben, wird aus 4,0 g 3-Amino-4-iminorifamycin S in 50 ml THF und 4,0 g 1-Formyl-4-cycloheptylmethylpiperazin-dimethylacetal die Titelverbindung (56) ($C_{50}H_{69}N_5O_{11}$, MG = 915) vom Smp. 179-180°C (Zersetzung) hergestellt. MG gefunden: 915.

Das als Reagens benötigte 1-Formyl-4-cycloheptylmethylpiperazin-dimethylacetal wird in analoger Weise, wie in Beispielen 80-82 beschrieben, aus 10 g N-Cycloheptylmethylpiperazin und 20 g N,N-Dimethylformamid-dimethylacetal erhalten.

Beispiel 57: 4-(4-Benzylpiperazinyl)-imidazolo[4,5-c]rifamycin SV (57)

In analoger Weise, wie in Beispiel 1 beschrieben, wird aus 4,0 g 3-Amino-4-iminorifamycin S in 50 ml THF und 4,0 g 1-Formyl-4-benzylpiperazin-dimethylacetal 4-(4-benzylpiperazinyl)-imidazolo[4,5-c]rifamycin SV (57) ($C_{49}H_{61}N_5O_{11}$, MG = 885) hergestellt.

Das als Reagens benötigte 1-Formyl-4-benzylpiperazin-dimethylacetal wird in analoger Weise wie in Beispielen 80-82 aus 10 g N-Benzylpiperazin und 20 g N,N-Dimethylformamid-dimethylacetal erhalten.

Beispiel 58: 4-[4-(2-Methyl-3-phenyl-propyl)-piperazinyl]-imidazolo-[4,5-c]rifamycin SV (58)

In analoger Weise, wie in Beispiel 1 beschrieben, wird aus 4 g 3-Amino-4-iminorifamycin S in 50 ml THF und 4 g 1-Formyl-4-(2-methyl-3-phenyl-propyl)-piperazin-dimethylacetal die Titelverbindung (58) ($C_{52}H_{67}N_5O_{11}$, MG = 937) hergestellt. MG gefunden: 937.

Das als Reagens benötigte 1-Formyl-4-(2-methyl-3-phenyl-propyl)-piperazin-dimethylacetal wird in analoger Weise wie in Beispiel 82 beschrieben aus 10 g N-(2-Methyl-3-phenyl-propyl)-piperazin und 20 g N,N-Dimethylformamid-dimethylacetal erhalten.

Beispiel 59: 4-[4-(2-Pyridyl)-piperazinyl]-imidazolo[4,5-c]rifamycin SV (59)

In analoger Weise, wie in Beispiel 1 beschrieben, werden aus 2,0 g 3-Amino-4-iminorifamycin S in 100 ml THF und 4,01 g 1-Formyl-4-(2-pyridyl)-piperazin-dimethylacetal 1,28 g 4-[4-(2-Pyridyl)-piperazinyl]-imidazolo[4,5-c]rifamycin SV (59) ($C_{47}H_{58}N_6O_{11}$, MG = 882) hergestellt.

Das als Reagens benötigte 1-Formyl-4-(2-pyridyl)-piperazin-dimethylacetal (Sdp.: 154-156°C/0,7 Torr) wird in analoger Weise, wie im Beispiel 80 beschrieben, aus 25 g N-(2-Pyridyl)-piperazin und 45 g N,N-Dimethylformamid-dimethylacetal erhalten.

Beispiel 60: 4-[4-(2-Pyridylmethyl)-piperazinyl]-imidazolo[4,5-c]-rifamycin SV (60)

In analoger Weise, wie in Beispiel 1 beschrieben, werden aus 2,0 g 3-Amino-4-iminorifamycin S in 100 ml THF und 4,26 g 1-Formyl-4-(2-pyridylmethyl)-piperazin-dimethylacetal 0,97 g 4-[4-(2-Pyridylmethyl)-piperazinyl]-imidazolo[4,5-c]rifamycin SV (60) ($C_{48}H_{60}N_6O_{11}$, MG = 896)

hergestellt.

Das als Reagens benötigte 1-Formyl-4-(2-pyridylmethyl)-piperazin-dimethylacetal (Sdp.: 150-160°C/0,1 Torr) wird in analoger Weise, wie in Beispiel 80 beschrieben aus 15,85 g N-(2'-Pyridylmethyl)-piperazin und 29,8 g N,N-Dimethylformamid-dimethylacetal erhalten.

Beispiel 61: 4-[4-(2-Tetrahydrofurylmethyl)-piperazinyl]-imidazolo-[4,5-c]rifamycin SV (61)

In analoger Weise, wie in Beispiel 1 beschrieben, wird aus 4,0 g 3-Amino-4-iminorifamycin S in 50 ml THF und 4,0 g 1-Formyl-4-(2-tetrahydrofurylmethyl)-piperazin-dimethylacetal die Titelverbindung (61) ($C_{47}H_{63}N_5O_{12}$, MG = 889) hergestellt. MG gefunden: 889.

Das als Reagens benötigte 1-Formyl-4-(2-tetrahydrofurylmethyl)-piperazin-dimethylactal wird in analoger Weise, wie in Beispielen 80-82 beschrieben, aus 10 g N-(2-Tetrahydrofurylmethyl)-piperazin und 20 g N,N-Dimethylformamid-dimethylacetal erhalten.

Beispiel 62: 4-{4-[2-(1,3-Dioxolan-2-yl)-ethyl]-piperazinyl}-imidazolo-[4,5-c]rifamycin SV (62)

In analoger Weise, wie in Beispiel 1 beschrieben, wird aus 4,0 g 3-Amino-4-iminorifamycin-S in 50 ml THF und 4,0 g 1-Formyl-4-[2-(1,3-dioxolan-2-yl)-ethyl]-piperazin-dimethylacetal die Titelverbindung (62) ($C_{47}H_{63}N_5O_{13}$, MG = 905) hergestellt. MG gefunden (MS): 905.

Das benötigte Formamidacetal-Reagens wird analog dem Beispiel 82 aus 10 g N-[2-(1,3-Dioxolan-2-yl)-ethyl]-piperazin und 20 g N,N-Dimethylformamid-dimethylacetal erhalten.

Beispiel 63: 4-[4-(2-Methoxyethyl)-piperazinyl]-imidazolo[4,5-c]rifamycin SV (63)

In analoger Weise, wie in Beispiel 1 beschrieben, werden aus 2,0 g 3-Amino-4-iminorifamycin S in 100 ml THF und 3,19 g 1-Formyl-4-(2-methoxyethyl)-piperazin-dimethylacetal 0,43 g der Titelverbindung (63) ($C_{45}H_{61}N_5O_{12}$, MG = 863) hergestellt.

Das als Reagens benötigte 1-Formyl-4-(2-methoxyethyl)-piperazin-dimethylacetal (Sdp.: 101-103°C/0,2 Torr) wird in analoger Weise, wie im Beispiel 80 beschrieben, aus 19 g N-(2-Methoxyethyl)-piperazin und 40 g N,N-Dimethylformamid-dimethylacetal erhalten.

Beispiel 64: 4-[4-(2-Ethoxyethyl)-piperazinyl]-imidazolo[4,5-c] rifamycin SV (64)

In analoger Weise, wie in Beispiel 1 beschrieben, wird aus 4,0 g 3-Amino-4-iminorifamycin S in 50 ml THF und 4,0 g 1-Formyl-4-(2-ethoxyethyl)-piperazin-dimethylacetal die Titelverbindung (64) ($C_{46}H_{63}N_5O_{12}$, MG = 877) vom Smp. 166°C (Zersetzung) hergestellt.

Das als Reagens benötigte 1-Formyl-4-(2-ethoxyethyl)-piperazin-dimethylacetal wird in analoger Weise wie in Beispielen 80-82 aus 10 g N-(2-Ethoxyethyl)-piperazin und 20 g N,N-Dimethylformamid-dimethylacetal erhalten.

Beispiel 65: 4-[4-(2,2-Dimethoxyethyl)-piperazinyl]-imidazolo-[4,5-c]rifamycin SV (65)

In analoger Weise, wie in Beispiel 1 beschrieben, wird aus 4,0 g 3-Amino-4-iminorifamycin S in 50 ml THF und 4,0 g 1-Formyl-4-(2,2-dimethoxyethyl)-piperazin-dimethylacetal 4-[4-(2,2-Dimethoxyethyl)-piperazinyl]-imidazolo[4,5-c]rifamycin SV (65) ($C_{46}H_{63}N_5O_{13}$, MG = 893) hergestellt, MG gefunden: 893.

Das als Reagens benötigte 1-Formyl-4-(2,2-dimethoxyethyl)-piperazin-dimethylacetal wird in analoger Weise, wie in Beispiel 82 beschrieben, aus 10 g N-(2,2-Dimethoxyethyl)-piperazin und 20 g N,N-Dimethylformamid-dimethylacetal erhalten.

Beispiel 66: 4-[4-(2,2-Diethoxyethyl)-piperazinyl]-imidazolo[4,5-c]-rifamycin SV (66)

In analoger Weise, wie in Beispiel 1 beschrieben, werden aus 2,0 g 3-Amino-4-iminorifamycin S in 100 ml THF und 4,87 g 1-Formyl-4-(2,2-diethoxyethyl)-piperazin-dimethylacetal 2,0 g der Titelverbindung (66) ($C_{48}H_{67}N_5O_{13}$, MG = 921) hergestellt.

Das als Reagens benötigte 1-Formyl-4-(2,2-diethoxyethyl)-piperazin-dimethylacetal wird in analoger Weise, wie im Beispiel 82 beschrieben, aus 6,19 g N-(2,2-Diethoxyethyl)-piperazin und 8,60 g N,N-Dimethylformamid-dimethylacetal erhalten.

Beispiel 67: 4-(4-Ethoxycarbonylpiperazinyl)-imidazolo[4,5-c]rifamycin SV (67)

In analoger Weise, wie in Beispiel 1 beschrieben, wird aus 4,0 g 3-Amino-4-iminorifamycin S in 50 ml THF und 4,0 g N-Formyl-N'-ethoxycarbonylpiperazin-dimethylacetal die Titelverbindung (67) ($C_{45}H_{59}N_5O_{13}$, MG = 877) hergestellt.

Die Herstellung des Formamidacetal-Reagens ist im Beispiel 90 beschrieben.

Beispiel 68: 4-(4-Isopropyloxycarbonylpiperazinyl)-imidazolo[4,5-c]-rifamycin SV (68)

In analoger Weise, wie in Beispiel 1 beschrieben, werden aus 2,0 g 3-Amino-4-iminorifamycin S in 100 ml THF und 4,16 g 1-Formyl-4-isopropyloxycarbonylpiperazin-dimethylacetal 1,17 g 4-(4-Isopropyloxycarbonylpiperazinyl)-imidazolo[4,5-c]rifamycin SV (68) ($C_{46}H_{61}N_5O_{13}$, MG = 891) hergestellt.

Das als Reagens benötigte 1-Formyl-4-isopropyloxycarbonylpiperazin-dimethylacetal (Sdp.: 118-119°C/0,15 Torr) wird in analoger Weise, wie im Beispiel 80 beschrieben, aus 8,5 g N-Isopropyloxycarbonylpiperazin und 14,8 g N,N-Dimethylformamid-dimethylacetal erhalten.

Beispiel 69: 4-[4-(2-Dimethylaminoethyl)-piperazinyl]-imidazolo[4,5-c]-rifamycin SV (69)

In analoger Weise, wie in Beispiel 1 beschrieben, werden aus 2,0 g 3-Amino-4-iminorifamycin S in 100 ml THF und 3,9 g 1-Formyl-4-(2-dimethylaminoethyl)-piperazin-dimethylacetal 1,05 g der Titelverbindung (69) ($C_{46}H_{64}N_6O_{11}$, MG = 876) hergestellt.

Das als Reagens benötigte 1-Formyl-4-(2-dimethylaminoethyl)-piperazin-dimethylacetal (Sdp.: 110-111°C/0,1 Torr) wird analog Beispiel 80 aus 21,26 g 2-Dimethylaminoethylpiperazin und 40,28 g N,N-Dimethyl-formamid-dimethylacetal erhalten.

Beispiel 70: 4-(4-Isobutyrylpiperazinyl)-imidazolo[4,5-c]rifamycin SV (70)

In analoger Weise, wie in Beispiel 1 beschrieben, werden aus 2,0 g 3-Amino-4-iminorifamycin S in 100 ml THF und 3,9 g 1-Formyl-4-iso-butyrylpiperazin-dimethylacetal 0,6 g 4-(4-Isobutyrylpiperazinyl)-imidazolo[4,5-c]rifamycin SV (70) ($C_{46}H_{61}N_5O_{12}$, MG = 875) hergestellt.

Das als Reagens benötigte 1-Formyl-4-isobutyryl-piperazin-dimethyl-acetal (Sdp.: 145-150°C/0,5 Torr) wird in analoger Weise, wie in Beispiel 80 beschrieben, aus 25 g N-Isobutyrylpiperazin und 47,4 g N,N-Dimethylformamid-dimethylacetal erhalten.

Beispiel 71: 4-(4-Methoxycarbonylmethylpiperazinyl)-imidazolo[4,5-c]-rifamycin SV (71)

In analoger Weise, wie in Beispiel 1 beschrieben, werden aus 2,0 g 3-Amino-4-iminorifamycin S in 100 ml THF und 3,85 g 1-Formyl-4-methoxycarbonylmethylpiperazin-dimethylacetal 0,75 g 4-(4-Methoxy-carbonylmethylpiperazinyl)-imidazolo[4,5-c]rifamycin SV (71) ($C_{45}H_{59}N_5O_{13}$, MG = 877) hergestellt.

Das als Reagens benötigte 1-Formyl-4-methoxycarbonylmethyl-piperazin-dimethylacetal (Sdp.: 114-115°C/1 Torr) wird in analoger Weise, wie in

Beispiel 80 beschrieben, aus 10 g N-Methoxycarbonylmethylpiperazin und 75 g N,N-Dimethylformamid-dimethylacetal erhalten.

Beispiel 72: 4-(4-Isopropylcarbamoylmethylpiperazinyl)-imidazolo-[4,5-c]rifamycin SV (72)

In analoger Weise, wie in Beispiel 1 beschrieben, werden aus 2,0 g 3-Amino-4-iminorifamycin S in 100 ml THF und 4,38 g 1-Formyl-4-isopropylcarbamoylmethyl-piperazin-dimethylacetal 0,62 g der Titelverbindung (72) ($C_{47}H_{64}N_6O_{12}$, MG = 904) hergestellt.

Das als Reagens benötigte 1-Formyl-4-isopropylcarbamoylmethyl-piperazin-dimethylacetal (Sdp.:161-163°C/0,2 Torr) wird in analoger Weise wie in Beispiel 80 beschrieben, aus 15 g N-Isopropyl-1-piperazin-acetamid und 24,1 g N,N-Dimethylformamid-dimethylacetal erhalten.

Beispiel 73: 4-(4-Morpholinocarbonylmethylpiperazinyl)-imidazolo-[4,5-c]rifamycin SV (73)

In analoger Weise, wie in Beispiel 1 beschrieben, werden aus 2,0 g 3-Amino-4-iminorifamycin S in 100 ml THF und 4,85 g 1-Formyl-4-morpholinocarbonylmethylpiperazin-dimethylacetal 1,03 g der Titelverbindung (73) ($C_{48}H_{64}N_6O_{13}$, MG = 932) hergestellt.

Das als Reagens benötigte 1-Formyl-4-morpholinocarbonylmethylpiperazin-dimethylacetal wird analog Beispiel 82 aus 25 g 4-Piperazinylacetylmorpholin und 35 g N,N-Dimethylformamid-dimethylacetal erhalten.

Beispiel 74: 4-[4-(1-Methyl-2-dimethylaminoethyl)-piperazinyl]-imidazolo[4,5-c]rifamycin SV (74)

In analoger Weise, wie in Beispiel 1 beschrieben, werden aus 2,0 g 3-Amino-4-iminorifamycin S in 100 ml THF und 4,15 g 1-Formyl-4-(1-methyl-2-dimethylaminoethyl)-piperazin-dimethylacetal 0,84 g der Titelverbindung (74) ($C_{47}H_{66}N_6O_{11}$, MG = 890) hergestellt.

Das benötigte Formamidacetal-Reagens (Sdp.: 115-117°C/0,2 Torr) wird in analoger Weise,wie im Beispiel 80 beschrieben, aus 17,1 g N-(1-Methyl-2-dimethylaminoethyl)-piperazin und 30 g N,N-Dimethylformamid-dimethylacetal erhalten.

Beispiel 75: 4-(4-Isobutyl-2-methyl-piperazinyl)-imidazolo[4,5-c]rifamycin SV (75)

In analoger Weise, wie in Beispiel 1 beschrieben, wird aus 4,0 g 3-Amino-4-iminorifamycin S in 50 ml THF und 4,0 g 1-Formyl-4-isobutyl-2-methyl-piperazin-dimethylacetal die Titelverbindung (75) ($C_{47}H_{65}N_5O_{11}$, MG = 875) vom Smp. 175-180° (Zersetzung) hergestellt. MG gefunden: 875.

Das als Reagens benötigte 1-Formyl-4-isobutyl-2-methylpiperazin-dimethylacetal wird analog Beispiel 82 aus 10 g 1-Isobutyl-3-methyl-piperazin und 20 g N,N-Dimethylformamid-dimethylacetal erhalten.

Beispiel 76: 4-(4-Isobutyl-2,5-dimethyl-piperazinyl)-imidazolo[4,5-c]-rifamycin SV (76)

In analoger Weise, wie in Beispiel 1 beschrieben, wird aus 4,0 g 3-Amino-4-iminorifamycin S in 50 ml THF und 4,0 g 1-Formyl-4-isobutyl-2,5-dimethylpiperazin-dimethylacetal 4-(4-Isobutyl-2,5-dimethylpiperazinyl)-imidazolo[4,5-c]rifamycin SV (76) ($C_{48}H_{67}N_5O_{11}$, MG= 889) hergestellt. MG gefunden:889.

Das als Reagens benötigte 1-Formyl-4-isobutyl-2,5-dimethylpiperazin-dimethylacetal wird in analoger Weise, wie im Beispiel 82 beschrieben, aus 10 g 1-Isobutyl-2,5-dimethylpiperazin und 20 g N,N-Dimethylformamid-dimethylacetal erhalten.

Beispiel 77: 4-(4-Isobutyl-perhydro-1,4-diazepinyl)-imidazolo[4,5-c]-rifamycin SV (77)

In analoger Weise, wie in Beispiel 1 beschrieben, wird aus 4,0 g 3-Amino-4-iminorifamycin S in 50 ml THF und 4,0 g 1-Formyl-4-isobutyl-perhydro-1,4-diazepin-dimethylacetal 4-(4-Isobutyl-perhydro-1,4-diazepinyl)-imidazolo[4,5-c]rifamycin SV (77) ($C_{47}H_{65}N_5O_{11}$, MG = 875 hergestellt. MG gefunden: 875.

Das als Reagens benötigte 1-Formyl-4-isobutyl-perhydro-1,4-diazepin-dimethylacetal wird in analoger Weise, wie in Beispielen 80-82 beschrieben, aus 10 g N-Isobutyl-perhydro-1,4-diazepin und 20 g N,N-Dimethylformamid-dimethylacetal erhalten.

Beispiel 78: 3-Dimethylaminomethylenamino-Rifamycin S (78)

1 g 3-Aminorifamycin S wird in 50 ml absolutem Methylenchlorid gelöst, mit 425 mg Triethylamin (3 Aequivalente) und 1,28 g N,N-Dimethylformamid-dimethylacetal (5 Aequivalente) versetzt, und die Reaktionslösung 45 Minuten bei Raumtemperatur stehen gelassen. Das Gemisch wird mit 50 ml Wasser versetzt, die organische Phase abgetrennt und die wässerige zweimal mit Methylenchlorid extrahiert. Die vereinigten organischen Extrakte werden über Natriumsulfat getrocknet und im Vakuum eingedampft. Der tiefblau gefärbte ölige Rückstand wird auf eine Säule von 30 g Silicagel in Chloroform aufgetragen und mit Chloroform mit steigenden Mengen Methanol (2 - 5 Vol.-%) eluiert. Durch Eindampfen wird rohes 3-Dimethylaminomethylenamino-Rifamycin S erhalten, dass man aus Ether/Hexan kristallisiert und als blauschwarzes Pulver, Smp. 150 - 155°C, erhält.

Citratbildung

400 mg 3-Dimethylaminomethylenamino-Rifamycin S werden in 5 ml Dioxan gelöst, mit 1 ml 0,5-M Zitronensäurelösung versetzt, und die Lösung lyophilisiert, wodurch 500 mg 3-Dimethylaminomethylenamino-Rifamycin S-Citrat als blau-scharzes Pulver resultiert.

Beispiel 78A: 3-Dimethylaminomethylenamino-Rifamycin SV (durch Reduktion des entsprechenden Rifamycin S-Derivats).

Eine Lösung von 0,5 g 3-Dimethylaminomethylenamino-Rifamycin S in 5 ml Methanol wird mit 2 ml einer 10 % (G/V) wässerigen Natriumbicarbonatlösung und 5 ml einer 10 % (G/V) Lösung von $K_4[Fe(CN)_6]$ Kaliumferrocyanid) in Wasser versetzt, die Lösung 3 Minuten intensiv gerührt und mit je 20 ml Wasser und Chloroform verdünnt. Die Chloroformphase wird abgetrennt und die wässerige Phase noch zweimal mit

Chloroform extrahiert. Die vereinigten Chloroformextrakte werden getrocknet und eingedampft, wodurch 3-Dimethylaminomethylenamino-Rifamycin SV als gelbes Pulver resultiert. Beim Stehen in einer Lösung wird das letztere sehr schnell wieder zum Chinon durch Luftsauerstoffoxidation umgewandelt.

Beispiel 79: 3-Diethylaminomethylenamino-Rifamycin S (79)

Eine Lösung von 1 g 3-Aminorifamycin S in 50 ml alkoholfreiem Methylenchlorid wird auf -70°C gekühlt und mit 285 mg Triethylamin (2 Aequivalente) und 460 mg Iminoylchlorid (2,1 Aequivalente) behandelt, 5 Minuten bei -70°C gerührt und mit 50 ml Wasser versetzt. Die organische Phase wird abgetrennt und die wässerige Phase zweimal mit Methylenchlorid extrahiert, die vereinigten organischen Extrakte über Natriumsulfat getrocknet und im Vakuum eingedampft. Der tiefblau gefärbte ölige Rückstand wird auf einer Säure von 100 g Silicagel in Ethylacetat-Hexan (1:1) aufgetragen und mit Gemischen von Hexan mit steigenden Mengen (50 - 75 Vol.%) Ethylacetat eluiert; durch Eindampfen der entsprechenden Fraktionen und Kristallisation aus Ether-Hexan resultiert 3-Diethylaminomethylenamino-Rifamycin S als blau-schwarzes Pulver, Smp. 133 - 135°C.

Das als Reagens verwendete Iminoylchlorid kann folgendermassen hergestellt werden:

Eine Lösung von 10,1 g Diethylformamid in 50 ml absolutem Ether wird in Stickstoffatmosphäre unter Rühren bei 0°C tropfenweise mit 12,7 g Oxalylchlorid versetzt und weitere 3 Stunden bei +50°C gerührt. Der weisse Niederschlag des Iminoylchlorids wird in einer Drucknutsche abgenutscht, mit Ether gewaschen und im Hochvakuum getrocknet. Das stark hygroskopische Iminoylchlorid wird ohne weitere Reinigung für die Umsetzung von 3-Aminorifamycin S eingesetzt.

Beispiel 80: 3-(N-Benzyl-N-methylamino)methylenamino-Rifamycin S (80)

Eine Lösung von 1,5 g 3-Aminorifamycin S in 50 ml alkoholfreiem Methylenchlorid wird mit 637 mg Triethylamin (3 Aequivalente) und 4,1 g N-Benzyl-N-methylformamid-dimethylacetal (10 Aequivalente)

behandelt, 3 1/2 Stunden bei Raumtemperatur stehen gelassen und mit 75 ml Wasser versetzt. Die organische Phase wird abgetrennt und die wässerige zweimal mit Methylenchlorid extrahiert. Die vereinigten organischen Extrakte werden über Natriumsulfat getrocknet und im Vakuum eingedampft. Der tiefblau gefärbte ölige Rückstand wird auf eine Säule von 200 g Silicagel in Chloroform aufgetragen und mit Gemischen von Chloroform mit steigenden Mengen (1 - 5 Vol. %) Methanol eluiert. Durch Eindampfen der entsprechenden Fraktionen und Kristallisation aus Ether-Hexan resultiert das 3-(N-Benzyl-N-methylamino)-methylenamino-Rifamycin S als blau-schwarzes Pulver, Smp. 138 - 140°C.

Das als Reagens verwendete N-Benzyl-N-methylformamid-dimethylacetal kann folgendermassen erhalten werden:
Ein Gemisch von 7,27 g N-Benzyl-N-methylamin und 20 g N,N-Dimethylformamid-dimethylacetal wird während 3 Stunden am Rückfluss erhitzt, überschüssiges Reaktionsmittel im Wasserstrahlvakuum bei 50° Oelbadtemperatur und 20 Torr abdestilliert, und das eingeengte Reaktionsgemisch fraktionierter Destillation unterworfen. N-Benzyl-N-methylformamid-dimethylacetal destilliert bei 104 - 106°C/20 Torr.

Beispiel 81: 3-Pyrrolidinylmethylenamino-Rifamycin S (81)
Eine Lösung von 1,4 g 3-Aminorifamycin S in 50 ml alkoholfreiem Methylenchlorid wird mit 298 mg Triethylamin (2 Aequivalente) und 1,43 g N-Formylpyrrolidin-dimethylacetal (5 Aequivalente) behandelt, 1 1/2 Stunden bei Raumtemperatur stehen gelassen, im Wasserstrahlvakuum schonend zur Trockne eingedampft und im Hochvakuum getrocknet. Der tiefblau gefärbte ölige Rückstand wird auf eine Säule von 150 g Silicagel in Chloroform aufgebracht und mit Gemischen von Chloroform mit steigenden Mengen (1 - 5 Vol. %) Methanol eluiert. Nach Eindampfen der entsprechenden Fraktionen und Kristallisation aus Methanol-Wasser resultiert 3-Pyrrolidinyl-methylenamino-Rifamycin S als blau-schwarzes Pulver, Smp. 165 - 170°C.

Das als Reagens benötigte N-Formylpyrrolidin-dimethylacetal kann

folgendermassen hergestellt werden:

Ein Gemisch von 10 g Pyrrolidin und 42 g N,N-Dimethylformamid-dimethylacetal wird während 3 Stunden am Rückfluss erhitzt, überschüssiges Reaktionsmittel im Wasserstrahlvakuum bei 50°C Oelbadtemperatur und 20 Torr abdestilliert,und die restliche Flüssigkeit fraktionierter Destillation unterworfen. N-Formyl-pyrrolidindimethylacetal destilliert bei 65°C/20 Torr.

Beispiel 82: 3-Indolinylmethylenamino-Rifamycin S (82)

Eine Lösung von 1,5 g 3-Aminorifamycin S in 50 ml alkoholfreiem Methylenchlorid wird mit 640 mg Triethylamin (2 Aequivalente) und 2,5 g N-Formylindolin-dimethylacetal (5 Aequivalente) behandelt, 1 1/2 Stunden bei Raumtemperatur stehen gelassen, im Wasserstrahlvakuum schonend eingedampft und im Hochvakuum getrocknet. Der tiefblau gefärbte ölige Rückstand wird auf eine Säule von 200 g Silicagel in Chloroform aufgebracht und mit Gemischen von Chloroform mit steigenden Mengen (0,5 - 5 Vol.%) Methanol eluiert. Die entsprechenden Fraktionen werden über Sephadex® LH-20 nochmals chromatographiert, wobei mit Methanol eluiert wird. Nach Eindampfen des Eluats und Kristallisation aus Ether-Hexan resultiert 3-(Indolinyl)methylenamino-Rifamycin S als blau-schwarzer amorphes Pulver ohne definierten Schmelzpunkt.

$^{13}$C-NMR (in $CDCl_3$)

(148,8; 141,6; 131,8; 127,8; 125,5; 124,2; 109,9; 46,7; 27,0 ppm.)

Das als Reagens benötigte N-Formylindolin-dimethylacetal kann folgendermassen erhalten werden:

Ein Gemisch von 6,8 g Indolin und 20 g N,N-Dimethylformamid-dimethylacetal wird während 3 Stunden am Rückfluss erhitzt, und das überschüssige Reaktionsmittel im Wasserstrahlvakuum bei 50°C Oelbadtemperatur und 20 Torr abdestilliert. Der Destillationsrückstand, bestehend hauptsächlich aus rohem N-Formyl-indolin-dimethylacetal, wird mit mehreren Portionen Toluol extrahiert und die vereinigten Lösungen im Wasserstrahlvakuum eingedampft. Der erhaltene Rückstand

wird ohne weitere Reinigung für die obige Umsetzung verwendet.

Beispiel 83: 3-Piperidinomethylenamino-Rifamycin S (83)

Eine Lösung von 1 g 3-Aminorifamycin S in 50 ml alkoholfreiem Methylenchlorid wird mit 425 mg Triethylamin (3 Aequivalente) und 2,24 g N-Formylpiperidin-dimethylacetal behandelt, 2 Stunden bei 0° stehen gelassen, und mit 50 ml Wasser versetzt. Die organische Phase wird abgetrennt und die wässerige zweimal mit Methylenchlorid extrahiert. Die vereinigten organischen Extrakte werden über Natriumsulfat getrocknet und im Vakuum eingedampft. Der tiefblau gefärbte ölige Rückstand wird auf einer Säule von 100 g Silicagel in Chloroform mit steigenden Mengen (2 - 5 Vol.%) Methanol eluiert. Nach Eindampfen entsprechender Fraktionen und Kristallisation aus Ether-Hexan wird 3-Piperidinomethylenamino)-Rifamycin S als blauschwarzes Pulver, Smp. 162 - 165°C,erhalten.

Das als Reagens benötigte N-Formylpiperidin-dimethylacetal kann folgendermassen hergestellt werden:

Ein Gemisch von 6,3 g Piperidin und 20 g N,N-Dimethylformamid-dimethylacetal wird während 3 Stunden am Rückfluss erhitzt, überschüssiges Reaktionsmittel im Wasserstrahlvakuum bei 50°C Oelbadtemperatur und 20 Torr abdestilliert und die restliche Flüssigkeit fraktionierter Destillation unterworfen. N-Formylpiperidin-dimethylacetal destilliert bei 74°C/20 Torr.

Beispiel 84:3-(4-Methylpiperidinyl)methylenamino-Rifamycin S (84)

Eine Lösung von 1,5 g 3-Aminorifamycin S in 50 ml alkoholfreiem Methylenchlorid wird mit 637 mg Triethylamin (3 Aequivalente) und 3,6 g N-Formyl-4-methylpiperidin-dimethylacetal (10 Aequivalente) behandelt, 2 1/2 Stunden bei Raumtemperatur stehen gelassen, und mit 50 ml Wasser versetzt. Die organische Phase wird abgetrennt,

und die wässerige zweimal mit Methylenchlorid extrahiert. Die vereinigten organischen Extrakte werden über Natriumsulfat getrocknet, im Vakuum eingedampft und über eine Säule von 200 g Silicagel in Chloroform chromatographiert. Durch Eluieren mit Chloroform mit steigenden Mengen (0 - 50 Vol. %) Aceton und Eindampfen der entsprechenden Fraktionen wird ein Rückstand erhalten, der nach Kristallisation aus Ether-Hexan das 3-(4-Methylpiperidinyl)-methylenamino-Rifamycin S als blau-schwarzes Pulver, Smp. 88 - 90°, ergibt.

Das als Reagens benötigte N-Formyl-4-methylpiperidin-dimethylacetal kann folgendermassen erhalten werden:
Ein Gemisch von 5 g 4-Methylpiperidin und 14,9 g N,N-Dimethyl-formamid-dimethylacetal wird während 3 Stunden am Rückfluss erhitzt, überschüssiges Reaktionsmittel im Wasserstrahlvakuum bei 50°C Oelbadtemperatur und 20 Torr abdestilliert, und die restliche Flüssigkeit fraktionierter Destillation unterworfen. N-Formyl-4-methylpiperidin-dimethylacetal destilliert bei 78°C/20 Torr.

Beispiel 85: 3-(8-Aza-1,4-dioxa-spiro[4,5]dec-8-yl)methylenamino-Rifamycin S (85)

Eine Lösung von 2 g 3-Aminorifamycin S in 50 ml alkoholfreiem Methylenchlorid wird mit 575 mg Triethylamin (2 Aequivalente) und 3,6 g N-Formyl-4-piperidon-ethylenketal-dimethylacetal (6 Aequivalente) behandelt, 2 1/2 Stunden bei Raumtemperatur stehen gelassen, und mit 50 ml Wasser versetzt. Die organische Phase wird abgetrennt und die wässerige zweimal mit Methylenchlorid extrahiert. Die vereinigten organischen Extrakte werden über Natriumsulfat getrocknet, im Vakuum eingedampft, und der tiefblau gefärbte ölige Rückstand über eine Säule von 200 g Silicagel in Toluol chromatographiert. Durch Eluieren mit Toluol mit steigenden Mengen (10 - 75 Vol.%) Ethylacetat erhaltene entsprechende Fraktionen werden an Sephadex® LH-20 nochmals chromatographiert, wobei mit Methanol eluiert wird. Nach Kristallisation der eingedampften Fraktionen

aus Methanol-Wasser wird 3-(8-Aza-1,4-dioxa-spiro[4,5]dec-8-yl)-methylenamino-Rifamycin S als blau-schwarzes Pulver, Smp. 178 - 180°C, erhalten.

Das als Reagens benötigte N-Formyl-4-piperidon-ethylenketal-dimethylacetal kann folgendermassen erhalten werden:
Ein Gemisch von 20 g 4-Piperidon—ethylenketal und 42 g N,N-Dimethylformamid-dimethylacetal wird während 3 Stunden am Rückfluss erhitzt, überschüssiges Reaktionsmittel im Wasserstrahlvakuum bei 50°C Oelbadtemperatur und 20 Torr abdestilliert, und die restliche Flüssigkeit fraktionierter Destillation unterworfen. N-Formyl-4-piperidon-ethylenketal-dimethylacetal destilliert bei 111 - 114°C/0,5 Torr.

Beispiel 86: 3-Morpholinylmethylenamino-Rifamycin S (86)
Eine auf -70°C gekühlte Lösung von 3-Aminorifamycin S in 50 ml alkoholfreiem Methylenchlorid wird mit 1,6 g Triethylamin (7,5 Aequivalente) und 3,36 g Morpholiniminoylchlorid (10 Aequivalente) versetzt, 1 Stunde bei -40° gerührt, und mit 50 ml Wasser behandelt. Die organische Phase wird abgetrennt und die wässrige noch zweimal mit Methylenchlorid extrahiert. Die vereinigten organischen Extrakte werden über Natriumsulfat getrocknet und im Vakuum eingedampft. Der Rückstand wird in einer minimalen Menge Methanol gelöst und mit einer 10 % (G/V) wässerigen Kaliumferricyanid-Lösung in Anwesenheit von Kaliumbicarbonat behandelt. Das Oxidationsgemisch wird zweimal mit Chloroform extrahiert, die Chloroform-Extrakte getrocknet, eingedampft und über eine Säule von 100 g Silicagel in Chloroform chromatographiert. Durch Eluieren mit Chloroform mit steigenden Mengen (1 - 50 Vol.%) Aceton und Eindampfen der entsprechenden Fraktionen wird ein Rückstand erhalten, der nach Kristallisation aus Methanol-Wasser das 3-Morpholinylmethylenamino-Rifamycin S als blauschwarzes amorphes Pulver ergibt:

$^{13}$C-NMR-Spektrum (in $CDCl_3$): 160,9; 67,2; 49,1 ppm.

Das als Reagens benötigte, vom Morpholin abgeleitete Iminoylchlorid kann folgendermassen erhalten werden:
Eine Lösung von 5 g N-Formylmorpholin in 50 ml absolutem Ether wird unter Stickstoff und mit Rühren bei 0° tropfenweise mit 5,52 g Oxalylchlorid versetzt, und die Reaktionslösung weitere 3 Stunden bei +50°C gerührt. Der weisse Niederschlag des Iminoylchlorids wird in einer Drucknutsche abgenutscht, mit Ether gewaschen und im Hochvakuum getrocknet. Das stark hygroskopische Iminoylchlorid wird ohne weitere Reinigung in der obigen Umsetzung eingesetzt.

Beispiel 87: 3-Thiomorpholinylmethylenamino-Rifamycin S(87)
Eine Lösung von 1,5 g 3-Aminorifamycin S in 50 ml alkoholfreiem Methylenchlorid wird mit 637 mg Triethylamin (3 Aequivalente) und 5,6 g N-Formyl-thiomorpholin-dimethylacetal (15 Aequivalente) versetzt, 60 Minuten bei Raumtemperatur stehen gelassen und mit 50 ml Wasser behandelt. Die organische Phase wird abgetrennt und die wässerige zweimal mit Methylenchlorid extrahiert. Die vereinigten organischen Extrakte werden über Natriumsulfat getrocknet, im Vakuum eingedampft, und der tiefblau gefärbte ölige Rückstand über eine Säule mit 250 g Silicagel in Chloroform chromatographiert. Durch Eluieren mit Chloroform mit steigenden Mengen (1 - 5 Vol %) Methanol und Eindampfen der entsprechenden Fraktionen resultiert ein Rückstand, der nach Kristallisation aus Ether-Hexan 3-Thiomorpholinylmethylenamino-Rifamycin S als blau-schwarzes Pulver, Smp. 177 - 182°C, ergibt.

Das als Reagens benötigte N-Formylthiomorpholin-dimethylacetal kann folgendermassen erhalten werden:
Ein Gemisch von 5,45 g Thiomorpholin und 15,7 g N,N-Dimethylformamid-dimethylacetal wird während 3 Stunden am Rückfluss erhitzt, überschüssiges Reaktionsmittel im Wasserstrahlvakuum bei 50°C Oelbadtemperatur und 20 Torr abdestilliert, und die restliche Flüssigkeit fraktionierter Destillation unterworfen. N-Formylthiomorpholin-dimethylacetal destilliert bei 110 - 113°C/20 Torr.

Beispiel 88: 3-(4-Methylpiperazinyl)-methylenamino-Rifamycin S (88)

Ein Gemisch von 1 g 3-Aminorifamycin S, 425 mg (3 Aequivalente) Triethylamin und 2,4 g (10 Aequivalente) N-Methyl-N'-formylpiperazin-dimethylacetal in 50 ml alkoholfreiem Methylenchlorid wird 2 1/2 Stunden bei Raumtemperatur stehen gelassen und analog, wie im Beispiel 10 beschrieben, verarbeitet. Durch Chromatographie über eine Säule von 120 g Silicagel in Chloroform, Eluieren mit Chloroform mit steigendem Anteil (2 - 20 Vol.%) Aceton, und Kristallisation aus Ether-Hexan wird die Titelverbindung als blau-schwarzes Pulver, Smp. 148 - 151°, erhalten.

Das als Reagens benötigte N-Methyl-N'-formylpiperazin-dimethylacetal kann folgendermassen erhalten werden: Ein Gemisch von 5 g N-Methylpiperazin und 14,9 g N,N-Dimethylformamid-dimethylacetal wird 3 Stunden am Rückfluss erhitzt. Nach Verarbeitung analog Beispielen 3 - 8 und 10 destilliert das gewünschte N-Methyl-N'-formylpiperazin-dimethylacetal bei 84 - 85°C/20 Torr.

Beispiel 89: 3-[4-(2-Ethyl-2-butenyl)-piperazinyl]-methylenamino-Rifamycin S (89)

Ein Gemisch von 2 g 3-Aminorifamycin S, 575 mg (2 Aequivalente) Triethylamin, 3,4 g (5 Aequivalente) N'-Formyl-N-(2-ethyl-2-butenyl)-piperazin-dimethylacetal in 50 ml alkoholfreiem Methylenchlorid wird 2 Stunden bei Raumtemperatur stehen gelassen und analog, wie in Beispiel 4 beschrieben, verarbeitet. Durch Chromatographie über eine Säule von 250 g Silicagel in Chloroform und Eluieren mit Chloroform mit steigendem Anteil (0,5 - 10 Vol.%) Methanol wird ein rohes Produkt erhalten, das durch Chromatographie an Sephadex® LH-20 (Elution mit Methanol) weiter gereinigt wird. Durch Kristallisation aus Ether-Hexan wird die Titelverbindung als blau-schwarzes

Pulver, Smp. 140 - 142°C, erhalten.

Das als Reagens benötigte N'-Formyl-N-(2-ethyl-2-butenyl)-piperazin-dimethylacetal kann folgendermassen erhalten werden:
Ein Gemisch von 10 g N-(2-Ethyl-2-butenyl)-piperazin und 16,7 g N,N-Dimethylformamid-dimethylacetal wird 5 Stunden am Rückfluss erhitzt. Nach Verarbeitung analog Beispielen 3 - 8 und 10 destilliert das gewünschte N'-Formyl-N-(2-ethyl-2-butenyl)-piperazin-dimethylacetal bei 121°C/0,5 Torr.

Beispiel 90: 3-(4-Ethoxycarbonylpiperazinyl)methylenamino-Rifamycin S(90)
Ein Gemisch von 2 g 3-Aminorifamycin S, 575 mg (2 Aequivalente) Triethylamin und 3,27 g (5 Aequivalente) N'-Formyl-piperazin-N-carbonsäureethylester-dimethylacetal in 50 ml alkoholfreiem Methylenchlorid wird 4 Stunden bei Raumtemperatur stehen gelassen und analog, wie im Beispiel 4 beschrieben, verarbeitet. Durch Chromatographie über eine Säule von 250 g Silicagel in Chloroform und Eluieren mit Chlorofom mit steigendem Anteil (0,5 - 10 Vol. %) Methanol wird ein rohes Produkt erhalten, das durch Chromatographie an Sephadex® LH-20 (Elution mit Methanol) weiter gereinigt wird. Durch Kristallisation aus Ether-Hexan wird die Titelverbindung als blau-schwarzes Pulver, Smp. 160 - 165°C, erhalten.

Das als Reagens benötigte N'-Formyl-piperazin-N-carbonsäureethylester-dimethylacetal kann folgendermassen erhalten werden:
Ein Gemisch von 10,87 g Piperazin-N-carbonsäureethylester und 20,44 g N,N-Dimethylformamid-dimethylacetal wird 4 Stunden am Rückfluss erhitzt. Nach Verarbeitung analog Beispielen 3 - 8 und 10 destilliert das gewünschte N'-Formyl-piperazin-N-carbonsäureethylester-dimethylacetal bei 128 - 130°C/0,75 Torr.

Beispiel 91: 3-(4-Phenylpiperazinyl)methylenamino-Rifamycin S(91)
Ein Gemisch von 1,5 g 3-Aminorifamycin S, 425 mg (2 Aequivalente)

Triethylamin und 3,5 g (7 Aequivalente) N'Formyl-N-phenyl-piperazin-dimethylacetal in 50 ml alkoholfreiem Methylenchlorid wird 6 1/4 Stunden bei Raumtemperatur stehen gelassen und analog, wie im Beispiel 10 beschrieben, verarbeitet. Durch Chromatographie über eine Säule von 250 g Silicagel in Chloroform und Eluieren mit Chloroform mit steigendem Anteil (0,5 - 5 Vol. %) Methanol wird ein rohes Produkt erhalten, das durch Chromatographie an Sephadex® LH-20 (Elution mit Methanol) weiter gereinigt wird. Durch Kristallisation aus Ether-Hexan wird die Titelverbindung als blau-schwarzes Pulver ohne definierten Schmelzpunkt erhalten.

Das als Reagens benötigte N'-Formyl-N-phenylpiperazin-dimethylacetal kann folgendermassen erhalten werden:
Ein Gemisch von 10 g N-Phenylpiperazin und 18,3 g N,N-Dimethylformamid-dimethylacetal wird 3 Stunden am Rückfluss erhitzt. Nach Verarbeitung analog Beispielen 3 - 8 und 10 destilliert das gewünschte N'-Formyl-N-phenylpiperazin-dimethylacetal bei 165 - 167°C/0,5 Torr.

Beispiel 92: 3-(4-Aza-tricyclo[5,2,2,$0^{2,6}$]undec-8-en-4-yl)methylenamino-Rifamycin S (92)

Ein Gemisch von 1 g 3-Aminorifamycin S, 283 mg (2 Aequivalente) Triethylamin und 1,26 g (4-Aequivalente) 4-Formyl-4-aza-tricyclo-[5,2,2,$0^{2,6}$]undec-8-en-dimethylacetal in 50 ml alkoholfreiem Methylenchlorid wird 6 Stunden bei Raumtemperatur stehen gelassen und analog, wie im Beispiel 10 beschrieben, verarbeitet. Durch Chromatographie über eine Säule von 100 g Silicagel in Chloroform, Eluieren mit Chloroform mit steigendem Anteil (0 - 10 Vol. %) Methanol und Kristallisation aus Methanol-Wasser wird die Titelverbindung als blau-schwarzes Pulver, Smp. 173 - 176°C, erhalten.

Beispiel 93: 3-Dipropylaminomethylenamino-Rifamycin S durch die Reaktion mit Dimethylsulfat-Komplex

2,0 g N,N-Dipropylformamid und 2,0 g Dimethylsulfat werden 3 Stunden bei Raumtemperatur stehen gelassen und das entstandene Oel zunächst mit Benzol, dann zweimal mit Ether gewaschen und von Resten des Ethers im Vakuum befreit. Das Oel, enthaltend den N,N-Dipropylformamid-Dimethylsulfat-Komplex wird in 50 ml absolutem Methylenchlorid gelöst und unter Rühren mit 500 mg Triethylamin und 2,25 g 3-Aminorifamycin S versetzt. Die Reaktionslösung wird so lange bei Raumtemperatur stehen gelassen, bis das eingesetzte 3-Aminorifamycin S vollständig reagiert hat. Anschliessend wird das Reaktionsgemisch mit 50 ml Wasser versetzt, die organische Phase abgetrennt und die wässrige Phase zweimal mit Methylenchlorid extrahiert. Die vereinigten organischen Extrakte werden über Natriumsulfat getrocknet und im Vakuum eingedampft. Der blau gefärbte Rückstand wird an Kieselgel chromatographiert, wobei Chloroform mit steigenden Mengen (2-10 %) Methanol als Eluens verwendet wird. Das in Form eines blauschwarzen Pulvers resultierende, praktisch reine 3-Dipropylaminmethylenamino-Rifamycin S kann analog Beispiel 6 mit Ammoniakgas in Tetrahydrofuran direkt zu 4-Dipropylamino-imidazolo[4,5-c]rifamycin SV (7) umgewandelt werden, welches mit dem Produkt des Beispiels 7 völlig identisch ist.

Beispiel 94: 4-Dimethylamino-imidazolo[4,5-c]rifamycin SV (1)

In analoger Weise, wie im Beispiel 6 beschrieben, wird eine Lösung von 1,0 g 3-Dimethylaminomethylenamino-Rifamycin S (78) in 50 ml Tetrahydrofuran mit Ammoniakgas behandelt. Durch die übliche Verarbeitung wird die Titelverbindung (1) erhalten, die mit dem Produkt des Beispiels 1 identisch ist.

Beispiel 95: 4-Morpholino-imidazolo[4,5-c]rifamycin SV (2)

In analoger Weise, wie im Beispiel 6 beschrieben, wird eine Lösung von 0,5 g 3-Morpholinomethylenamino-Rifamycin S (86) in 30 ml Tetrahydrofuran mit Ammoniakgas behandelt. Durch die übliche Verarbeitung wird die Titelverbindung (2) erhalten, die mit dem Produkt des Beispiels 2 identisch ist.

Beispiel 96: 4-(4-Methylpiperazinyl)-imidazolo[4,5-c]rifamycin SV (3)

In analoger Weise, wie im Beispiel 6 beschrieben, wird eine Lösung von 0,5 g 3-(4-Methylpiperazinyl)-methylenamino-Rifamycin S (88) in 25 ml Tetrahydrofuran mit Ammoniakgas behandelt. Durch die übliche Verarbeitung wird die Titelverbindung (3) erhalten, die mit dem Produkt des Beispiels 3 identisch ist.

Beispiel 97: 4-(N-Benzyl-N-methylamino)-imidazolo[4,5-c]rifamycin SV (12)

2,0 g 3-(N-Benzyl-N-methylamino)-methylenamino-Rifamycin S (80) in 100 ml Tetrahydrofuran mit Ammoniakgas behandelt. Durch die übliche Verarbeitung wird die Titelverbindung (12) erhalten, die mit dem Produkt des Beispiels 12 identisch ist.

Beispiel 98: 4-Pyrrolidinyl—imidazolo[4,5-c]rifamycin SV (19)

In analoger Weise, wie im Beispiel 6 beschrieben, wird eine Lösung von 0,45 g 3-Pyrrolidinylmethylenamino-Rifamycin S (81) in 25 ml Tetrahydrofuran mit Ammoniakgas behandelt. Durch die übliche Verarbeitung wird die Titelverbindung (19) erhalten, die mit dem Produkt des Beispiels 19 identisch ist.

Beispiel 99: 4-Indolinyl-imidazolo[4,5-c]rifamycin SV (20)

In analoger Weise, wie im Beispiel 6 beschrieben, wird eine Lösung von 0,25 g 3-Indolinylmethylenamino-Rifamycin S (82) in 15 ml Tetrahydrofuran mit Ammoniakgas behandelt. Durch die übliche Verarbeitung wird die Titelverbindung (20) erhalten, die mit dem Produkt des Beispiels 20 identisch ist.

Beispiel 100: 4-Piperidino-imidazolo[4,5-c]rifamycin SV (21)

In analoger Weise, wie im Beispiel 6 beschrieben, wird eine Lösung von 0,5 g 3-Piperidinomethylenamino-Rifamycin S (83) in 25 ml Tetrahydrofuran mit Ammoniakgas behandelt. Durch die übliche Verarbeitung wird die Titelverbindung (21) erhalten, die mit dem Produkt des Beispiels 21 identisch ist.

Beispiel 101: 4-(8-Aza-1,4-dioxa-spiro[4,5]dec-8-yl)-imidazolo-[4,5-c]rifamycin SV (25)

In analoger Weise, wie im Beispiel 6 beschrieben, wird eine Lösung von 0,2 g 3-(8-Aza-1,4-dioxa-spiro[4,5]dec-8-yl)-methylenamino-Rifamycin S (85) in 15 ml Tetrahydrofuran mit Ammoniakgas behandelt. Durch die übliche Verarbeitung wird die Titelverbindung (25) erhalten, die mit dem Produkt des Beispiels 25 identisch ist.

Beispiel 102: 4-(4-Thiomorpholinyl)imidazolo[4,5-c]rifamycin SV (30)

In analoger Weise, wie im Beispiel 6 beschrieben, wird eine Lösung von 0,5 g 3-Thiomorpholinomethylenamino-Rifamycin S (87) in 30 ml Tetrahydrofuran mit Ammoniakgas behandelt. Durch die übliche Verarbeitung wird die Titelverbindung (30) erhalten, die mit dem Produkt des Beispiels 30 identisch ist.

Beispiel 103: 4-[4-(2-Ethyl-2-butenyl)-piperazinyl]-imidazolo[4,5-c]-rifamycin SV (47)

In analoger Weise, wie im Beispiel 6 beschrieben, wird eine Lösung von 0,5 g 3-[4-(2-Ethyl-2-butenyl)-piperazinyl]-aminomethylenamino-Rifamycin (89) in 25 ml Tetrahydrofuran mit Ammoniakgas behandelt. Durch die übliche Verarbeitung wird die Titelverbindung (47) erhalten, die mit dem Produkt des Beispiels 47 identisch ist.

Beispiel 104: 4-(4-Ethoxycarbonyl-piperazinyl)-imidazolo[4,5-c]-rifamycin SV (67)

In analoger Weise, wie im Beispiel 6 beschrieben, wird eine Lösung von 0,3 g 3-(4-Ethoxycarbonylpiperazinyl)-methylenamino-Rifamycin S (90) in 20 ml Tetrahydrofuran mit Ammoniakgas behandelt. Durch die übliche Verarbeitung wird die Titelverbindung (67) erhalten, die mit dem Produkt des Beispiels 67 identisch ist.

Beispiel 105: 4-(4-Phenylpiperazinyl)-imidazolo[4,5-c]rifamycin SV (52)

In analoger Weise, wie im Beispiel 6 beschrieben, wird eine Lösung von 0,5 g 3-(4-Phenylpiperazinyl)-methylenamino-Rifamycin S (91) in 30 ml Tetrahydrofuran mit Ammoniakgas behandelt. Durch die übliche Verarbeitung wird die Titelverbindung (52) erhalten, die mit dem Produkt des Beispiels 52 identisch ist.

Beispiel 106: 4-Morpholino-imidazolo[4,5-c]rifamycin SV (2)

In analoger Weise, wie im Beispiel 2 beschrieben, wird 4,0 g 3-Amino-4-imino-rifamycin S in 50 ml Tetrahydrofuran mit 4,0 g Tris-morpholino-methan umgesetzt. Durch die Aufarbeitung gemäss Beispiel 2 wird die Titelverbindung (2) als gelbe Kristallblättchen, Smp. 192-193° (aus Ether) erhalten.

Das als Reagens verwendete Tris-morpholino-methan wird folgendermassen erhalten:

Ein Gemisch von 15 g Morpholin und 15 g N,N-Dimethylformamid-dimethylacetal wird 12 Stunden am Rückfluss unter Feuchtigskeitsausschluss erhitzt. Danach werden alle im Wasserstrahlvakuum bis 130° flüchtigen Anteile durch Destillation aus dem Reaktionsgemisch entfernt und der verbleibende braune, ölige Rückstand, der rasch zu kristallisieren einsetzt, mit einem Gemisch von Ether mit einem kleinen Anteil Petrolether verrührt und gewaschen. Die erhaltenen weissen Kristalle (18 g) von Tris-morpholinomethan schmelzen nach Umkristallisieren aus Chloroform/Ether bei 241-243°. Für $C_{13}H_{25}N_3O_3$ (MW 271,36) berechnet: 57,54% C, 9,29% H, 15,49% N; gefunden 57,5% C, 9,2% H, 15,4% N.

Beispiel 107: 4-Dimethylamino-imidazolo[4,5-c]rifamycin SV (1)

Analog Beispiel 1 wird 4,0 g 3-Amino-4-iminorifamycin S in 40 ml Tetrahydrofuran mit Tris-dimethylamino-methan (4,0 g) umgesetzt und weiterverarbeitet. Das resultierende Produkt, Smp. 180-185°, ist mit 4-Dimethylamino-imidazolo[4,5-c]rifamycin SV (1) gemäss Beispiel 1 identisch. In analoger Weise wird unter Anwendung von Tris-diethylamino-methan das 4-Diethylamino-imidazolo[4,5-c)rifamycin SV (6), identisch mit dem Produkt des Beispiels 6, erhalten.

Beispiel 108: 4-(4-Aza-tricyclo[5,2,2,$0^{2,6}$]undec-8-en-4-yl)-imidazolo-[4,5-c]rifamycin SV (93)

In analoger Weise , wie in Beispiel 1 beschrieben, wird aus 4 g

3-Amino-4-iminorifamycin S in 50 ml THF und 4 g 4-Formyl-4-aza-tricyclo[5,2,2,$0^{2,6}$]-undec-8-en-dimethylacetal die Titelverbindung (93) ($C_{48}H_{60}N_4O_{11}$, MG = 868) hergestellt.
Die Herstellung des als Reagens benötigten 4-Formyl-4-aza-tricyclo[5,2,2,$0^{2,6}$]-undec-8-en-dimethylacetals wird in Beispiel 92 beschrieben.

Beispiel 109: 4-[4-(2-Hydroxy-2-methylpropyl)-piperazinyl]-imidazolo[4,5-c]rifamycin SV (94)

In analoger Weise, wie in Beispiel 1 beschrieben, werden aus 2,0 g 3-Amino-4-iminorifamycin S in 100 ml THF und 5,2 g 1-Formyl-4-(2-hydroxy-2-methylpropyl)-piperazin-dimethylacetal 0,5 g der Titelverbindung (94) ($C_{46}H_{63}N_5O_{12}$, MG = 877) hergestellt.
Das als Reagens benötigte 1-Formyl-4-(2-hydroxy-2-methylpropyl)-piperazin-dimethylacetal (Sdp. 86-90°/1 Torr) wird in analoger Weise, wie im Beispiel 80 beschrieben, aus 30 g N-(2-Trimethylsilyloxy-2-methylpropyl)-piperazin und 50 g N,N-Dimethylformamid-dimethylacetal erhalten.

Beispiel 110: 4-[4-(2,2-Dimethyl-1,3-dioxolan-4-ylmethyl)-piperazinyl]-imidazolo[4,5-c]rifamycin SV (95)

In analoger Weise, wie in Beispiel 1 beschrieben, wird aus 4 g 3-Amino-4-iminorifamycin S in 50 ml THF und 4 g 1-Formyl-4-(2,2-dimethyl-1,3-dioxolan-4-ylmethyl)-piperazin-dimethylacetal die Titelverbindung (95) ($C_{48}H_{65}N_5O_{13}$, MG = 919) hergestellt. MG gefunden: 919.
Das als Reagens benötigte 1-Formyl-4-(2,2-dimethyl-1,3-dioxolan-4-ylmethyl)-piperazin-dimethylacetal wird in analoger Weise, wie in Beispiel 82 beschrieben, aus 10 g N-4-(2,2-Dimethyl-1,3-dioxolan-4-ylmethyl)-piperazin und 20 g N,N-Dimethylformamid-dimethylacetal erhalten.

Beispiel 111: 4-(4-Cyclobutylmethyl-piperazinyl)-imidazolo[4,5-c]-rifamycin SV (96)

In analoger Weise, wie in Beispiel 1 beschrieben, wird aus 4 g

3-Amino-4-iminorifamycin S in 50 ml THF und 4 g 1-Formyl-4-cyclobutylmethylpiperazin-dimethylacetal die Titelverbindung (96) ($C_{47}H_{63}N_5O_{11}$, MG = 873) hergestellt.
Das als Reagens benötigte 1-Formyl-4-cyclobutylmethylpiperazin-dimethylacetal wird in analoger Weise, wie in Beispiel 82 beschrieben, aus 10 g N-Cyclobutylmethyl-piperazin und 20 g N,N-Dimethylformamid-dimethylacetal erhalten.

Beispiel 112: Bildung von Salzen mit Basen

Eine unter Stickstoff gerührte Suspension von 15,50 g 4-Dimethylamino-imidazolo[4,5-c]rifamycin SV (1) in 150 ml Wasser wird tropfenweise mit 20,0 ml einer 1-N-wässrigen Natriumhydroxid-Lösung (0,987 Aequivalent) versetzt. Die resultierende gelbgefärbte Lösung wird filtriert und das Filtrat der Gefriertrocknung unterworfen. Das zurückbleibende 4-Dimethylamino-imidazolo[4,5-c]rifamycin SV-Natriumsalz ist ein amorphes, in Wasser klar lösliches Pulver; die Lösung weist einen pH von etwa 7,3 auf. Für $C_{40}H_{51}N_4O_{11}Na$ (MG 786,85) berechnet 2,92% Na; gefunden 2,87% Na.
In analoger Weise wird mit einer entsprechenden Kaliumhydroxid-Lösung das Kaliumsalz der Verbindung (1) erhalten.
In analoger Weise können allgemein sauer reagierende Imidazolorifamycine der SV-Reihe (Formel IA) zu entsprechenden Salzen verarbeitet werden.

Beispiel 113: Bildung von Säureadditionssalzen

Eine gerührte Suspension von 8,75 g 4-(4-Isobutylpiperazinyl)-imidazolo[4,5-c]rifamycin SV (4) in 165 ml Wasser wird mit 10,0 ml 1-normaler wässriger Salzsäure (0,985 Aequivalent) versetzt. Das Gemisch wird filtriert, und das Filtrat der Gefriertrocknung unterworfen. Das erhaltene 4-(4-Isobutylpiperazinyl)-imidazolo[4,5-c]rifamycin SV-Hydrochlorid bildet ein wasserlösliches, gelbes amorphes Pulver. Für $C_{46}H_{63}N_5O_{11}$ • HCl (898,49) berechnet: 3,95% Cl, gefunden: 4,08% Cl.

In analoger Weise können allgemein Hydrochloride basisch reagierender Imidazolo[4,5-c]rifamycine hergestellt werden. Das Verfahren ist in analoger Weise auch für die Herstellung von Additionssalzen anderer wasserlöslicher Säuren anwendbar.

Beispiel 114: Kapseln, enthaltend 0,25 g des Wirkstoffes, werden wie folgt hergestellt:

Zusammensetzung (für 1000 Kapseln):

| | |
|---|---|
| Wirkstoff | 250,0 g |
| Maisstärke | 50,0 g |
| Polyvinylpyrrolidon | 15,0 g |
| Magnesiumstearat | 5,0 g |
| Ethanol | q.s. |

Der Wirkstoff und die Maisstärke werden vermischt und mit einer Lösung des Polyvinylpyrrolidons in 50 g Ethanol befeuchtet. Die feuchte Masse wird durch ein Sieb mit einer Maschenweite von 3 mm gedruckt und bei 45° getrocknet. Das trockene Granulat wird durch ein Sieb mit einer Maschenweite von 1 mm gesiebt und mit 5 g Magnesiumstearat vermischt. Die Mischung wird in Portionen von 0,320 g in Steckkapseln der Grösse 0 abgefüllt.

Beispiel 115: Tabletten, enthaltend 250 mg des Wirkstoffes, werden wie folgt hergestellt:

Zusammensetzung (für 1 Tablette):

| | |
|---|---|
| Wirkstoff | 250 mg |
| Mikrokristalline Cellulose | 80 mg |
| Natrium-carboxymethyl-stärke | 10 mg |
| Magnesiumstearat | 3 mg |
| Talk | 7 mg |
| | 350 mg |

Der Wirkstoff wird mit den Zusatzstoffen homogen vermischt und zu

Tabletten gepresst.

Zur Herstellung vom Filmdragées werden die Tabletten je mit 1 mg wässrigem Lack überzogen.

Anstelle von Natrium-carboxymethyl-stärke kann auch Natriumcarboxymethylcellulose eingesetzt werden.

Beispiel 116: Steckkapseln, enthaltend 100 mg des Wirkstoffes, werden wie folgt hergestellt:

Zusammensetzung (für 1000 Kapseln):

| | |
|---|---|
| Wirkstoff | 100,00 g |
| Lactose | 50,00 g |
| Ethylcellulose | 1,50 g |
| Stearinsäure | 1,50 g |
| | 153,00 g |

Der Wirkstoff wird mit der Lactose gemischt, die entstandene Mischung mit einer Lösung von Ethylcellulose in einer 10-fachen Gewichtsmenge Methylenchlorid befeuchtet, durch ein Sieb mit 3-5 mm Maschenweite geschlagen und bei einer 40° nicht übersteigenden Temperatur getrocknet. Das trockene Granulat wird durch ein Sieb mit 0,5 mm Maschenweite geschlagen und mit der pulverförmigen Stearinsäure vermischt. Die Mischung wird sodann in Portionen von je 0,153 g in Steckkapseln der Grösse 2 abgefüllt.
Als Wirkstoff in den Beispielen 114-116 kann jede der Verbindungen (1) - (77), (93-96), sowie (78) - (92) eingesetzt werden. Besonders bevorzugt als Wirkstoff sind die Verbindungen (1), (2), (3), (4), (10), (12), (19), (21) und (57), sowie (82).

Beispiel 117: Trockenampullen oder Vials, enthaltend 500 mg des Wirkstoffs, können wie folgt hergestellt werden:

Zusammensetzung: (für 1 Ampulle oder Vial):

| | |
|---|---|
| Wirkstoff | 0,5 g |
| Mannit | 0,05 g |
| Wasser | |

Eine sterile wässrige Lösung des Wirkstoffes und des Mannits wird unter aseptischen Bedingungen in 5 ml-Ampullen oder 5 ml-Vials verschlossen und geprüft.
Als Wirkstoff wird vorzugsweise ein Alkalimetallsalz, z.B. ein solches von Beispiel 112, verwendet.

Beispiel 118: Versuchsbericht.

1. Getestete Verbindungen werden in den nachfolgenden Tabellen 1 und 2 durch dieselben Nummern gekennzeichnet, unter welchen ihre Herstellung in den vorangehenden Beispielen angegeben wird.

2. Testorganismen werden in den nachfolgenden Tabellen 1 und 2 in einer folgendermassen gekürzten Form angegeben:

| | |
|---|---|
| Staphylococcus aureus 10B | Staph. 10B |
| Staphylococcus aureus 2999 | Staph. 2999 |
| Escherichia coli 205 | Esch. 205 |
| Escherichia coli 2018 | Esch. 2018 |
| Salmonella Typhimurium 277 | Salm. 277 |
| Proteus morganii 2359 | Prot. 2359 |
| Proteus morganii 1518 | Prot. 1518 |
| Pseudomonas aeruginosa K 799/61 | Pseu. 799 |
| Mycobacterium tuberculosis | Myc.tub. |

3. Methodik
Die antibiotische Aktivität der Testverbindungen in vitro wurde durch die Agarverdünnungsmethode nach Ericsson, H.M., und Sherris, S.C., 1971, Acta Path. Microb. Scand. Section B, Suppl. No. 217, Bd. 1-90, in DST Agara ermittelt. Die gefundenen, das Wachstum der Testorganismen noch hemmenden Minimalkonzentrationen (MIC = minimum inhibiting concentrations) werden in Mikrogramm pro Milliliter (μg/ml) für die geprüften Verbindungen in der Tabelle 1 angegeben.

Tabelle 1

Antibiotische Aktivität der Imidazolo[4,5-c]rifamycine

| Verb. No. | Staph. 10B | Staph. 2999 | Esch. 205 | Esch. 2018 | Salm. 277 | Prot. 2359 | Prot. 1518 | Pseu. 799 | Myc. tub. |
|---|---|---|---|---|---|---|---|---|---|
| 1 | 0.005 | 0.005 | 8 | 4 | 16 | 8 | 4 | 2 | 0.97 |
| 2 | 0.01 | 0.005 | 16 | 16 | 32 | 4 | 4 | 8 | 1.9 |
| 3 | 0.01 | 0.01 | 16 | 16 | 32 | 16 | 16 | 4 | 0.97 |
| 4 | 0.005 | 0.005 | 4 | 4 | 8 | 4 | 4 | 8 | 0.97 |
| 5 | 0.005 | 0.005 | 4 | 4 | 8 | 16 | 8 | 16 | 1.9 |
| 6 | - | - | 128 | 128 | 128 | 128 | 128 | 128 | - |
| 7 | 0.005 | 0.005 | 8 | 8 | 16 | 8 | 8 | 8 | - |
| 8 | 0.01 | 0.01 | 8 | 8 | 32 | 8 | 8 | 8 | - |
| 9 | - | - | 8 | 8 | 32 | 8 | 1 | 8 | - |
| 10 | 0.005 | 0.005 | 4 | 4 | 16 | 4 | 4 | 8 | - |
| 11 | 0.01 | 0.01 | 8 | 8 | 32 | 8 | 2 | 8 | - |
| 12 | 0.001 | 0.001 | 8 | 8 | 32 | 8 | 1 | 8 | - |
| 13 | 0.05 | 0.05 | 8 | 8 | 32 | 16 | 16 | 8 | - |
| 14 | 0.05 | 0.01 | 8 | 16 | 32 | 16 | 16 | 16 | - |
| 15 | 0.005 | 0.005 | 8 | 8 | 32 | 4 | 8 | 16 | - |
| 16 | 0.01 | 0.01 | 8 | 8 | 32 | 4 | 2 | 8 | - |
| 17 | 0.005 | 0.005 | 32 | 64 | 64 | 32 | 64 | 64 | - |
| 18 | 0.005 | 0.005 | 2 | 4 | 16 | 2 | 4 | 8 | 0.48 |
| 19 | 0.005 | 0.005 | 2 | 4 | 8 | 4 | 4 | 8 | 0.48 |
| 20 | - | - | 8 | 8 | 32 | 8 | 4 | 16 | - |
| 21 | 0.005 | 0.005 | 4 | 4 | 16 | 4 | 4 | 16 | - |
| 22 | 0.005 | 0.005 | 16 | 8 | 32 | 4 | 4 | 16 | - |
| 23 | 0.1 | 0.1 | 16 | 8 | 16 | 16 | 16 | 16 | - |
| 24 | 0.05 | 0.01 | 8 | 4 | 16 | 4 | 8 | 16 | - |
| 25 | 0.005 | 0.005 | 8 | 16 | 16 | 8 | 8 | 16 | 0.97 |
| 26 | 0.05 | 0.01 | 16 | 32 | 84 | 16 | 16 | 16 | - |
| 27 | 0.001 | 0.001 | 8 | 8 | 32 | 8 | 2 | 8 | - |

| Verb. Nr. | Staph. 10B | Staph. 2999 | Esch. 205 | Esch. 2018 | Salm. 277 | Prot. 2359 | Prot. 1518 | Pseu. 799 | Myc. tub. |
|---|---|---|---|---|---|---|---|---|---|
| 28 | 0.005 | 0.005 | 4 | 4 | 16 | 2 | 2 | 8 | 0.97 |
| 29 | - | - | 32 | 32 | 128 | 8 | 8 | 8 | - |
| 30 | - | - | 8 | 8 | 32 | 8 | 8 | 32 | - |
| 31 | 0.002 | 0.001 | 8 | 8 | 32 | 8 | 8 | 8 | - |
| 32 | 0.001 | 0.001 | 8 | 8 | 32 | 8 | 8 | 8 | - |
| 33 | 0.001 | 0.001 | 8 | 2 | 32 | 4 | 4 | 32 | - |
| 34 | - | - | 8 | 2 | 32 | 8 | 8 | 32 | - |
| 35 | - | - | 8 | 8 | 32 | 8 | 8 | 8 | - |
| 36 | - | - | 2 | 8 | 32 | 8 | 8 | 8 | - |
| 38 | 0.005 | 0.005 | 4 | 2 | 16 | 2 | 4 | 16 | - |
| 39 | - | - | 8 | 8 | 32 | 8 | 8 | 32 | - |
| 40 | 0.005 | 0.005 | 8 | 8 | 32 | 8 | 8 | 16 | - |
| 41 | 0.005 | - | 8 | 2 | - | 8 | 8 | 32 | - |
| 42 | 0.005 | - | 8 | 8 | 32 | 8 | 8 | 32 | - |
| 43 | 0.01 | 0.005 | 8 | 4 | 32 | 8 | 8 | 16 | - |
| 44 | - | - | 8 | 8 | 32 | 8 | 8 | 32 | - |
| 45 | 0.01 | 0.005 | 8 | 1 | 32 | 8 | 8 | 32 | - |
| 46 | - | - | 32 | 8 | 32 | 8 | 8 | 32 | - |
| 47 | 0.005 | 0.005 | 8 | 4 | 16 | 4 | 4 | 32 | - |
| 48 | 0.001 | 0.001 | 32 | 8 | 32 | 8 | 8 | 32 | - |
| 49 | 0.001 | 0.001 | 8 | 32 | 32 | 8 | 8 | 8 | - |
| 50 | 0.01 | 0.01 | 8 | 8 | 32 | 8 | 8 | 8 | - |
| 51 | 0.01 | - | 8 | 32 | 32 | 8 | 8 | 8 | - |
| 52 | - | - | 32 | 8 | 32 | 8 | 8 | 32 | - |
| 53 | 0.01 | 0.01 | 8 | 8 | 32 | 8 | 2 | 32 | - |
| 54 | 0.005 | 0.005 | 8 | 8 | 32 | 8 | 8 | 16 | - |
| 55 | 0.01 | 0.001 | 8 | 1 | 32 | 2 | 2 | 32 | - |
| 56 | 0.005 | - | 8 | 8 | 32 | 8 | 8 | 32 | - |
| 57 | 0.005 | 0.005 | 8 | 4 | 16 | 4 | 4 | 16 | 0.97 |
| 58 | 0.005 | 0.005 | 16 | 3α | 32 | 16 | 16 | 16 | - |

| Verb. No. | Staph. 10B | Staph. 2999 | Esch. 205 | Esch. 2018 | Salm. 277 | Prot. 2359 | Prot. 1518 | Pseu. 799 | Myc. tub. |
|---|---|---|---|---|---|---|---|---|---|
| 59 | 0.01 | 0.01 | 8 | 8 | 32 | 8 | 2 | 8 | - |
| 60 | 0.02 | 0.02 | 32 | 32 | 128 | 32 | 32 | 64 | - |
| 61 | 0.005 | 0.005 | 16 | 4 | 32 | 4 | 4 | 32 | - |
| 62 | 0.02 | 0.02 | 32 | 32 | 128 | 8 | 8 | 32 | - |
| 63 | 0.01 | 0.01 | 16 | 8 | 32 | 8 | 8 | 32 | - |
| 64 | 0.01 | 0.005 | 16 | 16 | 64 | 16 | 16 | 16 | - |
| 65 | 0.05 | 0.01 | 32 | 32 | 128 | 32 | 32 | 32 | - |
| 66 | 0.01 | 0.01 | 16 | 16 | 128 | 16 | 16 | 32 | - |
| 67 | 0.005 | 0.005 | 16 | 16 | 64 | 16 | 16 | 32 | - |
| 68 | 0.01 | - | 32 | 32 | 32 | 32 | 8 | 8 | - |
| 69 | 0.05 | 0.05 | 32 | 32 | 128 | 32 | 32 | 128 | - |
| 70 | 0.02 | 0.02 | 32 | 32 | 128 | 32 | 32 | 128 | - |
| 71 | 0.02 | 0.02 | 32 | 32 | 128 | 32 | 32 | 128 | - |
| 72 | 0.05 | 0.05 | 32 | 32 | 128 | 32 | 32 | 64 | - |
| 73 | 0.05 | 0.05 | 128 | 128 | 128 | 32 | 32 | 128 | - |
| 74 | 0.5 | 0.5 | 128 | 32 | 128 | 32 | 32 | 128 | - |
| 75 | 0.01 | - | 8 | 8 | 32 | 8 | 2 | 32 | - |
| 76 | 0.01 | 0.01 | 32 | 8 | 32 | 8 | 8 | 32 | - |
| 77 | 0.01 | 0.01 | 8 | 8 | 32 | 8 | 8 | 8 | - |

- = nicht getestet.

Tabelle 2

Antibiotische Aktivität der Rifamycin-formamidine

| Verb. No. | Staph. 10B | Staph. 2999 | Esch. 205 | Esch. 2018 | Salm. 277 | Prot. 2354 | Prot. 1518 | Pseu. 799 | Myc. tub. |
|---|---|---|---|---|---|---|---|---|---|
| 78 | 0.05 | 0.05 | 16 | 16 | 16 | 16 | 4 | 4 | 0.00045 |
| 79 | 0.05 | 0.05 | 32 | 64 | 32 | 16 | 16 | 16 | 0.0037 |
| 80 | - | 0.005 | 64 | 64 | 64 | 16 | 32 | 64 | 0.0075 |
| 81 | 0.005 | 0.005 | 16 | 16 | 8 | 4 | 4 | 16 | 0,00022 |
| 82 | 0.005 | 0.01 | 16 | 16 | 16 | 8 | 4 | 16 | 0,0037 |
| 84 | 128 | 0.05 | 64 | 32 | 64 | 16 | 16 | 64 | - |
| 85 | - | 0.005 | 64 | 128 | 32 | 16 | 32 | 64 | - |
| 86 | 0.05 | 0.05 | 4 | 4 | 4 | 16 | 4 | 4 | - |
| 87 | 0.005 | 0.005 | 64 | 64 | 64 | 16 | 32 | 32 | - |
| 88 | 0.005 | 0.005 | 16 | 16 | 16 | 64 | 4 | 8 | 0.015 |
| 89 | 0.05 | 0.1 | 64 | 64 | 64 | 64 | 16 | 16 | 0.03 |
| 90 | 0.01 | 0.05 | 128 | 64 | 128 | 16 | 16 | 16 | - |
| 91 | 0.005 | 0.05 | 16 | 32 | 16 | 16 | 16 | 16 | 0.00045 |
| 92 | 128 | 0.005 | 64 | 16 | 64 | 16 | 16 | 64 | 0.0018 |

Patentansprüche: (für alle benannten Länder ausser Oesterreich)

1. Ein 4-Aminoimidazolo[4.5-c]rifamycin SV oder S der Formel

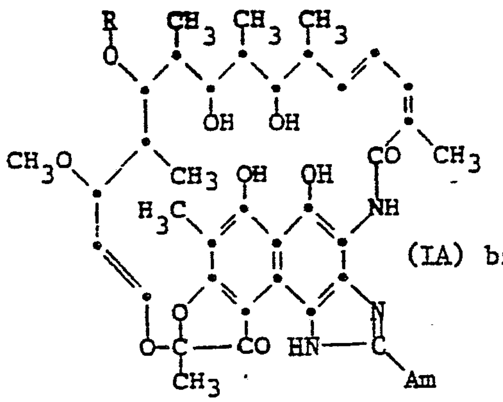
(IA) bzw.

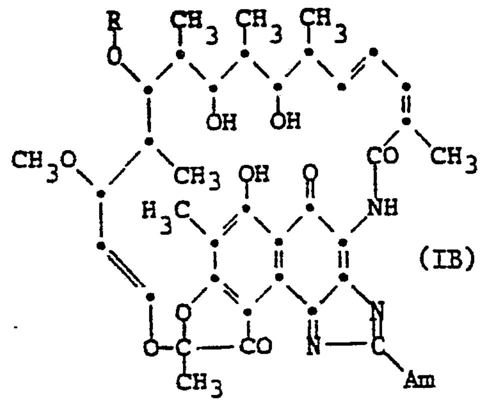
(IB)

worin R Wasserstoff oder Acetyl und Am eine von einem sekundären Amin abgeleitete Aminogruppe bedeutet, sowie Salze von entsprechenden Verbindungen mit salzbildenden Eigenschaften.

2. Ein 4-Aminoimidazolo[4,5-c]rifamycin SV oder S gemäss Anspruch 1, worin das Symbol Am in der Formel IA bzw. IB eine Aminogruppe bedeutet, die zwei gleiche oder verschiedene einwertige, gegebenenfalls substituierte Hydrocarbyl- oder Heterocyclylreste mit höchstens 20 C-Atomen, oder einen entsprechenden zweiwertigen Rest, welcher mit der Aminogruppe einen nichtaromatischen stickstoffhaltigen Heterocyclylrest bildet, trägt.

3. Ein 4-Aminoimidazolo[4,5-c]rifamycin SV oder S gemäss Anspruch 1, worin in der Formel IA bzw. IB Am durch die Partialformel

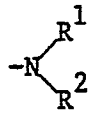
($Am_A$)

charakterisiert ist, worin $R^1$ und $R^2$ unabhängig voneinander je ein Niederalkyl oder Niederalkenyl, das einfach oder mehrfach durch Hydroxyl, Mercapto, Niederalkoxyl, Niederalkoxycarbonyl, ein unsubstituiertes oder N-mono- oder disubstituiertes Carbamoyl, Formyl, Oxo,

acetalisiertes bzw. ketalisiertes Oxo und/oder eine disubstituierte Aminogruppe substituiert sein kann; ein Niederalkinyl; ein Cycloalkyl oder Cycloalkyl-niederalkyl, das gegebenenfalls eine oder zwei Doppelbindungen aufweisen kann; ein unsubstituiertes oder durch Halogen, Hydroxy, Trifluormethyl, ein unsubstituiertes oder N-mono- oder-di-substituiertes Carbamoyl oder Niederalkoxycarbonyl substituiertes Phenyl oder Phenylniederalkyl; oder ein höchstens bicyclisches Heterocyclyl oder Heterocyclylalkyl mit höchstens zwei Heteroatomen ausgewählt von Sauerstoff, Stickstoff und/oder Schwefel bedeuten, wobei $R^1$ und $R^2$ durch eine einfache Kohlenstoff-Kohlenstoff-Bindung oder über ein Sauerstoff-, Schwefel(II)- oder gegebenenfalls substituiertes Stickstoffatom miteinander verbunden sein können und zusammen mit dem Aminostickstoffatom einen 4- bis 8-gliedrigen nicht-aromatischen heterocyclischen Ring bilden können.

4. Ein 4-Aminoimidazolo[4,5-c]rifamycin SV oder S gemäss Anspruch 1 oder 2, worin das Symbol Am einen Rest der Partialformel

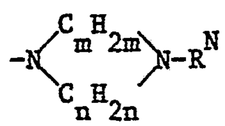

$(Am_B)$ ,

worin m und n unabhängig je eine ganze Zahl von 1 bis 5 darstellen, wobei ein mindestens 5-gliedriger Ring gebildet wird, und $R^N$ ein Wasserstoffatom, eine Formylgruppe, eine funktionell abgewandelte Carboxylgruppe oder einen unsubstituierten oder substituierten, höchstens 10 C-Atome aufweisenden Hydrocarbyl- oder Heterocyclyl-Rest bedeutet, wobei beim Heterocyclylrest die freie Valenz jeweils von einem C-Atom ausgeht.

5. Ein 4-Aminoimidazolo[4,5-c]rifamycin SV oder S gemäss Anspruch 4, worin das Symbol Am ein 1-Piperazinyl-Rest der Formel

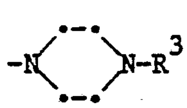

$(Am_C)$

bedeutet, worin $R^3$ ein Niederalkyl oder Niederalkenyl, das einfach oder mehrfach durch Hydroxyl, Mercapto, Niederalkoxy, Niederalkoxycarbonyl, ein gegebenenfalls N-mono- oder -di-substituiertes Carbamoyl, Formyl, Oxo, acetalisiertes bzw. ketalisiertes Oxo und/oder eine disubstituierte Aminogruppe substituiert sein kann, ein Niederalkynyl, ein Cycloalkyl oder Cycloalkyl-niederalkyl, das gegebenenfalls eine oder zwei Doppelbindungen aufweisen kann, ein unsubstituiertes oder durch Halogen, Hydroxyl, Trifluormethyl, ein gegebenenfalls N-mono- oder -di-substituiertes Carbamoyl oder Niederalkoxycarbonyl einfach oder mehrfach substituiertes Phenyl oder Phenylniederalkyl, oder ein höchstens bicyclisches Heterocyclyl oder Heterocyclylalkyl mit höchstens zwei Heteroatomen ausgewählt von Sauerstoff, Stickstoff und/oder Schwefel bedeutet.

6. Ein 4-Aminoimidazolo[4,5-c]rifamycin SV oder S gemäss Anspruch 5, worin das Symbol $R^3$ in der Partialformel $Am_c$ ein geradkettiges oder einfach verzweigtes Niederalkyl, Niederalkenyl oder Niederalkinyl, ein Cycloalkyl oder Cycloalkylmethyl mit 3-6 Ringgliedern, oder ein Phenyl oder Benzyl bedeutet, welches durch Trifluormethyl, Carbamoyl oder Niederalkoxycarbonyl substituiert sein kann.

7. Ein 4-Aminoimidazolo[4,5-c]rifamycin SV oder S gemäss Anspruch 3, worin die Symbole $R^1$ und $R^2$ im Rest $Am_A$ je ein unsubstituiertes Alkyl mit 1-4 C-Atomen darstellen, oder eines davon ein Cycloalkyl oder ein Cycloalkylmethyl mit 3-6 Ringgliedern, oder Benzyl bedeutet, oder beide zusammen mit dem Stickstoffatom einen gesättigten, monocyclischen, 5-8 Ringglieder enthaltenden Heterocyclylrest bedeuten, welcher auch ein Sauerstoff- oder Schwefel-(II)-Atom enthalten kann, wobei die Heteroatome vom Stickstoffatom durch mindestens 1 C-Atom getrennt sind.

8. Eine Verbindung gemäss einem der Ansprüche 1-7, in Form des Hydrochinons der SV-Reihe.

9. 4-Dimethylamino-imidazolo[4,5-c]rifamycin SV.

10. 4-Morpholino-imidazolo[4,5-c]rifamycin SV.

11. 4-(4-Isobutylpiperazinyl)-imidazolo[4,5-c]rifamycin SV.

12. 4-Pyrrolidinyl-imidazolo[4,5-c]rifamycin SV.

13. Eine der in Ansprüchen 1-12 genannten Verbindungen in Form eines physiologisch verträglichen Salzes.

14. Ein 4-Aminoimidazolo[4,5-c]rifamycin SV oder S gemäss der Ansprüche 1-13 als Antibiotikum.

15. Verfahren zur Herstellung eines 4-Aminoimidazolo[4,5-c]rifamycins SV oder S der im Anspruch 1 definierten Formeln IA bzw. IB, dadurch gekennzeichnet, dass man

a) ein 3-Aminorifamycin-S-4-imin der Formel

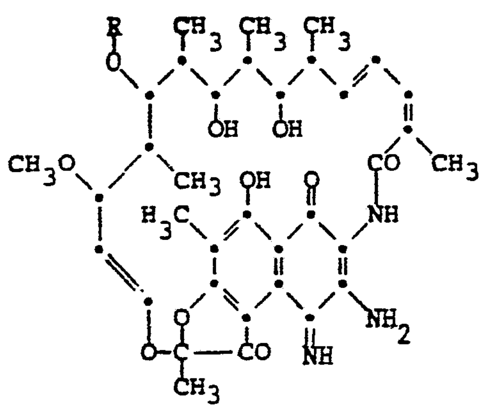

(IV),

worin R die im Anspruch 1 genannte Bedeutung hat, mit einem reaktionsfähigen Derivat eines N,N-disubstituierten Formamids oder Thioformamids der Formel

Am-CH=Y (III),

worin Y Sauerstoff oder Schwefel ist und Am die im Anspruch 1 genannte Bedeutung hat, umsetzt oder

b) ein N',N'-disubstituiertes 3-Aminomethylenaminorifamycin-Derivat der Formel

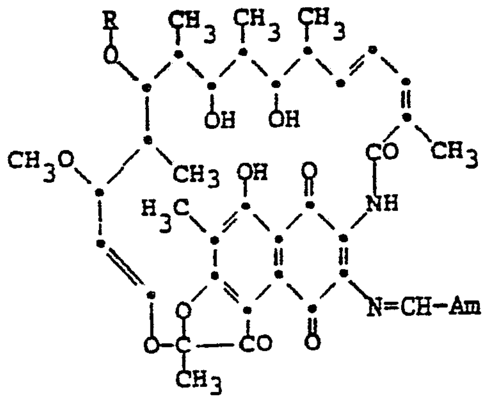

(V),

worin R und Am die im Anspruch 1 genannten Bedeutungen haben, mit Ammoniak behandelt, oder

c) eine 3-Aminorifamycin-S-Verbindung der Formel

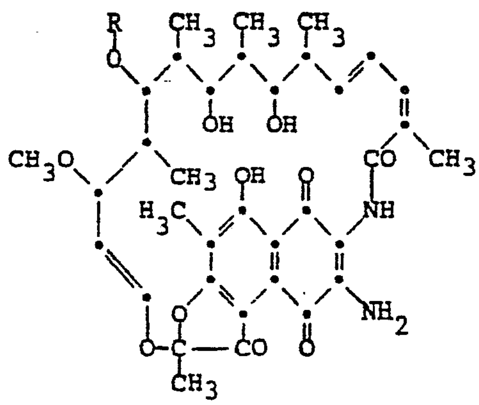

(II),

worin R die im Anspruch 1 genannte Bedeutung hat, in beliebiger Reihenfolge A) mit Ammoniak und B) mit einem reaktionsfähigen Derivat eines N,N-disubstituierten Formamids oder Thioformamids der Formel

Am-CH=Y (III),

worin Y Sauerstoff oder Schwefel ist und Am die im Anspruch 1 genannte Bedeutung hat, umsetzt, und gewünschtenfalls eine erhaltene Verbindung des SV-Typs zu einer Verbindung des S-Typs oxidiert oder eine erhaltene Verbindung des S-Typs zu einer Verbindung des SV-Typs reduziert und/oder eine erhaltene freie Verbindung mit salzbildenden Eigenschaften in ein entsprechendes Salz oder ein erhaltenes Salz in die entsprechende freie Verbindung umwandelt.

16. Ein pharmazeutisches Präparat enthaltend mindestens eine Verbindung der Formel I gemäss einem der Ansprüche 1-13 als Wirkstoff.

17. Verfahren zur Herstellung eines pharmazeutischen Präparats gemäss Anspruch 16, dadurch gekennzeichnet, dass man eine Verbindung der Formel I gemäss einem der Ansprüche 1-13 mit mindestens einem pharmazeutischen Trägermaterial auf nicht-chemischem Wege verarbeitet.

18. Verwendung einer Verbindung der Formel I gemäss einem der Ansprüche 1-13 allein oder in Form eines pharmazeutischen Präparats gemäss Anspruch 16 zur Bekämpfung von mikrobiellen Infektionen in einem Warmblüter, der mit einem gegen mindestens ein der Rifamycin-Derivate der Formel I empfindlichen Mikroorganismus infiziert ist, welche Verwendung durch die Behandlung dieses Mikroorganismus oder eines durch diesen Mikroorganismus infizierten Mediums mit einer antimikrobiell wirksamen Dosis eines Rifamycin-Derivats der Formel I gemäss einem der Ansprüche 1-13 gekennzeichnet ist.

19. Ein Formamidin der Formel

$$Rif{=}CH{-}Am \qquad (VI),$$

worin Am eine von einem sekundären Amin abgeleitete Aminogruppe und Rif einen zweiwertigen 3-Aminorifamycin-Rest der Formel

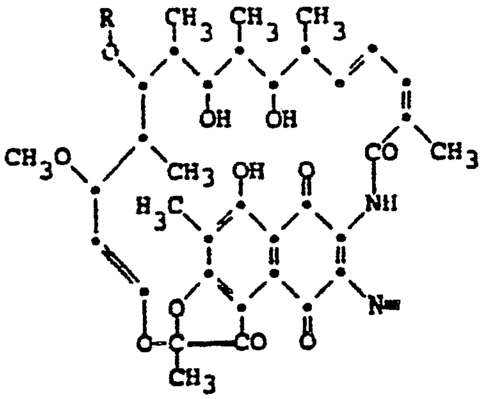

oder

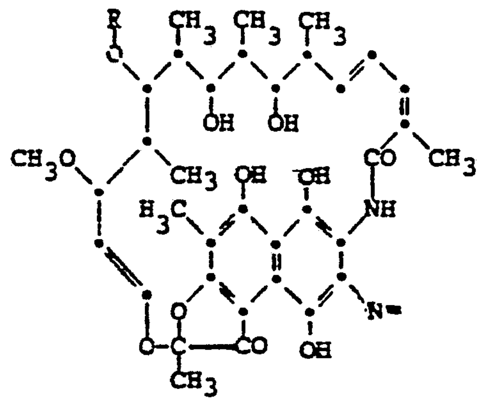

[Rif(S)] [Rif(SV)]

worin R Wasserstoff oder Acetyl ist, bedeutet, sowie entsprechende Salze von einer solchen Verbindung mit salzbildenden Eigenschaften.

20. Ein Formamidin gemäss Anspruch 19, worin das Symbol Am eine der in Ansprüchen 2-7 angegebenen Bedeutungen hat.

21. Ein Formamidin gemäss Anspruch 19 oder 20 in der Chinon-Form entsprechend dem Rest Rif(S).

22. 3-Indolinylmethylenamino-Rifamycin S.

23. Ein Formamidin gemäss einem der Ansprüche 19-22 als Antibiotikum.

24. Verfahren zur Herstellung eines Formamidins der im Anspruch 19 definierten Formel VI, dadurch gekennzeichnet, dass man eine 3-Amino-rifamycin-Verbindung der Formel

$$Rif{=}H_2 \text{ ,}$$

worin Rif die im Anspruch 19 genannte Bedeutung hat, mit einem reaktionsfähigen Derivat eines N,N-disubstituierten Formamids oder Thioformamids der Formel

$$Am\text{-}CH{=}Y \qquad \text{(III),}$$

worin Y Sauerstoff oder Schwefel ist und Am die im Anspruch 19 definierte Bedeutung hat, umsetzt und gewünschtenfalls eine erhaltene Verbindung des S-Typs zu einer Verbindung des SV-Typs reduziert oder eine erhaltene Verbindung des SV-Typs zu einer Verbindung des S-Typs oxidiert und/oder eine erhaltene freie Verbindung mit salzbildenden Eigenschaften in ein entsprechendes Salz oder ein erhaltenes Salz in die entsprechende freie Verbindung umwandelt.

25. Ein pharmazeutisches Präparat enthaltend mindestens ein Formamidin der Formel VI gemäss einem der Ansprüche 19-22 als Wirkstoff.

26. Verfahren zur Herstellung eines pharmazeutischen Präparates gemäss Anspruch 25, dadurch gekennzeichnet, dass man eine Verbindung der Formel VI gemäss einem der Ansprüche 19-22 mit mindestens einem pharmazeutischen Trägermaterial auf nicht-chemischem Wege verarbeitet.

27. Verwendung einer Verbindung der Formel VI gemäss einem der Ansprüche 19-22 allein oder in Form eines pharmazeutischen Präparates gemäss Anspruch 25 zur Bekämpfung von mikrobiellen Infektionen in einem Warmblüter, der mit einem gegen mindestens ein der Rifamycin-Derivate der Formel I empfindlichen Mikroorganismus infiziert ist, welche Verwendung durch die Behandlung dieses Mikroorganismus oder eines durch diesen Mikroorganismus infizierten Mediums mit einer antimikrobiell wirksamen Dosis eines Rifamycin-Derivats der Formel VI gemäss einem der Ansprüche 19-22 gekennzeichnet ist.

Patentansprüche (Oesterreich)

1. Verfahren zur Herstellung eines 4-Aminoimidazolo[4.5-c]rifamycins SV oder S der Formel

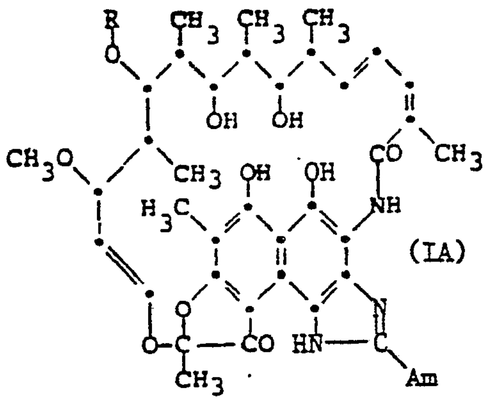

(IA) bzw.

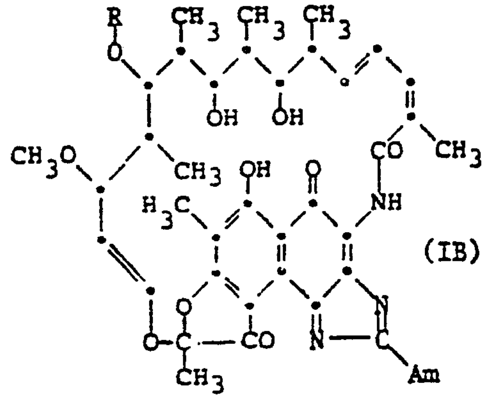

(IB)

worin R Wasserstoff oder Acetyl und Am eine von einem sekundären Amin abgeleitete Aminogruppe bedeutet, sowie von Salzen von entsprechenden Verbindungen mit salzbildenden Eigenschaften, dadurch gekennzeichnet, dass man

a) ein 3-Aminorifamycin-S-4-imin der Formel

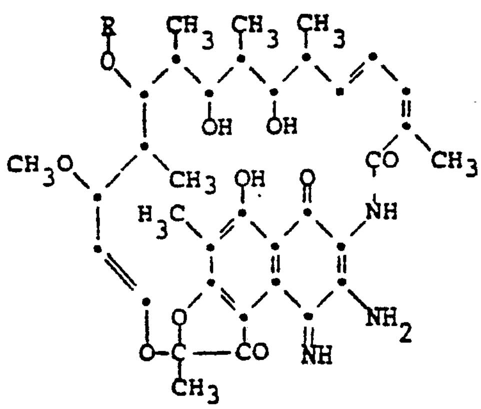

(IV),

worin R die oben genannte Bedeutung hat, mit einem reaktionsfähigen Derivat eines N,N-disubstituierten Formamids oder Thioformamids der Formel

Am-C=Y (III),

worin Y Sauerstoff oder Schwefel ist und Am die oben genannte Bedeutung hat, umsetzt oder

b) ein N',N'-disubstituiertes 3-Aminomethylenaminorifamycin-Derivat der Formel

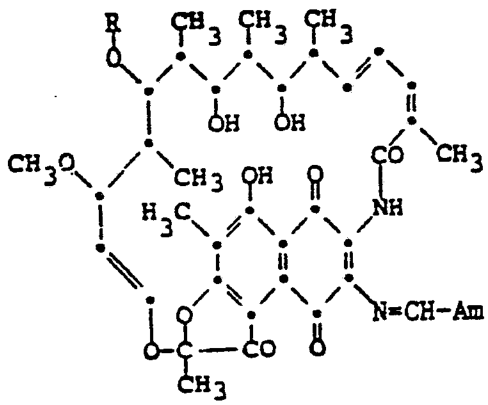

(V),

worin R und Am die oben genannten Bedeutungen haben, mit Ammoniak behandelt, oder

c) eine 3-Aminofifamycin-S-Verbindung der Formel

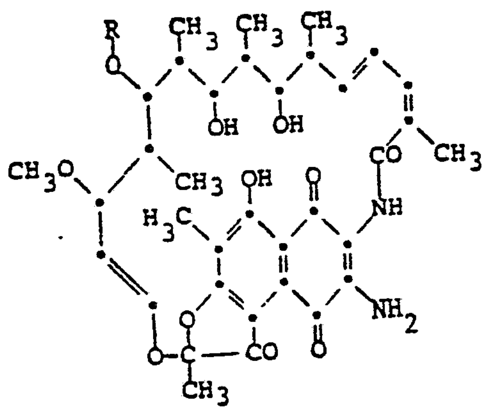

(II),

worin R die oben genannte Bedeutung hat, in beliebiger Reihenfolge A) mit Ammoniak und B) mit einem reaktionsfähigen Derivat eines N,N-disubstituierten Formamids oder Thioformamids der Formel

Am-CH=Y (III),

worin Y Sauerstoff oder Schwefel ist und Am die oben genannte Bedeutung hat, umsetzt, und gewünschtenfalls eine erhaltene Verbindung des SV-Typs zu einer Verbindung des S-Typs oxidiert oder eine erhaltene

Verbindung des S-Typs zu einer Verbindung des SV-Typs reduziert und/oder eine erhaltene freie Verbindung mit salzbildenden Eigenschaften in ein entsprechendes Salz oder ein erhaltenes Salz in die entsprechende freie Verbindung umwandelt.

2. Verfahren gemäss Anspruch 1, dadurch gekennzeichnet, dass man ein 4-Aminoimidazolo[4,5-c]rifamycin SV oder S der Formel IA bzw. IB herstellt, worin Am eine Aminogruppe bedeutet, die zwei gleiche oder verschiedene einwertige, gegebenenfalls substituierte Hydrocarbyl- oder Heterocyclylreste mit höchstens 20 C-Atomen, oder einen entsprechenden zweiwertigen Rest, welcher mit der Aminogruppe einen nichtaromatischen stickstoffhaltigen Heterocyclylrest bildet, trägt.

3. Verfahren gemäss Anspruch 1, dadurch gekennzeichnet, dass man ein 4-Aminoimidazolo[4,5-c]rifamycin SV oder S der Formel IA bzw. IB herstellt, worin Am durch die Partialformel

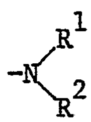

$(Am_A)$

charakterisiert ist, worin $R^1$ und $R^2$ unabhängig voneinander je ein Niederalkyl oder Niederalkenyl, das einfach oder mehrfach durch Hydroxyl, Mercapto, Niederalkoxyl, Niederalkoxycarbonyl, ein unsubstituiertes oder N-mono- oder disubstituiertes Carbamoyl, Formyl, Oxo, acetalisiertes bzw. ketalisiertes Oxo und/oder eine disubstituierte Aminogruppe substituiert sein kann; ein Niederalkinyl; ein Cycloalkyl oder Cycloalkyl-niederalkyl, das gegebenenfalls eine oder zwei Doppelbindungen aufweisen kann; ein unsubstituiertes oder durch Halogen, Hydroxy, Trifluormethyl, ein unsubstituiertes oder N-mono- oder-disubstituiertes Carbamoyl oder Niederalkoxycarbonyl substituiertes Phenyl oder Phenylniederalkyl; oder ein höchstens bicyclisches Heterocyclyl oder Heterocyclylalkyl mit höchstens zwei Heteroatomen ausgewählt von Sauerstoff, Stickstoff und/oder Schwefel bedeuten, wobei $R^1$

und $R^2$ durch eine einfache Kohlenstoff-Kohlenstoff-Bindung oder über ein Sauerstoff-, Schwefel(II)- oder gegebenenfalls substituiertes Stickstoffatom miteinander verbunden sein können und zusammen mit dem Aminostickstoffatom einen 4- bis 8-gliedrigen nicht-aromatischen heterocyclischen Ring bilden können.

4. Verfahren gemäss Anspruch 1, dadurch gekennzeichnet, dass man ein 4-Aminoimidazolo[4,5-c]rifamycin SV oder S der Formel IA bzw. IB herstellt, worin das Symbol Am einen Rest der Partialformel

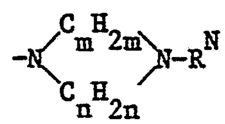

($Am_B$)

bedeutet, worin m und n unabhängig je eine ganze Zahl von 1 bis 5 darstellen, wobei ein mindestens 5-gliedriger Ring gebildet wird, und $R^N$ ein Wasserstoffatom, eine Formylgruppe, eine funktionell abgewandelte Carboxylgruppe oder einen unsubstituierten oder substituierten, höchstens 10 C-Atome aufweisenden Hydrocarbyl- oder Heterocyclyl-Rest bedeutet, wobei beim Heterocyclylrest die freie Valenz jeweils von einem C-Atom ausgeht.

5. Verfahren gemäss Anspruch 1, dadurch gekennzeichnet, dass man ein 4-Aminoimidazolo[4,5-c]rifamycin SV oder S der Formel IA bzw. IB herstellt, worin das Symbol Am ein 1-Piperazinyl-Rest der Formel

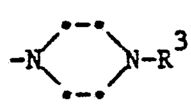

($Am_C$)

bedeutet, worin $R^3$ ein Niederalkyl oder Niederalkenyl, das einfach oder mehrfach durch Hydroxyl, Mercapto, Niederalkoxy, Niederalkoxycarbonyl, ein gegebenenfalls N-mono- oder -di-substituiertes Carbamoyl, Formyl, Oxo, acetalisiertes bzw. ketalisiertes Oxo und/oder eine disubstituierte Aminogruppe substituiert sein kann, ein Niederalkynyl, ein Cycloalkyl oder Cycloalkyl-niederalkyl, das gegebenenfalls eine oder zwei Doppelbindungen aufweisen kann, ein unsubstituiertes oder durch Halogen, Hydroxyl, Trifluormethyl, ein gegebenenfalls N-mono- oder -di-substituiertes Carbamoyl oder Niederalkoxycarbonyl einfach oder mehrfach substituiertes Phenyl oder Phenylniederalkyl, oder ein höchstens bicyclisches Heterocyclyl oder Heterocyclylalkyl mit höchstens zwei Heteroatomen ausgewählt von Sauerstoff, Stickstoff und/oder Schwefel bedeutet.

6. Verfahren gemäss Anspruch 1, dadurch gekennzeichnet, dass man ein 4-Aminoimidazolo[4,5-c]rifamycin SV oder S der Formel IA bzw. IB herstellt, worin Am der im Anspruch 5 angegebenen Teilformel $Am_c$ entspricht, in welcher das Symbol $R^3$ ein geradkettiges oder einfach verzweigtes Niederalkyl, Niederalkenyl oder Niederalkinyl, ein Cycloalkyl oder Cycloalkylmethyl mit 3-6 Ringgliedern, oder ein Phenyl oder Benzyl bedeutet, welches durch Trifluormethyl, Carbamoyl oder Niederalkoxycarbonyl substituiert sein kann.

7. Verfahren gemäss Anspruch 1, dadurch gekennzeichnet, dass man ein 4-Aminoimidazolo[4,5-c]rifamycin SV oder S der Formel IA bzw. IB herstellt, worin Am der im Anspruch 3 angegebenen Teilformel $Am_A$ entspricht, in welcher die Symbole $R^1$ und $R^2$ je ein unsubstituiertes Alkyl mit 1-4 C-Atomen darstellen, oder eines davon ein Cycloalkyl oder ein Cycloalkylmethyl mit 3-6 Ringgliedern, oder Benzyl bedeutet, oder beide zusammen mit dem Stickstoffatom einen gesättigten, monocyclischen, 5-8 Ringglieder enthaltenden Heterocyclylrest bedeuten, welcher auch ein Sauerstoff- oder Schwefel-(II)-Atom als Ringglied enthalten kann, wobei die Heteroatome vom Stickstoffatom durch mindestens 1 C-Atom getrennt sind.

8. Verfahren gemäss einem der Ansprüche 1-7, dadurch gekennzeichnet, dass man den Endstoff in Form eines Hydrochinons der SV-Reihe herstellt.

9. Verfahren gemäss Anspruch 1, dadurch gekennzeichnet, dass man 4-Dimethylamino-imidazolo[4,5-c]rifamycin SV herstellt.

10. Verfahren gemäss Anspruch 1, dadurch gekennzeichnet, dass man 4-Morpholino-imidazolo[4,5-c]rifamycin SV herstellt.

11. Verfahren gemäss Anspruch 1, dadurch gekennzeichnet, dass man 4-(4-Isobutylpiperazinyl)-imidazolo[4,5-c]rifamycin SV herstellt.

12. Verfahren gemäss Anspruch 1, dadurch gekennzeichnet, dass man 4-Pyrrolidinyl-imidazolo[4,5-c]rifamycin SV herstellt.

13. Verfahren gemäss einem der Ansprüche 1-12, dadurch gekennzeichnet, dass man den Endstoff in Form eines physiologisch verträglichen Salzes herstellt.

14. Verfahren zur Herstellung eines pharmazeutischen Präparates enthaltend mindestens eine Verbindung der Formel I gemäss einem der Ansprüche 1-13 als Wirkstoff, dadurch gekennzeichnet, dass man eine solche Verbindung mit mindestens einem pharmazeutischen Trägermaterial auf nicht-chemischem Wege verarbeitet.

15. Verwendung einer Verbindung der Formel I, erhältlich gemäss einem der Ansprüche 1-13 allein oder in Form eines pharmazeutischen Präparats gemäss Anspruch 14 zur Bekämpfung von mikrobiellen Infektionen.

16. Verfahren zur Herstellung eines Formamidins der Formel

Rif=CH-Am (VI),

worin Am eine von einem sekundären Amin abgeleitete Aminogruppe und Rif einen zweiwertigen 3-Aminorifamycin-Rest der Formel

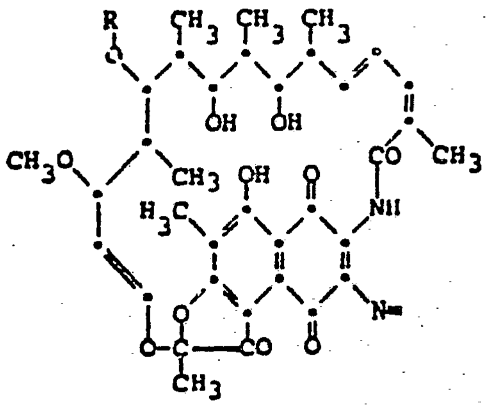

[Rif(S)]

oder

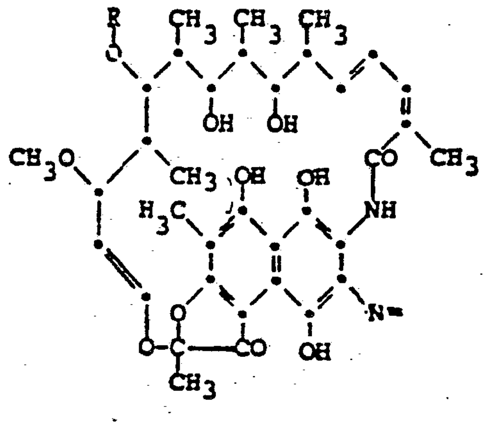

[Rif(SV)]

worin R Wasserstoff oder Acetyl ist, bedeutet, sowie entsprechender Salze von einer solchen Verbindung mit salzbildenden Eigenschaften, dadurch gekennzeichnet, dass man eine 3-Aminorifamycin-Verbindung der Formel

$$Rif=H_2,$$

worin Rif die oben genannte Bedeutung hat, mit einem reaktionsfähigen Derivat eines N,N-disubstituierten Formamids oder Thioformamids der Formel

$$Am-CH=Y \qquad (III),$$

worin Y Sauerstoff oder Schwefel ist und Am die oben definierte Bedeutung hat, umsetzt und gewünschtenfalls eine erhaltene Verbindung des S-Typs zu einer Verbindung des SV-Typs reduziert oder eine erhaltene Verbindung des SV-Typs zu einer Verbindung des S-Typs oxidiert und/oder eine erhaltene freie Verbindung mit salzbildenden Eigenschaften in ein entsprechendes Salz oder ein erhaltenes Salz in die entsprechende freie Verbindung umwandelt.

17. Verfahren gemäss Anspruch 16 zur Herstellung von Verbindungen der Formel VI, worin das Symbol Am eine der in Ansprüchen 2-7 angegebenen Bedeutungen hat.

18. Verfahren gemäss Anspruch 16 oder 17, dadurch gekennzeichnet, dass man ein Formamidin in der Chinon-Form entsprechend dem Rest Rif(S)

herstellt.

19. Verfahren gemäss Anspruch 16, dadurch gekennzeichnet, dass man 3-Indolinylmethylenamino-Rifamycin S herstellt.

20. Verfahren zur Herstellung eines pharmazeutischen Präparates, enthaltend als Wirkstoff mindestens ein Formamidin der Formel VI, erhältlich gemäss einem der Ansprüche 16-19, dadurch gekennzeichnet, dass man eine solche Verbindung mit mindestens einem pharmazeutischen Trägermaterial auf nicht-chemischem Wege verarbeitet.

21. Verwendung einer Verbindung der Formel VI, erhältlich gemäss einem der Ansprüche 16-19 allein oder in Form eines pharmazeutischen Präparates gemäss Anspruch 20 zur Bekämpfung von mikrobiellen Infektionen.